(19) Europäisches Patentamt
European Patent Office
Office européen des brevets

(11) EP 4 429 658 B1

(12) **EUROPEAN PATENT SPECIFICATION**

(45) Date of publication and mention of the grant of the patent:
**24.06.2026 Bulletin 2026/26**

(21) Application number: **22821853.3**

(22) Date of filing: **08.11.2022**

(51) International Patent Classification (IPC):
***A61K 31/404*** *(2006.01)* ***A61K 31/405*** *(2006.01)*
***A61P 3/04*** *(2006.01)* ***A61P 3/10*** *(2006.01)*
***A61P 5/50*** *(2006.01)* ***G01N 33/00*** *(2006.01)*

(52) Cooperative Patent Classification (CPC):
**A61K 31/404; A61K 31/405; A61P 3/04; A61P 3/10; A61P 5/50**

(86) International application number:
**PCT/EP2022/081120**

(87) International publication number:
**WO 2023/079170 (11.05.2023 Gazette 2023/19)**

(54) **BETA2-ADRENERGIC RECEPTOR ANTAGONISTS FOR USE IN TREATING DIABETES OR OBESITY**

BETA2-ADRENERGISCHE REZEPTORANTAGONISTEN ZUR VERWENDUNG BEI DER BEHANDLUNG VON DIABETES ODER ADIPOSITAS

ANTAGONISTES DU RÉCEPTEUR BÊTA2-ADRÉNERGIQUE À UTILISER DANS LE TRAITEMENT DU DIABÈTE OU DE L'OBÉSITÉ

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **08.11.2021 EP 21206861**

(43) Date of publication of application:
**18.09.2024 Bulletin 2024/38**

(73) Proprietor: **NovelYeast bv**
**3052 Oud-Heverlee (Blanden) (BE)**

(72) Inventors:
- **THEVELEIN, Johan Maria J**
**3052 Oud-Heverlee (Blanden) (BE)**
- **KULKARNI, Chetan Pradeep**
**3000 Leuven (BE)**
- **LUYTEN, Walter Herman Maria Louis**
**3060 Bertem (BE)**

(74) Representative: **Nederlandsch Octrooibureau**
**P.O. Box 29720**
**2502 LS The Hague (NL)**

(56) References cited:
**WO-A1-2005/112949**

- **KULKARNI CHETAN P ET AL: "Characterization of SGLT1-mediated glucose transport in Caco-2 cell monolayers, and absence of its regulation by sugar or epinephrine", EUROPEAN JOURNAL OF PHARMACOLOGY, ELSEVIER SCIENCE, NL, vol. 897, 3 February 2021 (2021-02-03), XP086511758, ISSN: 0014-2999, [retrieved on 20210203], DOI: 10.1016/J.EJPHAR.2021.173925**
- **HOSTRUP MORTEN ET AL: "The beta 2 -adrenergic receptor - a re-emerging target to combat obesity and induce leanness?", THE JOURNAL OF PHYSIOLOGY, vol. 600, no. 5, 22 October 2021 (2021-10-22), GB, pages 1209 - 1227, XP093021962, ISSN: 0022-3751, DOI: 10.1113/JP281819**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 429 658 B1

- TAN SIN YEE ET AL: "Type 1 and 2 diabetes mellitus: A review on current treatment approach and gene therapy as potential intervention", DIABETES & METABOLIC SYNDROME: CLINICAL RESEARCH & REVIEWS, ELSEVIER, AMSTERDAM, NL, vol. 13, no. 1, 10 October 2018 (2018-10-10), pages 364 - 372, XP085577632, ISSN: 1871-4021, DOI: 10.1016/J.DSX.2018.10.008
- WILLIAMS DAVID M. ET AL: "Drug Therapy in Obesity: A Review of Current and Emerging Treatments", DIABETES THERAPY, vol. 11, no. 6, 15 April 2020 (2020-04-15), pages 1199 - 1216, XP093022149, ISSN: 1869-6953, DOI: 10.1007/s13300-020-00816-y
- DO VALE GABRIEL T. ET AL: "Three Generations of [beta]-blockers: History, Class Differences and Clinical Applicability", CURRENT HYPERTENSION REVIEWS, vol. 15, no. 1, 29 January 2019 (2019-01-29), pages 22 - 31, XP093022153, ISSN: 1573-4021, DOI: 10.2174/1573402114666180918102735

## Description

## Introduction

Diabetes and obesity are two widespread diseases that could strongly profit from a precisely controlled reduction of sugar uptake from the gut. Extensive evidence has been reported that the main cells in the intestinal epithelium, the enterocytes, have mechanisms to sense the presence of sugar in the gut and respond by increasing sugar uptake from the gut lumen (1). Most of this uptake is mediated by the $Na^+$-glucose linked transporter SGLT1, located in the apical membrane of enterocytes facing the intestinal lumen (2). Sugar-induced upregulation of SGLT1 activity and corresponding stimulation of glucose uptake from the gut is well-documented (3-6). A wide range of sugars, including non-permeable sugar analogs, is able to elicit the response, which requires no metabolism of transported sugars (7). Evidence was obtained that the sugar sensing involved a G protein-coupled receptor (GPCR) acting through the cAMP-Protein Kinase A (PKA) pathway (4, 5, 8). Sweet taste receptors were shown to be involved in sugar-induced stimulation of glucose uptake by enterocytes (9, 10) and carnivores lacking a functional sweet taste receptor, like cats, also lack glucose-induced stimulation of glucose transport in response to dietary sugar (11, 12). Later work revealed that expression of the sweet taste receptors is confined to certain intestinal neuroendocrine cells (representing less than 1% of intestinal epithelial cells) to regulate the release of incretin peptide hormones that somehow stimulate glucose uptake in the absorptive enterocytes (13, 14). How these peptide hormones control SGLT1 activity in the neighbouring enterocytes has remained unclear (15). Over 99% of intestinal epithelial cells are absorptive enterocytes that express SGLT1, whose activity is regulated by the glucose level in the gut (4). The increase in enterocyte glucose transport starts within seconds of exposure to sugars, which appears too fast for the proposed neuroendocrine loop (9), but rather suggests an additional cell-autonomous process in the enterocyte itself. Moreover, incretin secretion is also stimulated by lipids and proteins (16), which do not trigger increased glucose transport, suggesting an additional requirement for sugar regulation of SGLT1. Thus, there seems to exist an elusive sugar-specific sensor in the enterocytes regulating sugar uptake via control of SGLT1 activity. Morten Hostrup et al., 2021, J. Physiol., 600(5) 1209, describe the $beta_2$-adrenergic receptor as target to combat obesity. Sin Yee Tan et al., 2018, Diabetes Metab. Syndr., 13(1) 364, describe current treatment approaches for type 1 and 2 diabetes mellitus. David M. Williams et al., 2020, Diabetes Ther., 11(6) 1199, describe current and emerging treatment approaches for obesity.

Description of the Invention

The invention is defined by the claims. In a first aspect, the invention relates to a β2-adrenergic receptor (β2-AR) antagonist for use in the treatment of at least one of diabetes and obesity.

We have previously discovered a glucose/sucrose-sensing GPCR, Gpr1, in the yeast *Saccharomyces cerevisiae.* It regulates energy and reserve carbohydrate metabolism through the cAMP-PKA signaling pathway (17, 18) in response to glucose and other rapidly-fermented sugars. Similar sugar-sensing receptors belonging to the same GPCR subfamily and also acting through the cAMP-PKA pathway have been found in *Schizosaccharomyces pombe* (19), *Candida albicans* (20, 21), *Neurospora crassa* (22), and homologs are present in many other fungi (23). The physiological function of the GPCR Gpr1 in *S. cerevisiae* resembles the regulation of energy and reserve metabolism in certain mammalian cell types, e.g. liver and fat cells, by the β2-adrenergic receptor ($\beta_2$-AR) in response to epinephrine (24, 25). The $\beta_2$-AR is a major drug target and likely the best-characterized mammalian GPCR (26, 27). Its three-dimensional structure has been determined for different agonist- and $G_s$-protein-associated states (28-32). The $\beta_2$-AR belongs to a family of closely related adrenergic receptors responsive to catecholamines, like epinephrine and norepinephrine (33). Antagonists and agonists with varying specificity for the different α- and β-adrenergic receptor subtypes have been identified, and many of those are in clinical use as drugs, e.g. in cardiovascular and respiratory medicine (27, 34-36).

Following our discovery of the yeast sugar-sensing GPCR Gpr1, we have explored the possible presence of a sugar-sensing GPCR in the apical membrane of mammalian enterocytes. Both cell types are exposed to highly variable nutrient conditions in their environment, and both also respond to glucose with specific GPCR- and cAMP/PKA-dependent responses controlling carbohydrate metabolism. As shown in the present disclosure, we surprisingly found that the $\beta_2$-AR is abundantly present in the apical membrane of enterocytes, a location more suggestive of a function as sensor for components like nutrients in the gut lumen, rather than for catecholamine sensing. The use of two different heterologous expression systems subsequently revealed that the $\beta_2$-AR responds to multiple sugars at concentrations (5-100 mM) found in the gut after a meal. Experiments with everted sacs prepared from rat intestine, and determination of blood glucose levels after *in vivo* administration of glucose together with a non-bioavailable $\beta$-AR antagonist confirmed that the $\beta_2$-AR mediates sugar-induced stimulation of glucose uptake from the gut lumen. The confirmation by STD-NMR of direct binding of glucose at mM concentrations to the $\beta_2$-AR, hints at a possible more universal role of the $\beta_2$-AR as glucose-sensing receptor in other mammalian cell types. Our results suggest that the $\beta_2$-AR evolved from an ancient sugar receptor in an ancestral primitive unicellular eukaryote, and has retained its sugar-binding capacity to function as a sugar-sensor in the gut and possibly in other mammalian tissues.

## Results

### The $\beta_2$-AR is expressed at the apical membrane of enterocytes.

We have first tested which GPCRs are expressed in enterocytes (Fig. 17), in order to investigate subsequently those located in the apical membrane for a possible sugar-sensing function. We first used PCR amplification of selected GPCRs(37) starting from cDNA derived from mouse proximal duodenal scrapings, and from the intestinal STC-1 cell line. Twenty-five GPCRs could be amplified in this way from both sources (Fig, 17). We then performed microarray gene expression analysis of STC-1 cells and SYBR Green qPCR for confirmation. We identified 22 GPCRs and one additional selected GPCR, not present on the microarray (Fig. 17). Finally, we confirmed by qPCR the expression of 18 GPCRs using RNA from STC-1 cells (Fig. 17).

Next, we used immunohistochemistry on human intestinal biopsy samples to determine if any of the GPCRs were located in the apical membrane. We first tested GPCRs for which antibodies were available. Among the first GPCRs tested: GPR105, GPR1, GPR120, GPRC5C and the $\beta_2$-AR, only the last one surprisingly showed staining at the apical cell membrane, which was very strong and highly reproducible, compared to the much weaker staining with some of the other GPCRs (Fig. 1a). These either showed staining at the basolateral membrane or showed staining too weak for reliable detection. Some much weaker cytoplasmic staining on the basal side of the enterocytes was also observed for the $\beta_2$-AR, possibly in organelles of the secretory pathway (Fig. 1b). Because this location (facing the luminal side of the gut) is highly unusual for a receptor sensing a hormone distributed through the bloodstream and since the $\beta_2$-AR is involved in glucose and energy homeostasis, and couples to the cAMP-PKA signaling pathway, we further concentrated on the possibility that the $\beta_2$-AR might have an additional glucose-sensing function. The apical localization of $\beta_2$-ARs in enterocytes was confirmed with paraffin-embedded tissue sections of proximal mouse intestine (Fig. 1c), and showed near complete overlap with immunoreactivity for SGLT1, which is specifically located in the apical membrane (Fig. 1d,e). This, however, does not necessarily indicate that the two proteins would physically interact. Human and mouse $\beta_2$-AR show 87% sequence identity (https://www.uniprot.org/align/A20200503DA437993067D6F643265E5E763500BDE D0207523). Our immunohistochemistry results demonstrating the apical localization of the $\beta_2$-AR in intestinal epithelial cells are consistent with previous results in the literature. Singh et al. (2009) reported the mRNA expression level for $\beta_2$-AR in murine duodenal epithelial cells, as well as the strong enrichment of $\beta_2$-ARs by Western blotting in the apical brush border membrane compared to the total cell lysate. Both $\beta_2$-AR bands (monomer and dimer) were completely blocked in the Western blot using the immunising peptide, showing the specificity of the antibody(38). Similar results were reported for colonic mucosa (39).

**The $\beta_2$-AR responds to sugars and this response is blocked by $\beta$-AR antagonists.** We subsequently constructed a stably transfected Flp-In-293 kidney cell line overexpressing a human $\beta_2$-AR, in which receptor activation is coupled via $G\alpha16$ to phospholipase-C-mediated $Ca^{2+}$ release from intracellular stores, as detected by Fluo4 fluorescence. These cells responded as expected to catecholamine agonists, like epinephrine, and the response was blocked by classical $\beta$-AR antagonists (34, 35), like nadolol (β1,2-antagonist), labetalol (α,β-antagonist), propranolol (β1,2-antagonist) and ICI 118,551 (β2-antagonist) (Fig. 2a). Control experiments with HEK293 cells without transfection of the $\beta_2$-AR failed to show a calcium response with epinephrine, indicating that any endogenous $\beta_2$-AR expression was too low to give a significant response with our reporter system (Fig. 8). Addition of ICI 118,551 alone did not trigger a significant change in the fluorescence read-out, with epinephrine (5 nM) and Hank's Buffered Salt Solution (HBSS) used as positive and negative control, respectively (Fig. 9). Using this reporter system, we next evaluated whether sugars were able to trigger activation of the $\beta_2$-AR. Glucose triggered a rapid, but less pronounced response compared to epinephrine, that was completely inhibited by the aforementioned $\beta$-AR antagonists (Fig. 2b). ICI 118,551 was equally effective in blocking the epinephrine and glucose responses at 30 μM. Other sugars also activated the $\beta_2$-AR in order of decreasing intensity: maltotriose, glucose, maltose, xylose, trehalose, fructose and 2 deoxy-D-glucose. all used at 50 mM (Fig. 2c), suggesting different degrees of agonist potency. Glucosamine (50 mM) provoked very little effect. ICI 118,551 inhibited all sugar responses at 1 μM. The sugar concentrations are in the same range as their estimated concentrations after a meal in the gut of mammals, including humans (40, 41). Rapid cell uptake of glucose at low concentrations hampered accurate determination of the apparent affinity of the $\beta_2$-AR for glucose. For maltotriose, a sugar not taken up by the cells, an $EC_{50}$ of ±10 mM was determined (Fig. 2d).

To exclude that the genetically engineered $G\alpha16$/phospholipase-C mediated $Ca^{2+}$ signaling was in some way affecting the specificity of the $\beta_2$-AR, we also used a cAMP-dependent reporter system. For that purpose, we expressed $\beta_2$-ARs in *Xenopus* oocytes, together with a reporter system consisting of the PKA-dependent cystic fibrosis transmembrane conductance regulator (CFTR) $Cl^-$channel. Thus, parallel changes in inward transmembrane current and conductance of the membrane served as readout for receptor activity. Here as well, both epinephrine and different sugars activated the $\beta_2$-AR (Fig. 2e,f) while the $\beta$-AR antagonist propranolol inhibited the responses (Fig. 2e). There was neither

an epinephrine nor a sugar response in control *Xenopus* oocytes injected with vector mRNA, while addition of forskolin (activator of adenylate cyclase) together with IBMX (inhibitor of cAMP phosphodiesterase) produced a strong response (Fig. 10).

**Sugars do not compete with antagonist binding to $\beta_2$-AR.**

We next investigated whether the two types of agonists, epinephrine and sugars, bind to the same ligand-binding site. For that purpose, we determined binding of $^{125}$I - cyanopindolol, a $\beta$-AR antagonist, to isolated cell-free membrane vesicles containing recombinant human $\beta_2$-AR (Perkin Elmer Cat. nr. RBHBE2M) in the absence or presence of glucose. We did not observe any inhibition of $^{125}$I -cyanopindolol binding in the presence of glucose (Fig. 11a), suggesting that the two compounds do not bind to the same site on the $\beta_2$-AR, in spite of our previous observation that the glucose response was blocked by a range of classical $\beta$-AR antagonists (Fig. 2b). Surprisingly, we even observed an increase up to approximately 30% of $^{125}$I-cyanopindolol binding with increasing glucose concentrations (Fig. 11a). This suggests that glucose binding affects the structure of the epinephrine-binding site. The affinity of this glucose response, however, should not be confused with the affinity by which $\beta_2$-AR triggers activation of the $G_s$-protein upon binding of glucose alone. In that case indeed there is no other compound bound into the epinephrine binding site. The stimulation of cyanopindolol binding by glucose merely confirms that glucose physically interacts with $\beta_2$-AR although it also suggests that glucose could modulate epinephrine binding *in vivo.* We also tested the effect of glucose + NaCl because of a previous report that NaCl enhanced $^{125}$I-cyanopindolol binding (42), which we also observed with increases up to roughly 35% (Fig. 11b). Glucose + NaCl caused an increase up to approximately 40% (Fig. 3a). On the other hand, increasing concentrations of isoproterenol caused the expected gradual inhibition of $^{125}$I-cyanopindolol binding (Fig. 3b). This appears to indicate that sugars and epinephrine may not bind precisely to the same site, although binding of the sugar does appear to affect the structure of the epinephrine binding site. This agrees with the demonstration of conformational coupling between the epinephrine binding site and an allosteric binding site for other ligands on the extracellular surface of the $\beta_2$-AR (43). Because both the epinephrine and sugar responses of the $\beta_2$-AR are inhibited by classical $\beta$-AR antagonists (Fig. 2a,b), the two binding sites may be located close to each other or may affect each other over a greater distance by the reported conformational coupling upon ligand binding. In recent years, an increasing number of allosteric ligands has been discovered for GPCRs. They bind to allosteric sites, as opposed to the orthosteric ligands that bind to the binding site for the native ligand (44). On the other hand, it is premature at present to suggest that glucose and adrenergic agonists would bind to two entirely different sites, or that glucose would bind to an allosteric site rather than the orthosteric site, especially because of the consistent inhibition of all sugar responses by $\beta$-AR antagonists.

**Some $\beta$-AR antagonists differentially inhibit $\beta_2$-AR responses to epinephrine and sugar.**

Because the two types of ligands, epinephrine and sugar, may bind to sites on the $\beta_2$-AR that differ at least to some extent, we tested several $\beta$-AR antagonists in search of compounds with a possible differential effect on the two ligand-binding sites. Responses to all sugars tested, i.e. maltotriose, glucose, maltose, xylose, trehalose, fructose and 2-deoxy-D-glucose were inhibited by β2-antagonists: 1 μM ICI 118,551 (Fig. 4), propranolol, labetalol and nadolol (Fig. 12). In contrast, β1-specific antagonists did not inhibit the epinephrine response (34, 35) (Fig. 13) but differentially affected the response to sugars. Metoprolol (Fig. 13), acebutolol or atenolol (Fig. 14) at 200 μM completely inhibited the response to 70 mM glucose, but only slightly affected the response to 70 mM maltose, 2-deoxy-glucose or xylose (Fig. 13; Fig. 14). These results are consistent with binding of epinephrine and glucose to two different sites. The stronger antagonism of β1-antagonists for the glucose response, at least under the conditions of our experiments, compared to the other sugars suggests that glucose binds somewhat differently to the $\beta_2$-AR compared to the other sugars tested.

**Everted sacs of rat intestine show sugar-induced, $\beta_2$-AR -dependent stimulation of glucose uptake.**

The importance of the apical $\beta_2$-AR for sugar-induced stimulation of glucose transport in the gut was evaluated with an everted sac model, in which facilitated glucose transport occurs from the external medium outside the sac, facing the intestinal mucosa, to the inside of the sac flanked by the serosa (45, 46). Glucose from the external medium (5 mM) indeed accumulated inside the everted sac at a rate that increased with longer incubation times (Fig. 5a). Glucose uptake into the everted sacs was inhibited for more than 90% by phlorizin (100 μM) and by LX4211 (2 μM), inhibitors of both SGLT1 and SGLT2 (Fig. 15). When epinephrine (10 μM) was added to the external medium, the amount of glucose accumulated after 10 min in the sac increased from 440 ± 132 to 833 ± 255 μM. This increase was completely prevented by the $\beta_2$-AR antagonist ICI 118,551 (10 μM), while phlorizin (100 μM) inhibited both basal and enhanced glucose uptake (Fig. 5b). This supports a role of the apical $\beta_2$-AR in stimulating glucose uptake through SGLT from the gut. Colchicine (5 μM), a microtubule-disrupting agent that blocks translocation of SGLT1 from a cytoplasmic pool to the apical plasma membrane (47), completely blocked epinephrine stimulation of glucose transport, but not basal glucose uptake, as was observed with

phlorizin (100 μM) (Fig. 5c). This suggests that epinephrine acts by increasing translocation of SGLT1 from a cytoplasmic pool to the enterocyte apical surface. Myristoylated PKI 14-22 amide (mPKI) (1 μM), a selective cell-permeable protein kinase A (PKA) inhibitor, partially inhibited epinephrine stimulation of glucose transport, as opposed to the complete inhibition by ICI 118,551 (Fig. 5d). The high mPKI concentration we used is known to cause complete inhibition of PKA in *ex vivo* preparations (48), and higher concentrations of mPKI also did not further reduce the response. If mPKI is able to cause complete inhibition of PKA in the epithelial cells of the everted sacs, these results would suggest that also PKA-independent signaling is involved in epinephrine stimulation of glucose transport through the $\beta_2$-AR.

Next, we investigated whether sugar sensing by apical $\beta_2$-ARs could enhance glucose transport into the everted intestinal sacs. To avoid interference with glucose uptake by SGLT1 into the everted sacs, the $\beta_2$-AR was stimulated with mannose. Mannose is transported by SGLT4 but is not a substrate of SGLT1 (49, 50). Hence, any stimulation of glucose uptake through SGLT1 by mannose has to act through another target. We previously showed that mannose is also one of the sugars that potently stimulates the $\beta_2$-AR, as measured in stably transfected Flp-In-293 cells incubated throughout in 5 mM glucose, and ICI 118,551 completely blocked the mannose response (Fig. 5e). Five mM glucose or mannose equally potentiated the response of $\beta_2$-ARs to 5 nM epinephrine, compared to sugar-free medium (to which 4 mM glutamine was added as energy source). Whereas 5 nM epinephrine still stimulated the $\beta_2$-AR in sugar-free medium, although significantly less compared to medium containing 5 mM glucose or 5 mM mannose, mannose could only activate the $\beta_2$-AR in cells pre-incubated with glucose or mannose, and not in sugar-free medium (Fig. 5f). In everted sacs, both glucose transport and the stimulation of glucose transport by mannose were completely inhibited by LX4211, a dual inhibitor of SGLT1 and SGLT2 (Fig. 5g). The stimulation of glucose transport by mannose was abolished by the $\beta_2$-AR-antagonist ICI 118,551 (Fig. 5h), indicating that mannose stimulates glucose transport through $\beta_2$-ARs. Thus, sugar sensing by apical $\beta_2$-ARs in enterocytes stimulates glucose absorption through SGLT1. ICI 118,551 (10 μM) by itself did not have any effect on glucose transport in everted sacs (Fig. 16). In this experiment, the everted sacs were incubated in a lower glucose concentration of 2.5 mM compared to the earlier experiments with 5 mM glucose, to reduce activation of $\beta_2$-ARs by glucose.

**STD-NMR demonstrates direct glucose binding to the $\beta_2$-AR.**

To confirm direct binding of glucose to the $\beta_2$-AR, Saturation Transfer Difference (STD) NMR spectroscopy was performed with a membrane preparation containing the $\beta_2$-AR, derived from the transfected Flp-in-293 cell line, and a control membrane preparation, with minimal inherent expression of the $\beta_2$-AR, derived from the parent untransfected HEK293 cell line, similar to previous work showing direct binding of sugars to the human sweet taste receptor (51, 52). STD NMR has proven to be a powerful technique to study ligand binding to membrane proteins (53, 54). The STD spectrum of the $\beta_2$-AR -containing membranes in the presence of 10 mM glucose was very pronounced, while the spectrum of membranes lacking $\beta_2$-ARs but with the same glucose concentration had only a very small amplitude (Fig. 6). The difference STDD spectrum provides clear evidence for direct binding of glucose to the $\beta_2$-AR, since the saturation transfer can only take place upon physical binding of glucose to the target, the $\beta_2$-AR, which is the only different component between sample and control membranes. This suggests that glucose interaction is not restricted to apical $\beta_2$-ARs in intestinal epithelial cells, but that $\beta_2$-ARs in general can interact with glucose and thus may act as a glucose sensor (or at least respond to glucose) also elsewhere in the body.

**Inhibition of apical $\beta_2$-AR in gut epithelium with a non-bioavailable β-antagonist reduces blood glucose levels after an oral glucose bolus**

Finally, we have performed *in vivo* experiments in which rats were orally administered a glucose bolus together with a non-bioavailable β-antagonist: CD3-403 (Fig. 7a). This compound was specially designed for our work based on the structure of ICI 118,551, which was modified by addition of a hydrophilic group and elimination of a hydrophobic group to ensure minimal permeability through biological membranes. This was confirmed by a Caco-2 permeability assay (Fig. 18). CD3-403 inhibited with an $IC_{50}$ of 46 nM the calcium response elicited by 5 nM epinephrine in Flp-In-293 cells stably transfected with *ADRB2* and *GNA15* (Fig. 7b,c). The CD3-403 compound was synthesized at 100 mg scale for oral administration in rats (GVK Biosciences Private Limited). The glucose bolus (2g/kg or 4 g/kg body weight) was administered by oral gavage to sets of three rats each, in the absence or presence of 5 μM (0.05 mg/kg) CD3-403. Glucose levels were measured in blood withdrawn from the tail vein just before, as well as 15, 30, 60 and 120 min after oral administration of the glucose bolus. The increase in blood glucose concentration after the glucose load is shown, normalized to the 0 min blood glucose concentration, which was set at 100 %. In the case of glucose 2 g/kg, the presence of the CD3-403 compound caused a reduced increase in blood glucose level, which was significant at 60 min (Fig. 7d). For the experiment with glucose 4 g/kg, we also saw a trend of decreased glucose concentration after treatment with CD3-403 (Fig. 7e). For the experiment with glucose 2 g/kg, the area under the curve (AUC) was also calculated by setting 100% as a baseline. The CD3-403 compound significantly reduced the glucose increase (5907 ± 2195; mean ± SD) compared to the

glucose 2 g/kg control group (8056 ± 2990; mean ± SD) (Fig. 7f).

**Discussion**

Our work provides strong evidence for an additional function of the $\beta_2$-AR as glucose receptor. Although the different results obtained might each be subject individually to more or less likely alternative explanations, the comprehensive body of evidence when taken together makes a compelling and consistent case that $\beta_2$-ARs in the apical enterocyte membrane sense the level of glucose in the gut to stimulate its uptake through SGLT1. This additional function is unexpected because the $\beta_2$-AR is one of the best-characterized GPCRs. It has served as a leading model for elucidation of the mechanisms involved in GPCR signaling, the three-dimensional structure of GPCRs and the conformational changes triggered by ligand binding (26, 28-32). As a major drug target, its pharmacology has been studied in great detail (27, 34, 35). It is the strong expression of $\beta_2$-ARs in the apical plasma membrane of the epithelial cells facing the lumen of the gut, a highly unusual location for a receptor sensing a hormone distributed through the bloodstream, that led us to the current discovery. Interestingly, strong expression of $\beta_2$-ARs in the apical membrane of the epithelium lining the proximal tubule of the nephron had already been reported (55), as well as stimulation of glucose reabsorption from the kidney tubules by epinephrine (56). This apical location was interpreted, however, to allow for a response to epinephrine leaking from the blood into the urine primary filtrate. Similarly, $\beta_2$-AR in the apical membrane of enterocytes might be activated fortuitously by epinephrine leaking through the tight junctions of the gut epithelium, although this is likely limited to pathological conditions given the lack of permeability in the tight junctions for small molecules like sugars and amino acids (57). Moreover, such explanations do not contradict our results demonstrating that enterocyte apical $\beta_2$-AR functions as a glucose receptor for stimulation of sugar uptake from the gut. Multiple studies on intestinal epithelial cells have reported results consistent with those in our work. Expression of β-AR subtypes in the gut has been demonstrated by [$^{125}$I] cyanopindolol binding and its competition with adrenoreceptor antagonists (58). mRNA expression and apical membrane location of $\beta_2$-ARs was reported in a study on the regulation of apically located CFTR in murine duodenal epithelia (38). Moreover, $\beta_2$-AR agonists activate CFTR in intestinal organoids through cAMP signaling (59), and optimal activity of CFTR in Caco-2 human colon carcinoma cells is dependent on the presence of glucose (60). Coupling of the $\beta$2-AR to adenylate cyclase has been demonstrated in Caco-2 cell membranes based on the increase in cAMP level upon addition of different agonists (61).

The SGLT2, and to a lesser extent SGLT1, present in the apical membrane of the kidney tubule, are responsible for the reabsorption of glucose from the urine primary filtrate (62). Their expression is enhanced by luminal glucose (63) and their plasma membrane insertion and/or activity are enhanced by elevated cAMP-PKA signaling, as in intestinal (62, 64) and ovary (65) epithelial cells. Similar regulation of intracellular SGLT1 and SGLT2 trafficking to the plasma membrane both in small intestinal mucosa and kidney tubules by RSCIA1 (RS1) has also been reported (64, 66). Our results strongly suggest that $\beta_2$-ARs may sense glucose also in kidney tubule epithelium to stimulate glucose recovery from the urine primary filtrate. Such a role makes more physiological sense than regulation by leaked catecholamines in the primary filtrate.

Although the main acute effect of $\beta_2$-AR stimulation is an increase in blood glucose concentration due to glycogen mobilization in the liver and inhibition of glucose disposal by insulin-dependent tissues, there have been many reports on stimulation of glucose uptake in different tissues by activation of adrenergic receptors independently of insulin (67-77). In astrocytes, adrenergic stimulation of the $\beta_2$-AR acts through GLUT1 by coupling to Gs and activation of the adenylate cyclase pathway (78), while in rat skeletal muscle cells it causes increased translocation of GLUT4 to the plasma membrane (73, 79, 80). Our results show that stimulation of glucose uptake through activation of $\beta_2$-ARs does not only appear to target GLUT facilitated diffusion carriers in diverse somatic cell types, but also targets the active glucose transporter SGLT1 in intestinal epithelial cells. Involvement of the cAMP-PKA signaling pathway may be a common theme in adrenergic stimulation of glucose uptake.

It has been reported that knock-out mice in the sweet taste receptor T1R2 + T1R3 or in gustducin lack the secretion of gut hormones triggered by dietary sugars and also lack sugar-induced upregulation of SGLT1 expression and glucose absortive capacity (5, 10, 81), while intestinal sweet taste receptor stimulation upregulates SGLT1 (82). This sweet taste sensing system is expressed in enteroendocrine cells, and has to communicate with the enterocytes in which SGLT1 mediates bulk sugar uptake. Its inactivation apparently also disables the $\beta_2$-AR-based sugar-sensing system in the enterocytes identified in this disclosure. An explanation may be that the enteroendocrine cells regulate the sensitivity of the $\beta_2$-AR-based sugar-sensing system in the enterocytes, particularly over the longer term (hours to days). Inactivation of the enteroendocrine sweet taste sensing system may thus make the $\beta_2$-AR sugar-sensing system in some way insensitive. This could happen at different levels, such as sorting of SGLT1 to the apical membrane and/or post-translational modification of SGLT1 or any component in the signaling pathway from $\beta_2$-AR to the SGLT1 sorting mechanism. The increase of SGLT1-mediated glucose transport is based on different components with a divergent time-dependency, whose regulation may well be distinct (83). Within seconds after exposure to glucose, pre-existing SGLT1 are translocated from a cytoplasmic pool to the cell surface membrane of the enterocytes. Those surface

transporters then accelerate their glucose transport activity, presumably due to phosphorylation as reported for SGLT1 in Chinese hamster ovary cells (65). Later, synthesis of new SGLT1 protein is induced. Finally, the basal SGLT1 expression level may change over days or weeks depending on the food composition, as has been observed when herbivores are weaned and switch from a milk to a grass diet (3, 4). Our study has focused on the short-term SGLT1 changes, whereas many studies of the enteroendocrine pathways have focused on longer-term effects.

GPCRs must be derived in evolution from proteins in unicellular organisms that lack the elaborate, extracellular endocrine signaling pathways present in higher eukaryotes, and nutrient-sensing GPCRs are prime candidates in this respect (84-87). Although members of the main GPCR families have been found in the most primitive metazoan organisms (85), the very poor sequence conservation between GPCRs makes it difficult to connect fungal and animal GPCRs in evolution (87). Our results suggest that $\beta_2$-ARs evolved from an ancient glucose receptor, an ancestral GPCR present in a primitive unicellular eukaryote, similar to the Gpr1 glucose-sensing GPCR present in yeast and other fungi (17, 18, 23). Since sequence conservation between GPCRs is very limited in general, and especially between distant relatives like the yeast and mammalian GPCRs (88), it is not possible at this point to make a meaningful prediction of a putative common glucose binding site. $\beta_2$-AR immunoreactivity is abundant in the pharynx rim of the unicellular protozoon *Paramecium,* and was proposed to function as a nutrient sensor (89). It undergoes isoproterenol-induced desensitization by endocytosis, possibly initiated through phosphorylation by a homolog of the human β-adrenergic receptor kinase (βARK2, GRK3) (90). Expression of a $\beta_2$-AR homolog in *Paramecium* was confirmed by RT-PCR and Northern blot analysis (91), and several adrenergic receptor orthologous genes have been annotated as such in the *Paramecium* genome (https://paramecium.i2bc.paris-saclay.fr/cgi/tool/search). Nascent phagosomes are formed at the pharynx rim, and β-adrenergic agonists stimulate phagocytosis in *Paramecium,* a response blocked by β-AR antagonists, and potentiated by forskolin, an activator of adenylate cyclase (92, 93). Our results suggest that the *Paramecium* $\beta_2$-AR homolog may indeed function as a sugar sensor, consistent with its localization at the pharynx rim and its involvement in triggering phagocytosis. Radioligand binding studies also provided evidence for the presence of β-AR s in the unicellular protozoon *Trypanosoma cruzi* (94).

The STD-NMR results provide clear molecular evidence that glucose directly binds to the $\beta_2$-AR, since the saturation transfer can only take place upon physical binding. The lack of competition between glucose and cyanopindolol for binding to the $\beta_2$-AR (Fig. 3a, Fig. 11a) suggests that the binding sites are different. On the other hand, the inhibition of the glucose-induced response by classical $\beta_2$-AR antagonists (Fig. 2b) and the stimulation of cyanopindolol binding to the $\beta_2$-AR by glucose (Fig. 3a, Fig. 11a), indicates that binding of a molecule into one of the two binding sites affects the other binding site and that therefore the two binding sites might be in close interaction and possibly close proximity. Further analysis of sugar binding affinity and specificity with purified $\beta_2$-ARs and determination of the precise binding site in relation to that of $\beta_2$-AR agonists and antagonists, goes beyond the scope of the present work, but should be a major focus in future research. This should also include the precise interaction of $\beta_2$-AR with Gs proteins in response to glucose and epinephrine, as well as the possible sugar-sensing function of the other members of the β-AR family. In the glucose-sensing yeast GPCR, Gpr1, evidence was reported for direct interaction of glucose with TMD VI (18), a transmembrane domain involved in binding of small ligand molecules in many GPCRs, including the $\beta_2$-AR (95). During its evolutionary development into a hormone receptor, the $\beta_2$-AR has apparently retained its ancient glucose-sensing function. The unexpected localization of $\beta_2$-ARs in the apical membrane of intestinal epithelial cells, making little physiological sense for a hormone receptor, has enabled us to identify this function. The $\beta_2$-AR is used in enterocytes to sense luminal sugar and regulate its uptake from the gut. Interestingly, both the $\beta_2$-AR and yeast Gpr1 use the cAMP-PKA signaling pathway to control storage carbohydrate levels and sugar catabolism (96). Our results also explain previous observations that perfusion of rat small intestine with epinephrine significantly increases transport of glucose from the gut lumen to the blood (97, 98). This is associated with an increase in SGLT1 protein and phlorizin binding, and was also elicited by perfusion with dibutyryl-cAMP (98). Epinephrine is a water-soluble compound with a very low membrane permeability coefficient of 2.7 $\pm 1.5 \times 10^{-6}$ cm/sec (99), which precludes any significant passive diffusion through membranes. Hence, epinephrine should in principle not be able to reach the baso-lateral membrane of the epithelial cells when administered in the gut lumen. Aschenbach *et al.* (100) reported stimulation of SGLT1-mediated glucose uptake in isolated ovine ruminal epithelia by several $\beta_2$-AR agonists. Stimulation by forskolin, an activator of adenylate cyclase, and inhibition by the PKA inhibitor H89 supported involvement of cAMP-PKA signaling. These observations are consistent with the presence of $\beta_2$-ARs in the apical membrane, and cAMP signaling as mediator of enhanced SGLT1 expression and glucose uptake as a result of $\beta_2$-AR stimulation. Our discovery that the $\beta_2$-AR can function as a sugar receptor on the apical side of the intestinal epithelial cells provides a logical explanation for upregulation of SGLT1 and glucose uptake elicited by perfused epinephrine. Future research should study in more detail the molecular mechanisms involved in $\beta_2$-AR-mediated upregulation of SGLT1 at the transcriptional and post-translational level, including SGLT1 intracellular trafficking and ligand-induced endocytosis, as well as the composition of the signaling pathway(s) involved and their possible interaction with incretin hormones, and other mechanisms of neuroendocrine signaling.

It would be useful to complement the present work with genetic experiments in mice lacking the $\beta_2$-AR and testing for the rate of glucose transport from the gut to the blood. However, given the complex role of the $\beta_2$-AR in glucose homeostasis in the mammalian body, acting in different ways on glucose mobilization from reserve tissues and glucose

sequestration in other tissues, it would be imperative to perform such genetic experiments with tissue-specific knock-out mice lacking the $\beta_2$-AR specifically in the intestinal epithelium. Otherwise, interpretation of the results would likely be very cumbersome and inconclusive. On the other hand, we would like to emphasize that the use of a non-bioavailable beta-blocker to specifically inhibit the sensing of glucose by the $\beta_2$-AR in the gut epithelium is a much stronger and more reliable scientific argument than the use of a tissue-specific knock-out because of the many possible complications that the latter may cause, including upregulation of related adrenergic receptors, abolishment of regular $\beta_2$-AR interactions and possible adverse effects on intestinal epithelial cell development, as well as the general shortcoming that elimination of a physiological response by deletion of a receptor protein does not necessarily imply that the sensing function of the receptor is directly responsible for the absence of the response.

Another aspect that is likely different between $\beta_2$-ARs in the apical membrane of enterocytes compared to the plasma membrane of other cell types is the degree and nature of post-translational modification, in particular glycosylation. The extracellular domains of apical membrane proteins in enterocytes are uniquely and heavily glycosylated (101). This glycosylation changes during their differentiation as they migrate upwards from the crypt base to the villus tip, and it is also influenced by the composition of the gut content (102). Glycosylation often affects protein functionality (103) and future research thus will have to determine whether changes in $\beta_2$-AR glycosylation affect the affinity and/or specificity of the sugar-sensing function of apical $\beta_2$-AR in the enterocytes.

Reduction of glucose uptake from the gut by SGLT1 inhibitors is being explored for treatment of diabetes and obesity (104, 105). Enhanced uptake of glucose from the gut by upregulation of SGLT1-activity was shown to cause obesity in mice (106). The expression of SGLT1 and other sugar transporters was found to be upregulated in gut epithelium from diabetic patients, suggesting that an increased capacity to absorb sugars from the gut may reinforce diabetic syndromes (64, 107, 108). Similar findings were made for obese patients (109). Apical $\beta_2$-ARs in enterocytes may constitute a more attractive target than SGLT1 for partially blocking postprandial glucose uptake from the gut in diabetic and obese patients, since antagonism of these $\beta_2$-ARs only blocks the sugar-induced stimulation of glucose uptake and not total glucose uptake, as is the case with SGLT1 antagonists. The latter easily results in osmotic diarrhea, enhanced microbial activity and flatulence, as a consequence of excessive gut sugar levels (110, 111). Obviously, $\beta_2$-AR antagonists with limited oral bio-availability would be the drugs of choice for this purpose, so as to avoid interference with the $\beta_2$-AR in other tissues of the body. Our work has now shown that a non-bioavailable $\beta_2$-AR antagonist is able to reduce the increase in blood glucose level when administered together with an oral glucose bolus. This suggests that non-bioavailable $\beta_2$-AR antagonists could be useful in humans to lower the postprandial increase in blood glucose level, and therefore might be used to reduce glucose uptake in diabetic and obese patients. It has been reported previously that in an intraperitoneal glucose tolerance test in $\beta_2$-AR knock-out mice (112) or after introducing an intravenous blood glucose load together with a $\beta_2$-AR antagonist in rats (113) or diabetic patients (114), a much higher increase in blood glucose level was observed compared to controls. This supports our conclusion that the reduction in blood glucose level observed in our *in vivo* experiments cannot be due to systemic inhibition of $\beta_2$-ARs, but must rather result from inhibition of the apical $\beta_2$-ARs in the gut epithelium. Future research should study the effect of administration of non-bioavailable $\beta_2$-AR antagonists as well as agonists in different nutrient regimes on glucose homeostasis, body weight gain and levels of insulin, glucagon, ghrelin, GLP-1 and GIP incretins, and other hormones known to be linked to glucose homeostasis in the body, in healthy individuals as well as in diabetic and obese patients.

We have used experimental conditions that in our view would maximize the chance of detecting a significant effect on the blood glucose level by administration of a non-bioavailable $\beta_2$-AR antagonist. However, it is unclear what the main driving force was in evolution to establish and maintain a glucose receptor in the gut to stimulate glucose uptake. Was it to maximize high glucose uptake during sparse meals? Or was its main function to stimulate uptake of low glucose levels under malnutrition conditions? Future research will have to investigate the effect of non-bioavailable $\beta_2$-AR antagonists under a variety of feeding conditions, not only on blood glucose levels, but also on general glucose homeostasis and other relevant parameters, such as body weight gain.

Glucose-induced regulatory effects are quite common in mammalian tissues, which is not surprising in view of the crucial role of glucose as a nutrient throughout the body. It is presently unclear whether the $\beta_2$-AR may also serve as glucose receptor for regulation of glucose-controlled processes in cell types or tissues other than those investigated in this study. The glucose-sensing function of the $\beta_2$-AR might play a role in multiple ways in the complex regulatory network controlling glucose homeostasis in the body. For instance, glucose sensing by $\beta_2$-ARs may modulate epinephrine sensing in blood and other body fluids, as suggested by the glucose stimulation of [$^{125}$I]cyanopindolol binding to the $\beta_2$-AR (Fig. 3a, Fig. 11a), or may support feedback inhibition of $\beta_2$-ARs by high glucose levels through desensitization, stimulation of its endocytosis and/or prevention of its recycling (115, 116).

The glucose-sensing function of the $\beta_2$-AR may provide an explanation for some of the hitherto unexplained observations in human (patho)physiology related to $\beta_2$-AR function, such as unexpected negative side effects of β-AR antagonist therapy (117, 118) or unexplained differences in therapeutic outcomes between different β-AR antagonists (119, 120). The abnormally high glucose levels in diabetic patients may cause spurious activation (or desensitization) of $\beta_2$-ARs throughout the body, and be responsible for hitherto unexplained symptoms and complications of diabetes, like

the well-established correlation between diabetes, hypertension and heart failure (121). Although epinephrine is well known to increase blood glucose levels through stimulation of glycogen breakdown, this appears to be a short-lived effect. In the long term, epinephrine increases muscle glucose uptake (122, 123). Stimulation of glucose uptake by epinephrine has been documented in muscle upon chronic administration (124) and also in brown adipose tissue (125). Stimulation of glucose uptake by $\beta_2$-ARs may be relevant in other cell types as well. Particularly cells in which very active glucose uptake is critical, e.g. in cancer cells where the reasons for the strong correlation between $\beta_2$-AR expression and cancer aggressiveness (126), the frequent involvement of $\beta_2$-ARs in multiple carcinogenic processes (127), as well as the beneficial effect of $\beta$-AR antagonists on the recovery of cancer patients during chemotherapy (128), have remained enigmatic up to now. The $\beta_2$-AR has also been reported to show basal ligand-independent activity, whereas this property is considerably weaker in the closely related $\beta_1$-AR subtype (129). Since glucose is present in most experimental media and body fluids, this sugar may have contributed to the basal "ligand-independent" activity, particularly since ICI 118,551 (which blocks the $\beta_2$-AR response to glucose) can block this spontaneous activity (129).

In conclusion, we have discovered that the $\beta_2$-AR, a well-established catecholamine receptor, is located at the apical side of intestinal epithelial cells to serve as a sugar sensor for stimulation of glucose uptake by SGLT1 from the lumen of the mammalian gut. We demonstrate direct binding of glucose at physiological concentrations to recombinant $\beta_2$-ARs contained in membrane vesicles, suggesting that the $\beta_2$-AR may be able to exert a more general glucose-sensing function also in other cells and tissues, and in other organisms.

**Materials and Methods**

**PCR techniques.** The templates used were RNA extracts from sheep mucosal scrapings and from STC-1 cells, isolated at the laboratory of Prof. Shirazi-Beechey (University of Liverpool), sent on dry ice and stored at -80°C. First-strand cDNA synthesis was achieved using the RevertAid™ H Minus First Strand cDNA synthesis kit (Fermentas) according to the manufacturer's protocol, using 5 μg of purified RNA. Both oligo(dT) and random hexamer primers were used in the reaction. The resulting cDNA was stored at -20°C. Alternatively, the Thermoscript® RT kit (Invitrogen) was used for cDNA synthesis according to the supplied protocol. PCR primers are listed in Supplemental Information (Fig. 19).

Quantitative PCR analysis was performed with SYBR® Green. Reactions were initiated with 100 ng of cDNA, 300 ng of both primers and the SYBR green MasterMix (Eurogentec) in a total reaction volume of 25 μL. Between 40-50 amplification cycles were performed in the ABI Prism® 7000 apparatus (Applied Biosystems).

**Micro-array genome-wide gene expression analysis.** RNA from STC-1 cells, cultured either in the presence of 5 or 25 mM glucose for the last 24 h, was used as template. First-strand cDNA synthesis was achieved using the RevertAid™ H Minus First Strand cDNA synthesis kit (Fermentas) according to the supplied protocol, using 5 μg of purified RNA. Both oligo(dT) and random hexamer primers were used in the reaction. The resulting cDNA was cleaned using the QIAquick PCR Purification Kit (Qiagen), and was used by the VIB Micro-Array Facility (VIB MAF vzw, Leuven, Belgium) for whole-genome expression analysis with Affymetrix GeneChip® Mouse Genome 430 2.0 Arrays.

**Immunohistochemistry.** Mouse and human tissues were embedded in paraffin after formaldehyde fixation. Both tissue samples were taken from the duodenal part of the small intestine. Anti-hu$\beta_2$-AR (1/100) (Abcam) was used as primary antibody for peroxidase staining on human tissue. We also detected $\beta_2$-AR in control tissues known to express this receptor using the same antibodies. Secondary antibodies were peroxidase-labeled goat anti-rabbit antibodies (Abcam). The stained tissues were developed with 3,3'-diaminobenzidine, and they were counterstained with hematoxylin (Gill III). Antibodies used for fluorescent staining and co-localization in mouse tissue were rabbit anti- $\beta_2$-AR (Assay Design) (1/50) and goat anti-SGLT1 (M19, Santa Cruz) (1/50). Secondary antibodies used were anti-rabbit immunoglobulin labeled with Cy3 (Jackson) and anti-goat immunoglobulin labeled with FITC (Jackson). Cells were counterstained with DAPI. For imaging, Zeiss 63x oil immersion was used, and photographs were made of the human samples with a Zeiss Axioplan 2 microscope using Axiovision software. For the mouse samples, a Hamamatsu Orca AG microscope was used with Smart Capture Software.

**Two-Electrode Voltage Clamp (*Xenopus* oocytes).** The cDNA sequence of a human $\beta_2$-AR (Missouri S&T cDNA Resource Center) was subcloned from the pcDNA3.1+ to the pGEMHE *Xenopus* expression vector (kind gift of Prof. Jan Tytgat, Leuven). The pGEMHE vector was cut with BamHI (New England Biolabs) and HindIII (Roche). The *ADRB2* coding region was amplified with primers containing the aforementioned restriction sites, the amplicons were purified from a TAE-agarose gel, and cut with the same enzymes. The resulting product was ligated using the Ligafast™ Rapid DNA Ligation System (Promega) and the constructs were verified by sequencing (VIB Genetic Service Facility, University of Antwerp). They were cut with NheI (Roche) for linearization. The gene encoding the FLAG-tagged Cystic Fibrosis Transmembrane conductance Regulator (CFTR) ion channel was derived from the M2 901/pBQ4.7 vector (kind gift of Prof. Jan Eggermont, Leuven) and was linearized with XhoI (New England Biolabs) for *in vitro* transcription. RNA was then transcribed *in vitro* with the Ribomax kit (Promega) or the mMESSAGE mMACHINE T7 kit (Ambion) following the manufacturer's protocol. RNA was visualized and its quality assessed on RNase-free agarose gels, and stored at -80°C. Buffers used were Ringer's buffer (ORi): 90 mM NaCl, 3 mM KCl, 2 mM $CaCl_2$, 5 mM HEPES, adjusted to pH 7.6 with

NaOH, and a high potassium buffer: 5 mM NaCl, 65 mM KCl, 2 mM $CaCl_2$, 5 mM HEPES and an equivalent amount of sugar or N-Methyl-D-glucamine (NMDG), ensuring equiosmolarity between sugar-containing and sugar-free buffers. The maximum measured difference in osmolarity between the buffers was 3.6%.

For oocyte collection, female *Xenopus* frogs were anesthetized by submersion in ice water/crushed ice for 30 min. After abdominal incision, the ovarian lobes were pulled out, cut off, and the oocyte clump was placed in ORi. Oocytes were then liberated in ORi-collagenase (1 mg/mL) for 1-2 h on a shaking incubator, and subsequently transferred to a $Ca^{2+}$-free solution using one washing step. They were shaken vigorously for maximum 15 min before collecting the individual oocytes in ORi buffer. Stage V oocytes were then selected under the microscope.

For injection of the oocytes, RNA encoding the CFTR and/or $\beta_2$-AR protein was drawn from a droplet afloat in mineral oil with a glass capillary. Injection volume was 46 nl. The amount of RNA varied between 10 and 25 ng. Injected oocytes were stored in ORi at 16°C for maximum 3 days until ready for testing. During the measurements, oocytes were continuously clamped to -60 mV. All measurements were recorded using the home-made software, DSPOOC.

**Assessment of $\beta_2$-AR responses in cultured mammalian cells.** A cDNA for the human *ADRB2* and *GNA15* was purchased from the Missouri S&T cDNA Resource Center and used for the construction of a stable Flp-In-293 (a derivative of the parent HEK293 cell line, Invitrogen) $\beta_2$-AR /G$\alpha$16 cell line. The cell line expressing both $\beta_2$-AR and G$\alpha$16 was selected in two successive transfection steps. First, Flp-In-293 cells were co-transfected with pMET7-ADRB2-FRT and pOG44. After Flp recombinase-assisted stable integration, an isogenic cell pool expressing the $\beta_2$-AR was selected in hygromycin-containing medium (100 $\mu$g/ml). Second, the isogenic cell pool was cotransfected with p-GNA15 and pIRES-Puro2 (Clontech) in a 5:1 ratio, followed by selection of single clones in medium containing puromycin (0.8 $\mu$g/ml). Flp-In-293 cells were maintained in DMEM medium (Gibco), containing penicillin/streptomycin (Sigma) and fetal bovine serum (Sigma) (10%). Hygromycin (100 $\mu$g/mL) was added for cells containing the *ADRB2* construct, and puromycin (1 $\mu$g/mL) was added for the cells containing the *GNA15* construct. The Flp-In-293 cells were transferred for $\beta_2$-AR assays to clear- and flat-bottom 96-well plates in DMEM (Gibco) containing dialyzed fetal bovine serum (Sigma). The plates were coated with fibronectin (Sigma). For that purpose, the fibronectin solution was diluted 40 times in PBS, added in the plate wells (60 $\mu$L), and removed again after incubation for 1 h, after which the plates were allowed to dry for 1 h. The Fluo-4 NW kit (Invitrogen) was used for detection of calcium signals following the manufacturer's protocol. All compounds tested were dissolved in HBSS (Sigma) at 37°C. Adrenergic antagonists (Sigma) were always added together with the Fluo4 compound, 50 min before addition of agonist. Agonists were added just prior to the actual measurement of the calcium signals generated. The Flexstation II apparatus (Molecular Devices) was used to perform calcium measurements. After overnight growth of the cells in transfer medium in the aforesaid multiwell-96 plates, the medium was removed, the cells incubated with 100 $\mu$L of the loading dye solution (Fluo-4), the plates covered with aluminum foil, and incubated at 37°C for 30-50 min. Then, 50 $\mu$L of a 3x concentrated agonist solution (sugar or epinephrine) was added just prior to the measurement in the Flexstation II, to give a total volume of 150 $\mu$L per well.

**Response to epinephrine and mannose in Flp-In-293 cells stably transfected with *ADRB2* and *GNA15*, incubated in different HBSS-based buffers.** This experiment was performed for checking the response to mannose in different conditions. Cells were incubated with Fluo-4 in different HBSS-based buffers either containing glucose 5 mM, mannose 5 mM or without glucose but supplemented with 4 mM L-glutamine (HBSS composition (mM): 1.26 $CaCl_2$, 0.49 $MgCl_2$, 5.33 KCl, 0.4 $MgSO_4$, 0.44 $KH_2PO_4$, 137.9 NaCl, 0.34 $Na_2HPO_4$).

**Competitive radioligand binding assay.** $\beta_2$-AR-containing membranes (RBHBE2M) and $^{125}$I-cyanopindolol (NEX189) (spec. act. 2200 Ci/mmol) were purchased from PerkinElmer. TRIS-HCl buffer containing $MgCl_2$ and EGTA was used as incubation and assay buffer, while TRIS-HCl buffer was used as wash buffer. All buffers were cooled and all further steps performed on ice. Membranes were diluted 150 times in assay buffer. The radioligand was diluted to a final concentration of 0.097 nM and the tested compounds were added at the indicated concentrations. The mixtures were incubated for 1 h at ambient temperature, and subsequently filtered using a vacuum pump over GF/C filters (Whatman), pre-soaked in 0.5% polyethyleneimine,. Following 9 rinses with ice-cold wash buffer, the filters were transferred to vials, which were read in a gamma-counter (Gamma master LKB Wallac 1277). Non-specific binding of $^{125}$I-cyanopindolol was determined with membranes devoid of $\beta_2$-AR.

**Animals and diet.** All the experiments were approved by the ethical committee on animal experimentation of the KU Leuven. Wistar rats (Harlan Netherlands B.V.) were maintained on a 12h light-12h dark cycle, and fed with standard rat food chow and water *ad libitum.*

**Preparation of everted intestinal sacs.** Rats weighing around 150 g were fasted overnight before the experiment, and euthanized by cervical dislocation on the day of the experiment. The abdomen was opened by a midline incision, and a segment of around 35 cm of proximal intestine was isolated. The intestinal segment was rinsed with ice-cold Ringer solution (composition (mM): 140 NaCl, 5 KCl, 1 $MgCl_2$, 2 $CaCl_2$, 10 HEPES, 10 TRIS, gassed with 95% $O_2$ and 5% $CO_2$, pH 7.4), and 4-7 everted sacs were prepared, each approximately 3 cm in length, by tying off the ends of the intestinal segments with threads (46). The sacs were filled with Ringer solution containing 5 mM mannitol (Sigma Aldrich) (to maintain osmotic balance) and L-glutamine (2 mM) (Sigma Aldrich) (as an energy source). Each everted sac was transferred to a separate glass beaker containing 50 mL continuously oxygenated Ringer solution with L-glutamine (2

mM), and maintained in a water bath at 37 $^0$C. Then, epinephrine 10 μM (Sigma Aldrich), mannose 5 mM (Sigma Aldrich), ICI 118,551 10 μM (Sigma Aldrich), phlorizin 100 μM (Sigma Aldrich), LX4211 2 μM (MedKoo Biosciences, Inc. USA.) or Ringer solution were added to the respective beaker for a 15-min pre-incubation (concentrations indicate final concentrations of the compounds). For the sacs treated with ICI 118,551, phlorizin or LX 4211, the inhibitors were added before mannose or epinephrine. After pre-incubation, 5 mM glucose, or 2.5 mM glucose for the ICI 118,551 control experiment, was added to the external buffer to initiate glucose transport. After 10 min incubation, a sample was collected from inside the sac using a 1 mL syringe (Terumo) with a 26 gauge needle (Terumo). These samples were analyzed using a glucose assay kit based on glucose oxidase (Sigma Aldrich, for details, see below). Sacs treated with colchicine 5 μM (SERVA Feinbiochemica) or myristoylated PKI 14-22 amide 1 μM (Tocris), were pre-incubated for an additional 10 min before adding epinephrine or ICI 118,551. Also for colchicine, a TRIS-free Ringer solution was used, as TRIS interferes with colchicine activity (130). For comparing glucose transport rates, each everted sac was first pre-incubated for 15 min in Ringer solution, then glucose was added to a final concentration of 10 mM for a further incubation during 5, 10 or 75 min. We noticed that it was important to take certain precautions during these experiments: the intestine was never allowed to overfill and be stretched while rinsing the intestinal lumen at the time of isolation and filling the everted sac with Ringer solution. Trapping air bubbles inside the everted sac was avoided. Also, the regions of the intestine where mucus was still present were not used for preparing everted sacs.

**Glucose assay.** The glucose concentration of the samples was assayed by a glucose assay kit (Sigma Aldrich, GOD/POD method). The assay procedure recommended by the kit was modified for small sample volumes as follows. Fifty μL of sample was transferred to a well of a 96-well plate, and at time zero, the reaction was started by adding 100 μL of assay reagent to the first well, and mixing. Each well was allowed to react for exactly 30 min at 37 °C. The reaction was stopped by adding 100 μL of 12 N $H_2SO_4$ (Merck) into each well, and carefully mixing. The absorbance was measured at 540 nm with a plate reader (Tecan 200). Mannose is only detected by the glucose oxidase assay with about 100-fold lower sensitivity than for glucose (131).

## ***In vivo* oral glucose tolerance test**

The oral glucose tolerance test was performed on normal rats weighing around 250 g, which were fasted for 16 h before the test. Blood was sampled by tail vein puncture, and the blood glucose level was measured by glucometer (Verio OneTouch glucometer). Rats were divided in four groups: one with glucose 2 g/kg or glucose 4 g/kg (Sigma Aldrich), both in the presence or absence of CD3-403. The CD3-403 compound displays very low cell permeability (assessed by a Caco-2 permeability assay), and hence we aimed for a local intestinal concentration of 5 μM, based on 10 times the $IC_{50}$ and using an additional safety factor of 10 for any non-specific binding to intestinal content such as mucus. Glucose or glucose along with CD3-403, dissolved in 0.9% saline, was administered to rats by oral gavage in the respective groups. Blood glucose was measured at 0, 15, 30, 60 and 120 minutes. All experiments were conducted around the same time in the morning.

## **Statistical analysis**

For the experiments on epinephrine and mannose stimulation of glucose transport and their inhibition by ICI 118,551, and for the experiments on the effect of LX 4211 and phlorizin on glucose and mannose transport, a one-way ANOVA was performed, followed by Tukey's test. For the experiment on the effect of colchicine on epinephrine stimulation of glucose transport, the direction of the effect of colchicine (inhibition) and epinephrine (stimulation) was known, hence, a one tailed t-test was applied. For the effect of epinephrine and mannose in the calcium assay experiment, the epinephrine and mannose groups were analyzed individually by one-way ANOVA, followed by Tukey's test. For the effect of ICI 118,551 on glucose transport, a two-tailed t-test was applied. In case of the *in vivo* glucose bolus administration, the direction of the effect that we wanted to test was known, i.e. inhibition by CD3-403, hence a one tailed t-test was applied. All the statistical analyses were performed using GraphPad Prism 5 software.

**STD-NMR.** Cell membranes were prepared as described in Hoare *et al.*(132), with some modifications. Flp-In-293 cells (derived from the parent HEK293 cell line) stably transfected with *ADRB2* and *GNA15* expression constructs were grown in DMEM medium, containing penicillin (100 U/mL)-streptomycin (100 μg/mL) and fetal bovine serum (10%). Hygromycin (100 μg/mL) was added for the *ADRB2* construct and puromycin (1 μg/mL) was added for the *GNA15* construct as mentioned in the methods section for the calcium assay. Non-transfected HEK293 cells, which have a low endogenous expression of the $\beta_2$-AR, were grown in culture medium as described above, but without hygromycin and puromycin. After reaching confluence in a culture flask of 150 $cm^2$, monolayers of both cell lines were dislodged by trituration with their respective culture media. Cells were centrifuged at 150 g, 20 °C for 4 min, the supernatant medium was discarded, and the cells were washed with PBS. The cells were then re-suspended in 40 mL lysis buffer (25 mM TRIS, 2 mM EDTA, 6 mM $MgCl_2$ and 0.1 mM 4-(2-aminoethyl) benzenesulfonyl fluoride hydrochloride (AEBSF) pH 7.5, for 4 confluent flasks), kept at 4 °C, and homogenized in an ice-cold glass Dounce homogenizer with 45 strokes. The homogenates were centrifuged at 1000 g, 4 °C for 10 min to remove intact cells. The supernatants were centrifuged

at 40,000 g, 4 °C for 30 min. To ensure that the cell membrane preparations were guanine-nucleotide-free, the resulting pellet was washed with 30 mL lysis buffer, and finally suspended in buffer with 20 mM HEPES, 100 mM NaCl, 1 mM EDTA and 3 mM $MgSO_4$, pH 7.5. Total protein was quantified with the bicinchoninic acid assay (BCA assay) using bovine serum albumin (BSA) as the standard. Cell membranes were stored at -80 °C until use. Membranes were reconstituted in PBS for the STD-NMR assay.

NMR experiments were performed at 5°C on a Bruker Avance II 600 NMR spectrometer equipped with a cryogenic TCI probe with a z-gradient. The standard Bruker pulse program stddiffesgp (54) was used for data collection using excitation sculpting to suppress the water signal and a 5-s STD saturation time. Data are collected with 32 k complex points for 2.5 s. A delay of 2 s is applied between each FID to ensure complete relaxation. For each of the STD experiments, 32 scans are accumulated. The spectra for both on-resonance and off-resonance saturation at resp. 0.7 and 12 ppm are collected interleaved. The Bruker command stdsplit was used to process and subtract on-and off-resonance FIDs.

## The Disclosure Explained

### Problem

Diabetes and obesity are two widespread diseases that could strongly profit from a precisely controlled reduction of sugar uptake from the gut. The sugar sensing systems that enhance sugar uptake from the gut could be appropriate drug targets but they are poorly understood. The glucose receptor system in the main intestinal epithelial cells, the enterocytes, has remained elusive up to now.

### Results

We show that the β2-adrenergic receptor is strongly expressed at the apical membrane of the enterocytes, a highly unusual location for a hormone receptor of which the ligand, epinephrine, is distributed through the bloodstream. We show with several heterologous expression systems that the $\beta_2$-AR responds to different sugars in appropriate concentrations for a nutrient in the gut and that these responses are inhibited by well-known antagonists of $\beta_2$-AR. Epinephrine and sugars appear to bind to different but closely connected binding sites. With everted intestinal sacs of rats we show that epinephrine and sugars stimulate the uptake of glucose from the gut lumen compartment through SGLT1, the main glucose transporter in the enterocytes. This stimulation depends on $\beta_2$-AR and its PKA signaling pathway. Finally, using a novel non-bioavailable $\beta_2$-AR antagonist, we show that inhibition of $\beta_2$-AR *in vivo* results in a lowered increase in blood glucose levels after oral administration of a glucose bolus.

### Impact

The administration of SGLT1 or intestinal carbohydrase inhibitors to lower glucose uptake from the gut in diabetic and obese patients generally results in uncontrolled increases in free sugar levels in the gut causing osmotic diarrhea, enhanced microbial activity and flatulence. Complete inhibition of $\beta_2$-AR with a non-bioavailable antagonist has the advantage that only sugar-induced stimulation of sugar uptake by SGLT1 is blocked and not the total sugar uptake activity, strongly reducing the risk of unwanted side effects. Controlled lowering of post-prandial peaks in the blood glucose level in diabetic and obese patients is highly desirable for a beneficial medical treatment.

### References

1. Dockray GJ. Luminal sensing in the gut: an overview. J Physiol Pharmacol. 2003;54 Suppl 4:9-17.
2. Ferraris RP. Dietary and developmental regulation of intestinal sugar transport. Biochem J. 2001;360(Pt 2):265-76.
3. Diamond JM, and Karasov WH. Adaptive regulation of intestinal nutrient transporters. Proc Natl Acad Sci U S A. 1987;84(8):2242-5.
4. Dyer J, Vayro S, King TP, and Shirazi-Beechey SP. Glucose sensing in the intestinal epithelium. Eur J Biochem. 2003;270(16):3377-88.
5. Shirazi-Beechey SP, Moran AW, Batchelor DJ, Daly K, and Al-Rammahi M. Glucose sensing and signalling; regulation of intestinal glucose transport. Proc Nutr Soc. 2011;70(2):185-93.
6. Sharp PA, Debnam ES, and Srai SK. Rapid enhancement of brush border glucose uptake after exposure of rat jejunal mucosa to . Gut. 1996;39(4):545-50.
7. Miyamoto K, Hase K, Takagi T, Fujii T, Taketani Y, Minami H, et al. Differential responses of intestinal glucose transporter mRNA transcripts to levels of dietary sugars. Biochem J. 1993;295 (Pt 1):211-5.

8. Sharp PA, and Debnam ES. The role of cyclic AMP in the control of sugar transport across the brush-border and basolateral membranes of rat jejunal enterocytes. Exp Physiol. 1994;79(2):203-14.
9. Dyer J, Salmon KS, Zibrik L, and Shirazi-Beechey SP. Expression of sweet taste receptors of the T1R family in the intestinal tract and enteroendocrine cells. Biochem Soc Trans. 2005;33(Pt 1):302-5.
10. Margolskee RF, Dyer J, Kokrashvili Z, Salmon KS, Ilegems E, Daly K, et al. T1R3 and gustducin in gut sense sugars to regulate expression of Na+-glucose cotransporter 1. Proc Natl Acad Sci U S A. 2007;104(38):15075-80.
11. Buddington RK, Chen JW, and Diamond JM. Dietary regulation of intestinal brush-border sugar and amino acid transport in carnivores. Am J Physiol. 1991;261(4 Pt 2):R793-801.
12. Batchelor DJ, Al-Rammahi M, Moran AW, Brand JG, Li X, Haskins M, et al. Sodium/glucose cotransporter-1, sweet receptor, and disaccharidase expression in the intestine of the domestic dog and cat: two species of different dietary habit. Am J Physiol Regul Integr Comp Physiol. 2011;300(1):R67-75.
13. Sternini C, Anselmi L, and Rozengurt E. Enteroendocrine cells: a site of 'taste' in gastrointestinal chemosensing. Curr Opin Endocrinol Diabetes Obes. 2008;15(1):73-8.
14. Steinert RE, and Beglinger C. Nutrient sensing in the gut: interactions between chemosensory cells, visceral afferents and the secretion of satiation peptides. Physiol Behav. 2011;105(1):62-70.
15. Glendinning JI. Oral Post-Oral Actions of Low-Calorie Sweeteners: A Tale of Contradictions and Controversies. Obesity (Silver Spring). 2018;26 Suppl 3:S9-S17.
16. Ezcurra M, Reimann F, Gribble FM, and Emery E. Molecular mechanisms of incretin hormone secretion. Curr Opin Pharmacol. 2013;13(6):922-7.
17. Kraakman L, Lemaire K, Ma P, Teunissen AW, Donaton MC, Van Dijck P, et al. A Saccharomyces cerevisiae G-protein coupled receptor, Gpr1, is specifically required for glucose activation of the cAMP pathway during the transition to growth on glucose. Mol Microbiol. 1999;32(5):1002-12.
18. Lemaire K, Van de Velde S, Van Dijck P, and Thevelein JM. Glucose and sucrose act as agonist and mannose as antagonist ligands of the G protein-coupled receptor Gpr1 in the yeast Saccharomyces cerevisiae. Mol Cell. 2004;16(2):293-9.
19. Welton RM, and Hoffman CS. Glucose monitoring in fission yeast via the Gpa2 galpha, the git5 Gbeta and the git3 putative glucose receptor. Genetics. 2000;156(2):513-21.
20. Maidan MM, De Rop L, Serneels J, Exler S, Rupp S, Tournu H, et al. The G protein-coupled receptor Gpr1 and the Galpha protein Gpa2 act through the cAMP-protein kinase A pathway to induce morphogenesis in Candida albicans. Mol Biol Cell. 2005;16(4):1971-86.
21. Miwa T, Takagi Y, Shinozaki M, Yun CW, Schell WA, Perfect JR, et al. Gpr1, a putative G-protein-coupled receptor, regulates morphogenesis and hypha formation in the pathogenic fungus Candida albicans. Eukaryot Cell. 2004;3(4):919-31.
22. Li L, and Borkovich KA. GPR-4 is a predicted G-protein-coupled receptor required for carbon source-dependent asexual growth and development in Neurospora crassa. Eukaryot Cell. 2006;5(8):1287-300.
23. Xue C, Hsueh YP, and Heitman J. Magnificent seven: roles of G protein-coupled receptors in extracellular sensing in fungi. FEMS Microbiol Rev. 2008;32(6):1010-32.
24. Rui L. Energy metabolism in the liver. Compr Physiol. 2014;4(1):177-97.
25. Arner P. Human fat cell lipolysis: biochemistry, regulation and clinical role. Best Pract Res Clin Endocrinol Metab. 2005;19(4):471-82.
26. Shukla AK, Sun JP, and Lefkowitz RJ. Crystallizing thinking about the beta2-adrenergic receptor. Mol Pharmacol. 2008;73(5):1333-8.
27. Frishman WH. beta-Adrenergic blockers: a 50-year historical perspective. Am J Ther. 2008;15(6):565-76.
28. Cherezov V, Rosenbaum DM, Hanson MA, Rasmussen SG, Thian FS, Kobilka TS, et al. High-resolution crystal structure of an engineered human beta2-adrenergic G protein-coupled receptor. Science. 2007;318(5854):1258-65.
29. Rasmussen SG, Choi HJ, Rosenbaum DM, Kobilka TS, Thian FS, Edwards PC, et al. Crystal structure of the human beta2 adrenergic G-protein-coupled receptor. Nature. 2007;450(7168):383-7.
30. Rosenbaum DM, Cherezov V, Hanson MA, Rasmussen SG, Thian FS, Kobilka TS, et al. GPCR engineering yields high-resolution structural insights into beta2-adrenergic receptor function. Science. 2007;318(5854):1266-73.
31. Rasmussen SG, Choi HJ, Fung JJ, Pardon E, Casarosa P, Chae PS, et al. Structure of a nanobody-stabilized active state of the beta(2) adrenoceptor. Nature. 2011;469(7329):175-80.
32. Rasmussen SG, DeVree BT, Zou Y, Kruse AC, Chung KY, Kobilka TS, et al. Crystal structure of the beta2 adrenergic receptor-Gs protein complex. Nature. 2011;477(7366):549-55.
33. Strosberg AD. Structure, function, and regulation of adrenergic receptors. Protein Sci. 1993;2(8):1198-209.
34. Baker JG. The selectivity of beta-adrenoceptor antagonists at the human beta1, beta2 and beta3 adrenoceptors. Br J Pharmacol. 2005;144(3):317-22.
35. Smith C, and Teitler M. Beta-blocker selectivity at cloned human beta 1- and beta 2-adrenergic receptors. Cardiovasc Drugs Ther. 1999;13(2):123-6.

36. Tyndall JD, and Sandilya R. GPCR agonists and antagonists in the clinic. Med Chem. 2005;1(4):405-21.

37. Vassilatis DK, Hohmann JG, Zeng H, Li F, Ranchalis JE, Mortrud MT, et al. The G protein-coupled receptor repertoires of human and mouse. Proc Natl Acad Sci U S A. 2003;100(8):4903-8.

38. Singh AK, Riederer B, Krabbenhoft A, Rausch B, Bonhagen J, Lehmann U, et al. Differential roles of NHERF1, NHERF2, and PDZK1 in regulating CFTR-mediated intestinal anion secretion in mice. J Clin Invest. 2009;119(3):540-50.

39. Zhang J, Halm ST, and Halm DR. Adrenergic activation of electrogenic K+ secretion in guinea pig distal colonic epithelium: involvement of beta1- and beta2-adrenergic receptors. Am J Physiol Gastrointest Liver Physiol. 2009;297(2):G269-77.

40. Olsen WA, and Ingelfinger FJ. The role of sodium in intestinal glucose absorption in man. J Clin Invest. 1968;47(5):1133-42.

41. Ferraris RP, Yasharpour S, Lloyd KC, Mirzayan R, and Diamond JM. Luminal glucose concentrations in the gut under normal conditions. Am J Physiol. 1990;259(5 Pt 1):G822-37.

42. Minuth M, and Jakobs KH. Sodium regulation of agonist and antagonist binding to beta-adrenoceptors in intact and Ns-deficient membranes. Naunyn Schmiedebergs Arch Pharmacol. 1986;333(2):124-9.

43. Bokoch MP, Zou Y, Rasmussen SG, Liu CW, Nygaard R, Rosenbaum DM, et al. Ligand-specific regulation of the extracellular surface of a G-protein-coupled receptor. Nature. 2010;463(7277):108-12.

44. Canals M, Sexton PM, and Christopoulos A. Allostery in GPCRs: 'MWC' revisited. Trends Biochem Sci. 2011;36(12):663-72.

45. Alam MA, Al-Jenoobi FI, and Al-Mohizea AM. Everted gut sac model as a tool in pharmaceutical research: limitations and applications. J Pharm Pharmacol. 2012;64(3):326-36.

46. Hamilton KL, and Butt AG. Glucose transport into everted sacs of the small intestine of mice. Adv Physiol Educ. 2013;37(4):415-26.

47. Yu LC, Huang CY, Kuo WT, Sayer H, Turner JR, and Buret AG. SGLT-1-mediated glucose uptake protects human intestinal epithelial cells against Giardia duodenalis-induced apoptosis. Int J Parasitol. 2008;38(8-9):923-34.

48. Nalli AD, Kumar DP, Mahavadi S, Al-Shboul O, Alkahtani R, Kuemmerle JF, et al. Hypercontractility of intestinal longitudinal smooth muscle induced by cytokines is mediated by the nuclear factor-kappaB/AMP-activated kinase/-myosin light chain kinase pathway. J Pharmacol Exp Ther. 2014;350(1):89-98.

49. Tazawa S, Yamato T, Fujikura H, Hiratochi M, Itoh F, Tomae M, et al. SLC5A9/SGLT4, a new Na+-dependent glucose transporter, is an essential transporter for mannose, 1,5-anhydro-D-glucitol, and fructose. Life Sci. 2005;76(9):1039-50.

50. Wright EM, Loo DD, and Hirayama BA. Biology of human sodium glucose transporters. Physiol Rev. 2011;91(2):733-94.

51. Assadi-Porter FM, Tonelli M, Maillet E, Hallenga K, Benard O, Max M, et al. Direct NMR detection of the binding of functional ligands to the human sweet receptor, a heterodimeric family 3 GPCR. J Am Chem Soc. 2008;130(23):7212-3.

52. Assadi-Porter FM, Tonelli M, Maillet EL, Markley JL, and Max M. Interactions between the human sweet-sensing T1R2-T1R3 receptor and sweeteners detected by saturation transfer difference NMR spectroscopy. Biochim Biophys Acta. 2010;1798(2):82-6.

53. Venkitakrishnan RP, Benard O, Max M, Markley JL, and Assadi-Porter FM. Use of NMR saturation transfer difference spectroscopy to study ligand binding to membrane proteins. Methods Mol Biol. 2012;914:47-63.

54. Mayer M, and Meyer B. Characterization of ligand binding by Saturation Transfer Difference NMR Spectroscopy. Angew Chem Int Ed Engl. 1999;38(12):1784-8.

55. Boivin V, Jahns R, Gambaryan S, Ness W, Boege F, and Lohse MJ. Immunofluorescent imaging of beta 1- and beta 2-adrenergic receptors in rat kidney. Kidney Int. 2001;59(2):515-31.

56. Blake WD. Effect of epinephrine on tubular reabsorption of glucose by dog kidney. Am J Physiol. 1962;202:897-900.

57. Goff JP. Invited review: Mineral absorption mechanisms, mineral interactions that affect acid-base and antioxidant status, and diet considerations to improve mineral status. J Dairy Sci. 2018;101(4):2763-813.

58. Yu O, and Ouyang A. Distribution of beta-adrenoceptor subtypes in gastrointestinal tract of nondiabetic and diabetic BB rats. A longitudinal study. Dig Dis Sci. 1997;42(6):1146-53.

59. Vijftigschild LA, Berkers G, Dekkers JF, Zomer-van Ommen DD, Matthes E, Kruisselbrink E, et al. beta2-Adrenergic receptor agonists activate CFTR in intestinal organoids and subjects with cystic fibrosis. Eur Respir J. 2016;48(3):768-79.

60. Mailleau C, Capeau J, and Brahimi-Horn MC. Interrelationship between the Na+/glucose cotransporter and CFTR in Caco-2 cells: relevance to cystic fibrosis. J Cell Physiol. 1998;176(3):472-81.

61. Re G, Badino P, De Angelis I, Odore R, Belloli C, Stammati A, et al. Identification and coupling to adenylate cyclase of three different [(3)H]CGP 12177 binding sites in Caco-2 cell membranes. Pharmacol Res. 2001;43(4):393-8.

62. Wright EM, Hirsch JR, Loo DD, and Zampighi GA. Regulation of Na+/glucose cotransporters. J Exp Biol. 1997;200(Pt 2):287-93.
63. Vestri S, Okamoto MM, de Freitas HS, Aparecida Dos Santos R, Nunes MT, Morimatsu M, et al. Changes in sodium or glucose filtration rate modulate expression of glucose transporters in renal proximal tubular cells of rat. J Membr Biol. 2001;182(2):105-12.
64. Shepard BD, and Pluznick JL. Saving the sweetness: renal glucose handling in health and disease. Am J Physiol Renal Physiol. 2017;313(1):F55-F61.
65. Subramanian S, Glitz P, Kipp H, Kinne RK, and Castaneda F. Protein kinase-A affects sorting and conformation of the sodium-dependent glucose co-transporter SGLT1. J Cell Biochem. 2009;106(3):444-52.
66. Schafer N, Rikkala PR, Veyhl-Wichmann M, Keller T, Jurowich CF, Geiger D, et al. A Modified Tripeptide Motif of RS1 (RSC1A1) Down-Regulates Exocytotic Pathways of Human Na(+)-d-glucose Cotransporters SGLT1, SGLT2, and Glucose Sensor SGLT3 in the Presence of Glucose. Mol Pharmacol. 2019;95(1):82-96.
67. Doenst T, and Taegtmeyer H. alpha-adrenergic stimulation mediates glucose uptake through phosphatidylinositol 3-kinase in rat heart. Circ Res. 1999;84(4):467-74.
68. Faintrenie G, and Geloen A. Alpha-1 adrenergic stimulation of glucose uptake in rat white adipocytes. J Pharmacol Exp Ther. 1998;286(2):607-10.
69. Abe H, Minokoshi Y, and Shimazu T. Effect of a beta 3-adrenergic agonist, BRL35135A, on glucose uptake in rat skeletal muscle in vivo and in vitro. J Endocrinol. 1993;139(3):479-86.
70. Chernogubova E, Cannon B, and Bengtsson T. Norepinephrine increases glucose transport in brown adipocytes via beta3-adrenoceptors through a cAMP, PKA, and PI3-kinase-dependent pathway stimulating conventional and novel PKCs. Endocrinology. 2004;145(1):269-80.
71. Catus SL, Gibbs ME, Sato M, Summers RJ, and Hutchinson DS. Role of beta-adrenoceptors in glucose uptake in astrocytes using beta-adrenoceptor knockout mice. Br J Pharmacol. 2011;162(8):1700-15.
72. Hutchinson DS, Chernogubova E, Dallner OS, Cannon B, and Bengtsson T. Beta-adrenoceptors, but not alpha-adrenoceptors, stimulate AMP-activated protein kinase in brown adipocytes independently of uncoupling protein-1. Diabetologia. 2005;48(11):2386-95.
73. Nevzorova J, Bengtsson T, Evans BA, and Summers RJ. Characterization of the beta-adrenoceptor subtype involved in mediation of glucose transport in L6 cells. Br J Pharmacol. 2002;137(1):9-18.
74. Kanda Y, and Watanabe Y. Adrenaline increases glucose transport via a Rap1-p38MAPK pathway in rat vascular smooth muscle cells. Br J Pharmacol. 2007;151(4):476-82.
75. Ngala RA, O'Dowd J, Wang SJ, Agarwal A, Stocker C, Cawthorne MA, et al. Metabolic responses to BRL37344 and clenbuterol in soleus muscle and C2C12 cells via different atypical pharmacologies and beta2-adrenoceptor mechanisms. Br J Pharmacol. 2008;155(3):395-406.
76. Nevzorova J, Evans BA, Bengtsson T, and Summers RJ. Multiple signalling pathways involved in beta2-adrenoceptor-mediated glucose uptake in rat skeletal muscle cells. Br J Pharmacol. 2006;147(4):446-54.
77. Liu YL, and Stock MJ. Acute effects of the beta 3-adrenoceptor agonist, BRL 35135, on tissue glucose utilisation. Br J Pharmacol. 1995;114(4):888-94.
78. Dong JH, Chen X, Cui M, Yu X, Pang Q, and Sun JP. beta2-adrenergic receptor and astrocyte glucose metabolism. J Mol Neurosci. 2012;48(2):456-63.
79. Dehvari N, Hutchinson DS, Nevzorova J, Dallner OS, Sato M, Kocan M, et al. beta(2)-Adrenoceptors increase translocation of GLUT4 via GPCR kinase sites in the receptor C-terminal tail. Br J Pharmacol. 2012;165(5):1442-56.
80. Mukaida S, Sato M, Oberg AI, Dehvari N, Olsen JM, Kocan M, et al. BRL37344 stimulates GLUT4 translocation and glucose uptake in skeletal muscle via beta2-adrenoceptors without causing classical receptor desensitization. Am J Physiol Regul Integr Comp Physiol. 2019;316(5):R666-R77.
81. Shirazi-Beechey SP, Moran AW, Bravo D, and Al-Rammahi M. Nonruminant Nutrition Symposium: intestinal glucose sensing and regulation of glucose absorption: implications for swine nutrition. J Anim Sci. 2011;89(6):1854-62.
82. Stearns AT, Balakrishnan A, Rhoads DB, and Tavakkolizadeh A. Rapid upregulation of sodium-glucose transporter SGLT1 in response to intestinal sweet taste stimulation. Ann Surg. 2010;251(5):865-71.
83. Gromova LV, Fetissov SO, and Gruzdkov AA. Mechanisms of Glucose Absorption in the Small Intestine in Health and Metabolic Diseases and Their Role in Appetite Regulation. Nutrients. 2021;13(7).
84. Thevelein JM, and Voordeckers K. Functioning and evolutionary significance of nutrient transceptors. Mol Biol Evol. 2009;26(11):2407-14.
85. Nordstrom KJ, Sallman Almen M, Edstam MM, Fredriksson R, and Schioth HB. Independent HHsearch, Needleman--Wunsch-based, and motif analyses reveal the overall hierarchy for most of the G protein-coupled receptor families. Mol Biol Evol. 2011;28(9):2471-80.
86. de Mendoza A, Sebe-Pedros A, and Ruiz-Trillo I. The evolution of the GPCR signaling system in eukaryotes: modularity, conservation, and the transition to metazoan multicellularity. Genome Biol Evol. 2014;6(3):606-19.

87. Krishnan A, Almen MS, Fredriksson R, and Schioth HB. The origin of GPCRs: identification of mammalian like Rhodopsin, Adhesion, Glutamate and Frizzled GPCRs in fungi. PLoS One. 2012;7(1):e29817.

88. Graul RC, and Sadee W. Evolutionary relationships among G protein-coupled receptors using a clustered database approach. AAPS PharmSci. 2001;3(2):E12.

89. Wiejak J, Surmacz L, and Wyroba E. Immunoanalogue of vertebrate beta-adrenergic receptor in the unicellular eukaryote Paramecium. Histochem J. 2002;34(1-2):51-6.

90. Wiejak J, Surmacz L, and Wyroba E. Dynamin-association with agonist-mediated sequestration of beta-adrenergic receptor in single-cell eukaryote Paramecium. J Exp Biol. 2004;207(Pt 10):1625-32.

91. Platek A, Wiejak J, and Wyroba E. RT-PCR and Northern blot analysis in search for a putative Paramecium beta-adrenergic receptor. Acta Biochim Pol. 1999;46(3):813-21.

92. Wyroba E. Stimulation of Paramecium phagocytosis by phorbol ester and forskolin. Cell Biol Int Rep. 1987;11(9):657-64.

93. Wyroba E. Beta-adrenergic stimulation of phagocytosis in the unicellular eukaryote Paramecium aurelia. Cell Biol Int Rep. 1989;13(8):667-78.

94. de Castro SL, and Oliveira MM. Radioligand binding characterization of beta-adrenergic receptors in the protozoa Trypanosoma cruzi. Comp Biochem Physiol C. 1987;87(1):5-8.

95. Ghanouni P, Steenhuis JJ, Farrens DL, and Kobilka BK. Agonist-induced conformational changes in the G-protein-coupling domain of the beta 2 adrenergic receptor. Proc Natl Acad Sci U S A. 2001;98(11):5997-6002.

96. Thevelein JM, and de Winde JH. Novel sensing mechanisms and targets for the cAMP-protein kinase A pathway in the yeast Saccharomyces cerevisiae. Mol Microbiol. 1999;33(5):904-18.

97. Aulsebrook KA. Intestinal absorption of glucose and sodium: Effects of epinephrine and norepinephrine. Biochem Biophys Res Commun. 1965;18:165-9.

98. Ishikawa Y, Eguchi T, and Ishida H. Mechanism of beta-adrenergic agonist-induced transmural transport of glucose in rat small intestine. Regulation of phosphorylation of SGLT1 controls the function. Biochim Biophys Acta. 1997;1357(3):306-18.

99. Bochain A, Estey L, Haronian G, Reale M, Rojas C, and Cramer J. Determination of catecholamine permeability coefficients for passive diffusion across phospholipid vesicle membranes. J Membr Biol. 1981;60(1):73-6.

100. Aschenbach JR, Borau T, and Gabel G. Glucose uptake via SGLT-1 is stimulated by beta(2)-adrenoceptors in the ruminal epithelium of sheep. J Nutr. 2002;132(6):1254-7.

101. Taatjes DJ, and Roth J. Glycosylation in intestinal epithelium. Int Rev Cytol. 1991;126:135-93.

102. Biol MC, Martin A, and Louisot P. Nutritional and developmental regulation of glycosylation processes in digestive organs. Biochimie. 1992;74(1):13-24.

103. Moremen KW, Tiemeyer M, and Nairn AV. Vertebrate protein glycosylation: diversity, synthesis and function. Nat Rev Mol Cell Biol. 2012;13(7):448-62.

104. Asano T, Ogihara T, Katagiri H, Sakoda H, Ono H, Fujishiro M, et al. Glucose transporter and Na+/glucose cotransporter as molecular targets of anti-diabetic drugs. Curr Med Chem. 2004;11(20):2717-24.

105. Wagman AS, and Nuss JM. Current therapies and emerging targets for the treatment of diabetes. Curr Pharm Des. 2001;7(6):417-50.

106. Osswald C, Baumgarten K, Stumpel F, Gorboulev V, Akimjanova M, Knobeloch KP, et al. Mice without the regulator gene Rsc1A1 exhibit increased Na+-D-glucose cotransport in small intestine and develop obesity. Mol Cell Biol. 2005;25(1):78-87.

107. Dyer J, Wood IS, Palejwala A, Ellis A, and Shirazi-Beechey SP. Expression of monosaccharide transporters in intestine of diabetic humans. Am J Physiol Gastrointest Liver Physiol. 2002;282(2):G241-8.

108. Koepsell H. Glucose transporters in the small intestine in health and disease. Pflugers Arch. 2020;472(9):1207-48.

109. Nguyen NQ, Debreceni TL, Bambrick JE, Chia B, Wishart J, Deane AM, et al. Accelerated intestinal glucose absorption in morbidly obese humans: relationship to glucose transporters, incretin hormones, and glycemia. J Clin Endocrinol Metab. 2015;100(3):968-76.

110. Soergel KH. Colonic fermentation: metabolic and clinical implications. Clin Investig. 1994;72(10):742-8.

111. Wright EM, Hirayama BA, and Loo DF. Active sugar transport in health and disease. J Intern Med. 2007;261(1):32-43.

112. Santulli G, Lombardi A, Sorriento D, Anastasio A, Del Giudice C, Formisano P, et al. Age-related impairment in insulin release: the essential role of beta(2)-adrenergic receptor. Diabetes. 2012;61(3):692-701.

113. Skikama H, and Ui M. Adrenergic receptor and epinephrine-induced hyperglycemia and glucose tolerance. Am J Physiol. 1975;229(4):962-6.

114. Holm G, Johansson S, Vedin A, Wilhelmsson C, and Smith U. The effect of beta-blockade on glucose tolerance and insulin release in adult diabetes. Acta Med Scand. 1980;208(3):187-91.

115. Moore CA, Milano SK, and Benovic JL. Regulation of receptor trafficking by GRKs and arrestins. Annu Rev

Physiol. 2007;69:451-82.

116. Yudowski GA, Puthenveedu MA, Henry AG, and von Zastrow M. Cargo-mediated regulation of a rapid Rab4-dependent recycling pathway. Mol Biol Cell. 2009;20(11):2774-84.

117. Barron AJ, Zaman N, Cole GD, Wensel R, Okonko DO, and Francis DP. Systematic review of genuine versus spurious side-effects of beta-blockers in heart failure using placebo control: recommendations for patient information. Int J Cardiol. 2013;168(4):3572-9.

118. Sears MR. Adverse effects of beta-agonists. J Allergy Clin Immunol. 2002;110(6 Suppl): S322-8.

119. Leonetti G, and Egan CG. Use of carvedilol in hypertension: an update. Vasc Health Risk Manag. 2012;8:307-22.

120. Messerli FH, and Grossman E. beta-Blockers in hypertension: is carvedilol different? Am J Cardiol. 2004;93(9A):7B-12B.

121. Nilsson PM, Cederholm J, Zethelius BR, Eliasson BR, Eeg-Olofsson K, and Gudbj Rnsdottir S. Trends in blood pressure control in patients with type 2 diabetes: data from the Swedish National Diabetes Register (NDR). Blood Press. 2011;20(6):348-54.

122. Elayan H, Milic M, Sun P, Gharaibeh M, and Ziegler MG. Chronic beta2 adrenergic agonist, but not exercise, improves glucose handling in older type 2 diabetic mice. Cell Mol Neurobiol. 2012;32(5):871-7.

123. Ziegler MG, Elayan H, Milic M, Sun P, and Gharaibeh M. Epinephrine and the metabolic syndrome. Curr Hypertens Rep. 2012;14(1):1-7.

124. Jensen J, Ruzzin J, Jebens E, Brennesvik EO, and Knardahl S. Improved insulin-stimulated glucose uptake and glycogen synthase activation in rat skeletal muscles after adrenaline infusion: role of glycogen content and PKB phosphorylation. Acta Physiol Scand. 2005;184(2):121-30.

125. Ernande L, Stanford KI, Thoonen R, Zhang H, Clerte M, Hirshman MF, et al. Relationship of brown adipose tissue perfusion and function: a study through beta2-adrenoreceptor stimulation. J Appl Physiol (1985). 2016;120(8):825-32.

126. Cole SW, and Sood AK. Molecular pathways: beta-adrenergic signaling in cancer. Clin Cancer Res. 2012;18(5):1201-6.

127. Perez-Sayans M, Somoza-Martin JM, Barros-Angueira F, Gayoso-Diz P, Otero-Rey EM, Gandra-Rey JM, et al. Activity of beta2-adrenergic receptor in oral squamous cell carcinoma is mediated by overexpression of the ADRBK2 gene: a pilot study. Biotech Histochem. 2012;87(3):179-86.

128. Ji Y, Chen S, Xiao X, Zheng S, and Li K. beta-blockers: a novel class of antitumor agents. Onco Targets Ther. 2012;5:391-401.

129. Chakir K, Xiang Y, Yang D, Zhang SJ, Cheng H, Kobilka BK, et al. The third intracellular loop and the carboxyl terminus of beta2-adrenergic receptor confer spontaneous activity of the receptor. Mol Pharmacol. 2003;64(5):1048-58.

130. Margulis L, Banerjee S, and White T. Colchicine-inhibited cilia regeneration: explanation for lack of effect in tris buffer medium. Science. 1969;164(3884):1177-8.

131. Raba J, and Mottola HA. Glucose oxidase as an analytical reagent. Crit Rev Anal Chem. 1995;25:1-42.

132. Hoare SR, and Usdin TB. Quantitative cell membrane-based radioligand binding assays for parathyroid hormone receptors. J Pharmacol Toxicol Methods. 1999;41(2-3):83-90.

## Figures

**Figure 1 | Localization of $\beta_2$-ARs at the apical side of intestinal epithelial cells. a,** $\beta_2$-AR immunostaining using Abcam antibodies in human duodenal biopsy sample as revealed by peroxidase staining (L: lumen of intestine). Secondary antibodies were peroxidase-labeled goat anti-rabbit antibodies (Abcam). **b,** magnification of $\beta_2$-AR immunostaining in individual epithelial cells (L: lumen of intestine), immunostaining in mouse intestinal tissue sections of **c,** $\beta_2$-AR with rabbit antibodies (Assay Design) and CY3-conjugated secondary anti-rabbit antibodies (Jackson) (red) and **d,** SGLT1 with goat antibodies (M19, Santa Cruz) and FITC-conjugated secondary anti-goat antibodies (Jackson) (green), **e,** overlay showing co-localization of $\beta_2$-AR and SGLT1. The arrow indicates either $\beta_2$-AR (a, b, c), SGLT1 (d) or co-localization of the two (e). The scale bar is 100 μm (a) or 10 μm (b-e).

**Figure 2 | Inhibition by $\beta$-AR antagonists, sugar specificity and affinity of $\beta_2$-AR. a,** Epinephrine- (20 nM, ●) and **b,** glucose- (70 mM, ●) induced responses and their inhibition by pre-incubation for 50 min together with the Fluo4 compound with various $\beta$-AR antagonists (all at 200 μM): nadolol ($\beta_{1,2}$-antagonist, ∘), labetalol ($\alpha,\beta$-antagonist, ▲), propranolol ($\beta_{1,2}$-antagonist, △) and ICI 118,551 ($\beta_2$-antagonist, ■) of $\beta_2$-AR expressed in Flp-In-293 cells. Addition of agonists was done just prior to the actual measurement of the calcium signals generated. Note the difference in Y-axis scale between a and b. Representative results from at least three repetitions are shown. **c,** Specificity of the

response to different sugars added at 50 mM. In all cases, except for 2-deoxyglucose (2DOG), the responses were completely blocked by 1 μM ICI 118,551. For 2DOG, only the response blocked by ICI 118,551 is shown. The y-axis displays peak fluorescence intensity (arbitrary units) for the response to the different sugars. All values are expressed as mean ± SD. **d,** Dose-response curve for maltotriose. $EC_{50}$ = ±10.00 mM. Maltotriose was used instead of glucose because of rapid uptake of glucose in the cells. **e,** Maltose and epinephrine responses of $\beta_2$-AR expressed in *Xenopus* oocytes. **f,** Responses to different sugars of $\beta_2$-AR expressed in *Xenopus* oocytes.

**Figure 3 | $^{125}$I-cyanopindolol binding in the presence of glucose to cell-free isolated membrane vesicles containing $\beta_2$-AR. a,** Stimulation of $^{125}$I -cyanopindolol binding with increasing equimolar concentrations of glucose + $Na^+$. Standard Error of the Mean (SEM) is indicated. Binding in the absence of glucose was normalized to 100%. Note that the Y-axis starts at 50%. **b,** Inhibition of $^{125}$I -cyanopindolol binding by increasing concentrations of the $\beta$-AR agonist, isoproterenol. The log values at -9 and -6 represent basal binding of $^{125}$I -cyanopindolol.

**Figure 4 | Effect of $\beta_2$-specific antagonist, ICI 118,551 on calcium response elicited by epinephrine (5 nM) and various sugars (50 mM) in Flp-In-293 cells stably transfected with ADRB2 and GNA15.** One μM ICI 118,551 inhibits epinephrine- and sugar-elicited $\beta_2$-AR -mediated calcium responses. X-axis shows time in s, and y-axis displays fluorescence intensity (arbitrary units). Representative results are shown from at least three repetitions.

**Figure 5 | Glucose transport in everted sacs prepared from rat intestine. a,** Comparison of the amount of glucose accumulated in the everted sacs, prepared from rat intestine (n=2), during different time periods (5, 10 and 75 min) after addition of 10 mM glucose **b,** Effect of epinephrine (Epi), the β2-specific antagonist ICI 118,551 (ICI) and the glucose transport inhibitor phlorizin (PZ) on the level of glucose (Glu) accumulated after 10 min inside everted sacs, prepared from rat intestine. ICI 118,551 blocks epinephrine-stimulated glucose transport. (Respective number of replicates n=16, n=10, n=7, n=6 for conditions shown in figure.) **c,** Effect of epinephrine (Epi), colchicine and phlorizin (PZ) on the level of glucose (Glu) accumulated after 10 min inside everted sacs prepared from rat intestine: colchicine inhibits Epi-stimulated Glu transport. (Respective number of replicates n=3, n=5, n=3, n=3 for conditions shown in figure.) **d,** Effect of epinephrine (Epi), the PKA inhibitor myristoylated PKI 14-22 amide (PKI), ICI 118,551 (ICI) and phlorizin (PZ) on the level of glucose (Glu) accumulated after 10 min inside everted sacs prepared from rat intestine: myristoylated PKI 14-22 amide inhibits Epi-stimulated Glu transport partially, while ICI 118,551 inhibits completely. (Respective number of replicates n=2, n=2, n=4, n=2 for conditions shown in figure). **e,** Calcium response elicited by 5 mM mannose in Flp-In-293 cells, stably transfected with *ADRB2* and *GNA15,* and incubated in Hank's Balanced Salt Solution (HBSS) throughout with glucose 5 mM (representative graph from n=3). Mannose elicits a $\beta_2$-AR-mediated calcium response. X-axis shows time in seconds and y-axis displays fluorescence intensities (arbitrary units) for 5 mM mannose and 5 mM mannose + 1 μM ICI 118,551. **f,** Calcium response elicited by 5 nM epinephrine or 5 mM mannose in Flp-In-293 cells, stably transfected with ADRB2 and GNA15, and incubated in different modifications of Hank's Balanced Salt Solution (HBSS), either with 5 mM glucose or 5 mM mannose throughout, or with no added sugar but 4 mM glutamine (as energy source) throughout. Mannose elicits a $\beta_2$-AR-mediated calcium response in HBSS containing either glucose or mannose throughout but not when either sugar is replaced by glutamine. X-axis shows different conditions: addition of 5 nM epinephrine or 5 mM mannose in HBSS with (1) 5 mM glucose (black) or (2) mannose (grey) or (3) with sugar replaced by glutamine (white) (n=2-3); y-axis represents peak relative fluorescence intensity (RFU, arbitrary units). **g,** Effect of phlorizin (PZ) and the dual inhibitor of SGLT1 and SGLT2, LX4211, on the level of glucose (Glu) accumulated after 10 min inside everted sacs, prepared from rat intestine. The presence of mannose enhances the level of glucose transported into the everted sacs. Mannose is virtually not detected by the glucose oxidase assay for glucose (about 100-fold lower sensitivity). (Respective number of replicates n=7, n=3, n=9, n=11, n=6 for conditions shown in figure.) **h,** Effect of mannose, ICI 118,551 (ICI) and phlorizin (PZ) on glucose (Glu) accumulated after 10 min inside everted sacs, prepared from rat intestine. ICI 118,551 inhibits mannose-stimulated Glu transport. (Respective number of replicates n=12, n=12, n=7, n=6 for conditions shown in figure.) The glucose transport for the different groups was expressed as a % of the glucose transport of the "glucose alone" group (for panel b, c, d, g and h). All values are expressed as mean ± SEM (or ± SD in panel a), $p < 0.001$:***; $p < 0.01$: **; $p < 0.05$: *; $p > 0.05$: ns.

**Figure 6 | STD-NMR evidence for direct glucose binding to $\beta_2$-AR.** (1) Region of the 1D $^1$H NMR spectrum, obtained with a pure glucose solution, using with water suppression and showing the glucose signals. (2) STD spectrum on a sample containing glucose and a membrane preparation with high expression of $\beta_2$-AR (Flp-In-293 cells) obtained by subtracting a spectrum with irradiation at 0.7 ppm, which saturates the receptor, from one with irradiation at 12 ppm, which does not affect the receptor. (3) STD control spectrum of a sample containing glucose and a membrane preparation without recombinant $\beta_2$-AR (HEK293 cells), obtained with identical parameters as the spectrum shown in (2). (4) STDD spectrum obtained by subtraction of the STD spectrum of the control sample without

$\beta_2$-AR (3) from that of the sample containing $\beta_2$-AR (2). The results show STD effects on the glucose ligand that arise from binding interaction with the $\beta_2$-AR.

**Figure 7 | Blood glucose levels after administration of glucose to rats in the presence and absence the non-bioavailable $\beta$-AR antagonist CD3-403. a,** Structure of the non-bioavailable $\beta$-AR antagonist CD3-403, a modified, more polar derivative of ICI 118,551. **b,** CD3-403 inhibits epinephrine-elicited $\beta_2$-AR-mediated calcium response *in vitro* in Flp-In-293 cells. X-axis shows time in s, and y-axis displays fluorescence intensities (arbitrary units) for 5 nM epinephrine and 5 nM epinephrine + 1.2 μM CD3-403 (representative graph from n=3). **c,** Concentration response curve for CD3-403 inhibition in Flp-In-293 cells, $IC_{50}$=46 ± 5 nM. X-axis shows log concentration CD3-403 (μM) and y-axis displays fluorescence intensities (arbitrary units), normalized to epinephrine response, which was set at 100% (n=3). A bolus of glucose 2 g/kg body weight (4 sets of 3 rats), **d,** or 4 g/kg body weight (1 set of 3 rats), **e,** was administered orally to sets of rats in the absence or presence of 5 μM (0.05 mg/kg) CD3-403. Glucose levels were measured in blood drawn from the tail vein at different time points after oral administration of the glucose bolus. The increase in blood glucose concentration is shown after the glucose load, normalized to the 0 min blood glucose concentration, which was set at 100 %. **f,** For the experiment with glucose 2 g/kg, the area under the curve (AUC) was calculated by setting 100% as a baseline. All values are expressed as mean ± SEM, $p < 0.05$: *.

**Figure 8 |** Effect of 1 μM epinephrine (Epi) and 1 μM epinephrine (Epi) together with 1 μM ICI 118,551 (ICI), a $\beta_2$-AR-specific inhibitor, on calcium response in HEK-293 cells. Epinephrine did not elicit a calcium response in HEK-293 cells via the endogenous β2-AR. X-axis shows time in s, and y-axis displays fluorescence intensity (arbitrary units) (n=3). All values are expressed as mean ± SEM.

**Figure 9 |** Effect of 1 μM ICI-118,551 (ICI), a $\beta_2$-AR-specific antagonist, in Flp-In-293 cells stably transfected with ADRB2 and GNA15. ICI-118,551 itself did not elicit a calcium response. Epinephrine (5 nM) was added as positive control, and Hank's Buffered Salt Solution (HBSS) as negative control. X-axis shows time in s and y-axis displays fluorescence intensity (arbitrary units) (n=3). All values are expressed as mean ± SEM.

**Figure 10 |** Absence of maltose response in *Xenopus* oocytes lacking heterologously expressed human $\beta_2$-AR. Forskolin/IBMX, which artificially increases the cAMP level, was added to demonstrate functionality of the CFTR channel.

**Figure 11 |** Binding (mean ± SEM) of $^{125}$I-cyanopindolol to vesicles containing $\beta_2$-AR. **a,** Stimulation of the binding with increasing concentrations of glucose (in the absence of $Na^+$), b, Stimulation of the binding with increasing concentrations of $Na^+$ (in the absence of glucose). Note that the Y-axis starts at 50%.

**Figure 12 |** Inhibition of epinephrine and sugar responses with $\beta_2$-AR-specific antagonists. **a,** 200 μM propranolol, **b,** 200 μM labetalol, c, 200 μM nadolol. 20 nM epinephrine or 70 mM sugar was added in the absence (●) or presence of inhibitor (○).

**Figure 13 |** Differential inhibition of the sugar response with a β1-antagonist, metoprolol (200 μM); 20 nM epinephrine or 70 mM sugar was added in the absence (●) or presence (○) of antagonist.

**Figure 14 |** Absence of inhibition of the epinephrine response and differential inhibition of the sugar response with β1-antagonists. **a,** 200 μM acebutolol, **b,** 200 μM atenolol. 20 nM epinephrine or 70 mM sugar was added in the absence (●) or presence (○) of antagonist.

**Figure 15 |** Effect of phlorizin (PZ) (100 μM) and LX4211 (2 μM) on glucose (Glu) accumulated after 75 min inside everted sacs, prepared from rat intestine. PZ and LX4211 inhibited more than 90 % of total glucose transport. (Respective number of replicates n=2, n=2, n=2 for conditions shown in the figure. For conditions with multiple measurements, the low and high values are indicated as the top and bottom of the box, with a horizontal line at the mean.)

**Figure 16 |** Effect of ICI-118,551 (ICI) and phlorizin (PZ) on glucose (Glu) accumulated after 10 min inside everted sacs, prepared from rat intestine. ICI itself did not have any effect on glucose transport. (Respective number of replicates n=6, n=7, n=3 for conditions shown in the figure; LLOQ is lower limit of quantification). The glucose transport for the different groups was expressed as a % of the glucose transport of the glucose control group. All values are expressed as mean ± SEM, $p > 0.05$: ns.

**Figure 17 |** GPCRs whose expression was detected in mouse proximal duodenal scrapings or in the intestinal STC-1 cell line cultured in medium with 5 or 25 mM glucose for the last 24 h. +, positive detection; -, no detection; /, not present on micro-array; Glu, glucose.

**Figure 18 |** Caco-2 cell permeability data of compound CD3-403 (apical to basal (A-B) and basal to apical (B-A), measured at pH 7.4 in both apical and basal compartment). Low permeability is defined as < $2x10^{-6}$ cm/s. Medium permeability is defined as $2x10^{-6}$ cm/s < permeability < $20x10^{-6}$ cm/s. High permeability is defined > $20x10^{-6}$ cm/s.

**Figure 19 |** PCR primers. Forward (fw) and reverse (re) primers used for reverse transcriptase PCR and for quantitative PCR (qPCR) were designed as required for SYBR Green (Eurogentec) qPCR reactions.

**Supplementary Table 2**

Caco-2 cell permeability data of compound CD3-403 (apical to basal (A-B) and basal to apical (B-A), measured at pH 7.4 in both apical and basal compartment)

**Supplementary Table 3**

PCR primers

**Supplementary Table 1**

**GPCRs whose expression was detected in mouse proximal duodenal scrapings or in the intestinal STC-1 cell line cultured in medium with 5 or 25 mM glucose for the last 24 h.**

| | **PCR** | | | **Micro-Array** | | **qPCR** | |
|---|---|---|---|---|---|---|---|
| | mouse | STC-1 | | STC-1 | | STC-1 | |
| | | 5 mM Glu | 25 mM Glu | 5 mM Glu | 25 mM Glu | 5 mM Glu | 25 mM Glu |
| **5-HT1b** | | | | + | + | | |
| **5-HT3a** | | | | + | + | | |
| **ADRB2** | + | + | | / | / | | |
| **AGTRL1** | + | | + | - | - | + | + |
| **ATIIR** | | | | - | + | | |
| **celsr3** | | | | + | + | | |
| **GPR1** | + | - | | + | + | | + |
| **GPR120** | + | - | | + | + | | + |
| **GPR125** | + | + | | / | / | | + |
| **GPR151** | + | | + | / | / | + | + |
| **GPR18** | - | - | | + | + | | + |
| **GPR19** | + | + | | + | + | | + |
| **GPR22** | - | - | | / | / | | + |
| **GPR35** | + | | + | / | / | + | + |
| **GPR39** | + | - | | + | + | | + |
| **GPR40** | + | + | | / | / | | + |
| **GPR43** | + | + | | + | + | | |
| **GPR48** | + | + | | + | + | | + |
| **GPR49** | - | + | | + | + | | + |
| **GPR55** | - | | - | / | / | | |

(continued)

| GPCRs whose expression was detected in mouse proximal duodenal scrapings or in the intestinal STC-1 cell line cultured in medium with 5 or 25 mM glucose for the last 24 h. | | | | | | | |
|---|---|---|---|---|---|---|---|
| | PCR | | | Micro-Array | | qPCR | |
| | mouse | STC-1 | | STC-1 | | STC-1 | |
| | | 5 mM Glu | 25 mM Glu | 5 mM Glu | 25 mM Glu | 5 mM Glu | 25 mM Glu |
| **GPR56** | + | + | | + | + | | + |
| **GPR61** | + | - | | + | + | | + |
| **GPR73** | - | | - | - | - | | |
| **GPR80** | + | - | | / | / | | + |
| **GPR85** | + | + | | + | + | | + |
| **GPR88** | - | - | | + | + | | + |
| **GPRC5C** | + | | + | + | + | + | + |
| **PGR22** | + | | + | / | / | | |
| **prosta E4** | | | | + | + | | |
| **RDC1** | + | | + | - | - | + | + |
| **TEM5** | - | | + | - | - | + | + |
| **TM7SF1** | + | - | | + | + | | + |
| **TM7SF2** | + | + | | + | + | | + |
| **TM7SF3** | + | + | | + | + | | + |
| **TPRA40** | + | | + | + | + | + | + |
| +, positive detection; -, no detection; /, not present on micro-array; Glu, glucose | | | | | | | |

**Supplementary Table 2**

| Caco-2 cell permeability data of compound CD3-403 (apical to basal (A-B) and basal to apical (B-A), measured at pH 7.4 in both apical and basal compartment) | | | | | | |
|---|---|---|---|---|---|---|
| **Compound** | **Test Concentration** | **A-B permeability** ($10^{-6}$ cm/s) | **Mean Recovery** (%) | **B-A permeability** ($10^{-6}$ cm/s) | **Mean Recovery** (%) | **Efflux** |
| CD3-403 | 1.0 μM | 0.4 | 83 | 1.3 | 94 | 3.25 |

| |
|---|
| Low permeability: < $2x10^{-6}$ cm/s |
| Medium permeability: $2x10^{-6}$ cm/s < permeability < $20x10^{-6}$ cm/s) |
| High permeability: > $20x10^{-6}$ cm/s |

**Supplementary Table 3**

**PCR primers**

Forward (fw) and reverse (re) primers used for reverse transcriptase PCR and for quantitative PCR (qPCR) were designed as required for SYBR Green (Eurogentec) qPCR reactions.

GPR1 fw TGGACCCCTTATCACCTGTTTAG
GPR1 re CCTGCAGCACATTCTGGAAA
GPR120 fw TCCGAGTGTCCCAACAAGACTA
GPR120 re ATGATGGGACTCCACATGATG
GPR125 fw CGTGCAGTTTCGAACAAACG

(continued)

**PCR primers**

Forward (fw) and reverse (re) primers used for reverse transcriptase PCR and for quantitative PCR (qPCR) were designed as required for SYBR Green (Eurogentec) qPCR reactions.

GPR125 re CTGGCCCGGTGTCCTTT
GPR18 fw GGAGAAGTCCATACGGATCATCA
GPR18 re GTGGAAGGGCACGAAGCA
GPR19 fw CTACCGCAGCAATGCCTACA
GPR19 re GATTTCCGAAATGCCCACAT
GPR22 fw TCGTGTTTGGTGTGAGAACTTCA
GPR22 re TCCCGGTGGCGTTTCA
GPR39 fw CCCATGGAGTTCTACAGCATCAT
GPR39 re CGTGTGGAGCTTACAGGACAGA
GPR40 fw TGCCTCCAATGTGGCTAGTTT
GPR40 re CCCTGTGATGAGTCCCAACTTC
GPR48 fw AAGACGACTGGAAGCTCCTGAA
GPR48 re CCGCCTTGGCTGCTGAT
GPR49 fw TGAGATCAAAACACGCGAGTCT
GPR49 re TGCTGGCGTGGGTAAAGG
GPR56 fw CCAAGGCTTCCTCATCTTCCT
GPR56 re GCGCTGTCTGAGTTGTTCTTCA
GPR61 fw AACCTGGATTGGCTACTTTTGC
GPR61 re TAAGCTCGCCCCGGATCT
GPR80 fw CCTTTGGCAACCTGCTGTTATAT
GPR80 re CCGCTGGCTTTGCATCTC
GPR85 fw TTGCACGAGGGCCTGTAGTAC
GPR85 re ATTCCTGCTTGGGCGAAACT
GPR88 fw GCTCTACACGTGGAGGAACGA
GPR88 re GTTGCGCCTGGGACATG
TM7SF1 fw CCCCCGAAGATATGACAGTGAT
TM7SF1 re GGAGCAAAACTTCCCTGAAGTC
TM7SF2 fw GCAGCAGTGCCTCCAAAAGT
TM7SF2 re GGTAAGGCACACGCTTGCA
TM7SF3 fw GAAAGAGGACAGCCGCCTTT
TM7SF3 re GATGTTGGTCACTCTGCGTTCTC

**Structural activity relationship (SAR) for CD3 compounds**

**Protocol**

- Cell line: HEK293 cells stably transfected with β2AR
- Epinephrine concentration: 5 nM
- Number of cells/well: 40,000
- Instrument: Flexstation II (Molecular Devices)
- Fluo-4 NW Calcium Assay Kit (Molecular Probes - Invitrogen)
- Inhibitor pre-incubation time: 45 min
- Data analysis: GraphPad Prism 5

**CD3 compounds overview**

Concentration used for inhibitors: 33.33 microM
Note: Numbers in red colour indicates toxicity to cells

$$K_i = \frac{IC_{50}}{\frac{[A]}{EC_{50}} + 1}$$

Ki: Inhibition constant
$IC_{50}$: Half maximal inhibitory concentration
[A]: Fixed concentration of agonist
$EC_{50}$:Half maximal activation of receptor (0.001 nM)

| Conc. (mM) | % INHIBITION | IC50 (microM) | Ki (microM) |
|---|---|---|---|
| A | 29 | - | - |
| B | 100 | - | - |
| C | 67 | 18.6 | 0.003719 |
| D | 91 | - | - |
| E (Precipitated) | 96 | 0.5 | 1E-04 μM |
| F | 76 | - | - |
| G | 100 | 0.71 | 0.000142 |
| H | 96 | 2.3 | 0.00046 |
| I | -2 | - | - |
| J | 0 | - | - |
| K | 0 | - | - |
| L | 12 | - | - |
| M | 51 | 28 | 0.005599 |
| N | 4 | - | - |
| O | 46 | - | - |
| P | -11 | - | - |
| S | 5 | - | - |

**CD3 compounds overview**

| Conc. (mM) | % INHIBITION | IC50 (microM) | Ki (microM) |
|---|---|---|---|
| T | 97 | 0.04 | 8E-06 |
| U | 72 | 44 | 0.008798 |
| W | 48 | - | - |
| X | 95 | 0.07 | 1.4E-05 |
| Y | -4 | - | - |
| Z | 97 | 0.02 | 4E-06 |
| ALPHA | 79 | 16.3 | 0.003259 |
| BETA | 36 | - | - |
| GAMMA | -3 | - | - |
| ICI-118551 | - | 0.004 | 8E-07 |
| Butoxamine | - | 14.3 | 0.002859 |

# Compounds with $IC_{50}$ <100nM

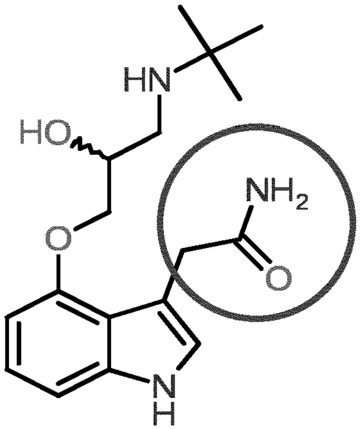

**CD3-T (CIM031403)**
$IC_{50}$= 40 nM
Ki= 8E-06 µM

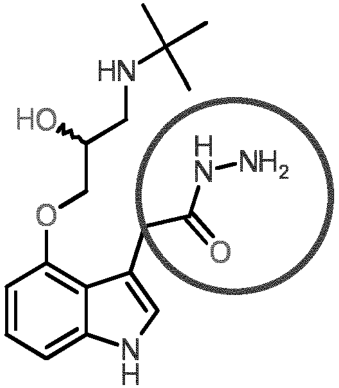

**CD3-X (CIM031413)**
$IC_{50}$= 70 nM
Ki= 1.4E-05 µM

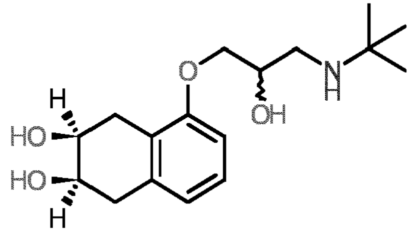

**CD3-Z (CIM031477)**
$IC_{50}$= 20 nM
Ki= 4E-06 µM

[redacted]

**ICI-118551**
$IC_{50}$= 4nM
Ki= 8E-07 µM

[redacted]

**Butaxamine**
$IC_{50}$= 14.3 µM
Ki= 0.002859 µM

-Amide or hydrazide group not look essential for the activity
-Nitrogen of heterocyclic ring not look essential in the ring

**Compounds with $IC_{50}$ >100nM**

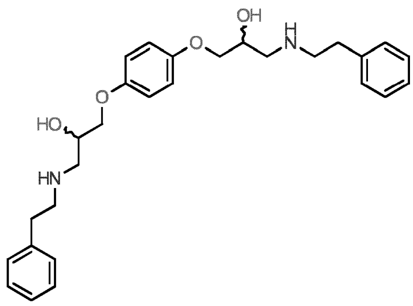

**CD3-C (CIM031231)**
$IC_{50}$= 18.6 μM
Ki= 0.003719 μM

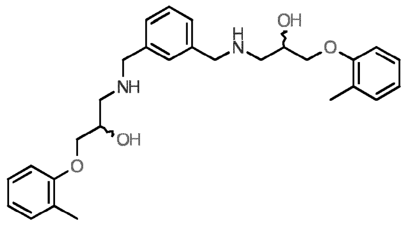

**CD3-G(CIM031239)**
$IC_{50}$= 0.71 μM
Ki= 0.000142 μM

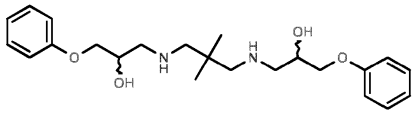

**CD3-H (CIM031240)**
$IC_{50}$= 2.3 μM
Ki= 0.00046μM

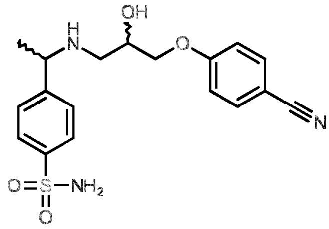

**CD3-M(CIM031291)**
$IC_{50}$= 28 μM
Ki= 0.005599 μM

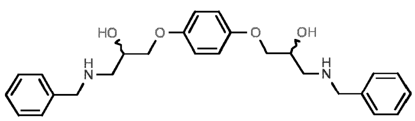

**CD3-U (CIM031407)**
$IC_{50}$= 44 μM
Ki= 0.008798 μM

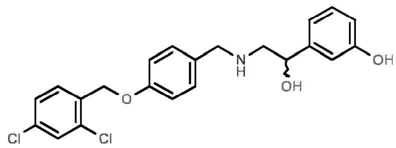

**CD3-alpha (CIM007167)**
$IC_{50}$= 16.3 μM
Ki= 0.003259 μM

**Compounds with $IC_{50}$ >100nM**

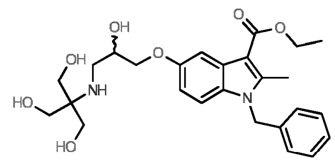

**CD3-A (CIM031108)**
% Inhibition= 29

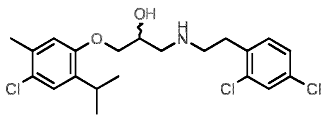

**CD3-E (CIM031237)**
$IC_{50}$= 0.5 μM
Ki= 1E-04 μM
(Precipitated)

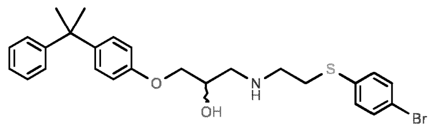

**CD3-F (CIM031238)**
% Inhibition= 76

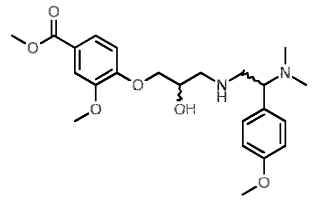

**CD3-I (CIM031246)**
% Inhibition= -2

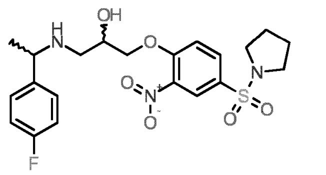

**CD3-J (CIM031248)**
% Inhibition= 0

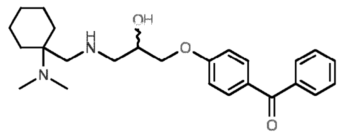

**CD3-K (CIM031271)**
% Inhibition= 0

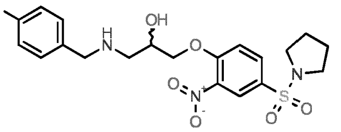

**CD3-L (CIM031275)**
% Inhibition= 12

**Compounds with $IC_{50}$ >100nM**

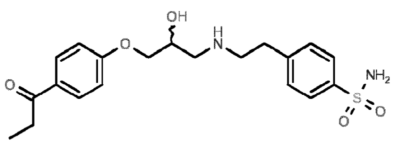

**CD3-N (CIM031309)**
% Inhibition= 4

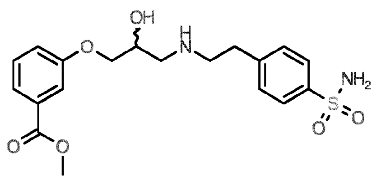

**CD3-O(CIM031312)**
% Inhibition= 46

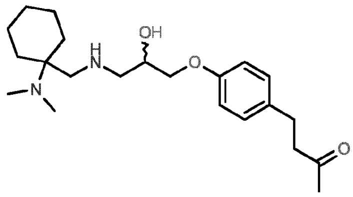

**CD3-P(CIM031313)**
% Inhibition= -11

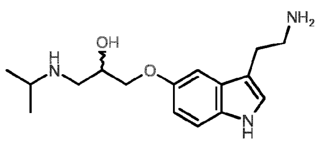

**CD3-S(CIM031402)**
% Inhibition= 5

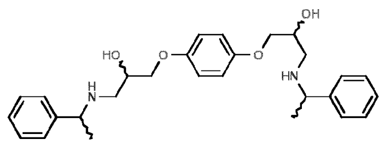

**CD3-W(CIM031412)**
% Inhibition= 48

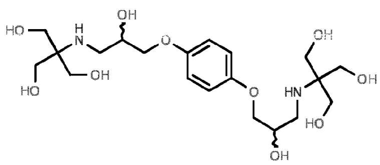

**CD3-Y(CIM031433)**
% Inhibition= -4

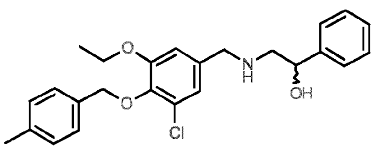

**CD3-γ(CIM033135)**
% Inhibition= -3

**Precipitated compounds**

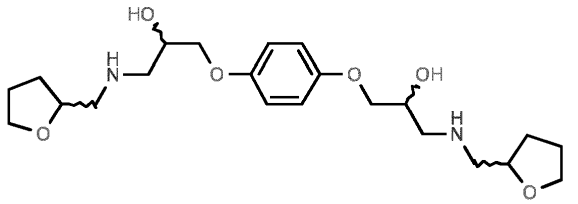

**CD3-Q(CIM031338)**
% Inhibition= 7

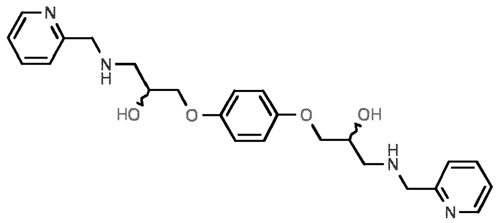

**CD3-R(CIM031359)**
% Inhibition= 8

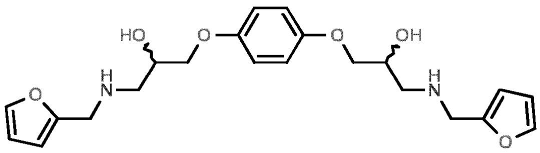

**CD3-V(CIM031411)**
% Inhibition=10

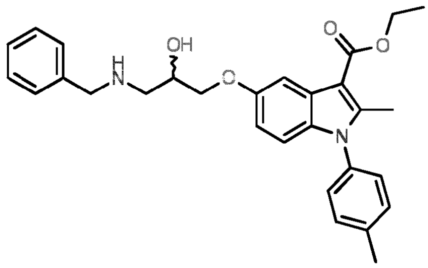

**CD3-Ω(CIM027127)**
% Inhibition= 18

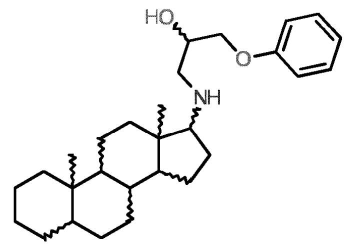

**CD3-β(CIM032727)**
% Inhibition= 36

**Toxic compounds**

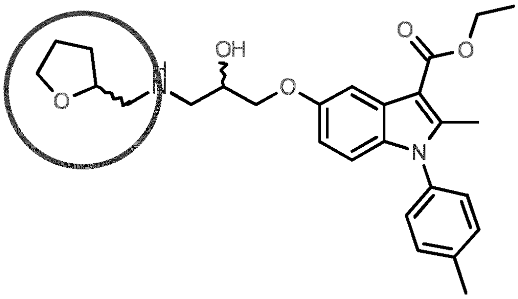

**CD3-B (CIM031110)**
% Inhibition= 100 (toxicity)

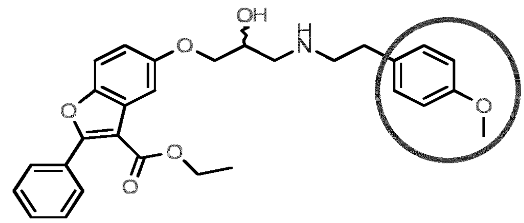

**CD3-D (CIM031234)**
% Inhibition= 91(little toxicity)

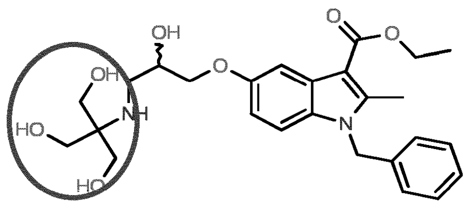

**CD3-A (CIM031108)**
% Inhibition= 29 **(Nontoxic compound)**

**CD3 Compounds (Interested)**

# Concentration response curve CD3-T new and old compound CIM-031403

| Parameter | Value | Std. error |
|---|---|---|
| IC50 (nM)1st. experiment | 221 | 199-244 |
| IC50 (nM)2nd. experiment | 46 | 41-50 |
| IC50 (nM) old stock | 40 | 37-51 |

| STRUCTURE | Client Compound I.D. | Test Concentration (M) | A-B permeability (Caco-2, pH 7.4/7.4) | MeanRecovery | B-A permeability (Caco-2, pH 7.4/7.4) | MeanRecovery | Efflux |
|---|---|---|---|---|---|---|---|
| 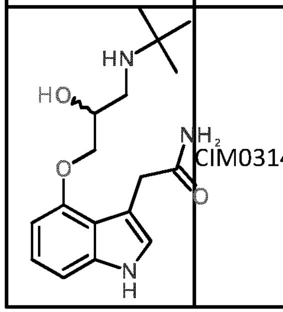 | CIM031403_02_01 | 1.0E-06 | 0.4 | 83 | 1.3 | 94 | 3.25 |

**Concentration response curve CD3-T1 (CIM-012783_03_01)**

| Parameter | Value | Std. error |
| --- | --- | --- |
| **IC50 (nM)** 1st expt | 6 | 5-8 |
| **IC50 (nM) 2nd expt** | 0.6 | 0.56-0.64 |

| STRUCTURE | Client Compound I.D. | Test Concentration (M) | A-B permeability (Caco-2, pH 7.4/7.4) | MeanRecovery | B-A permeability (Caco-2, pH 7.4/7.4) | MeanRecovery | Efflux |
|---|---|---|---|---|---|---|---|
| 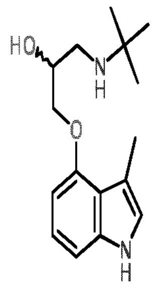 | CIM012783_03_01 | 1.0E-06 | 54.7 | 71 | 17.8 | 87 | 0.325411335 |

**Concentration response curve CD3-T2 (CIM-121255_01_02)**

| Parameter | Value | Std. error |
|---|---|---|
| **IC50 (nM)** | 3801 | 2733-5248 |

# Concentration response curve CD3-T3 (CIM-121256_01_01)

| Parameter | Value | Std. error |
|---|---|---|
| IC50 (nM) 1st expt | 66 | 60-74 |
| IC50 (nM) 2nd expt. | 78 | 70-86 |

| STRUCTURE | Client Compound I.D. | Test Concentration (M) | A-B permeability (Caco-2, pH 7.4/7.4) | MeanRecovery | B-A permeability (Caco-2, pH 7.4/7.4) | MeanRecovery | Efflux |
|---|---|---|---|---|---|---|---|
| 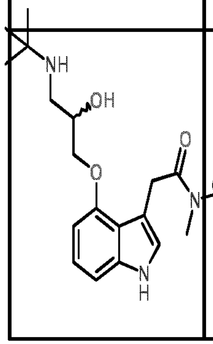 | CIM121256_01_01 | 1.0E-06 | 0.1 | 76 | 3.2 | 90 | 32 |

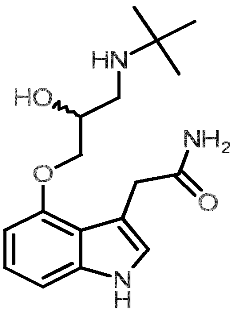

**CD3-T (CIM031403)**
$IC_{50}$= 40 nM

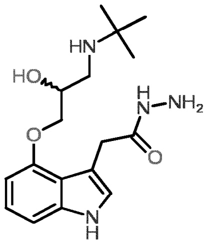

**CD3-X (CIM031413)**
$IC_{50}$= 70 nM
**Caco2** P(A-B): 0.2X$10^{-6}$ cm/s
P(B-A):0.5X$10^{-6}$ cm/s

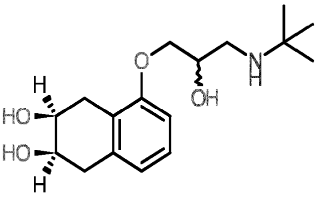

**CD3-Z (CIM031477) Nadolol**
$IC_{50}$= 20 nM
**Caco2** P(A-B): 0.56X$10^{-6}$ cm/s
Extent absorption: 35%

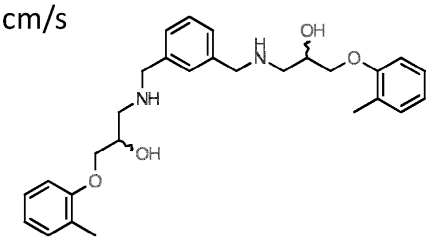

**CD3-G(CIM031239)**
$IC_{50}$= 0.71 μM
**Caco2** P(A-B): 0.9X$10^{-6}$ cm/s
P(B-A):3.4 X$10^{-6}$ cm/s

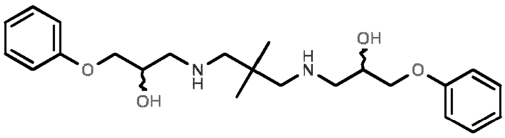

**CD3-H (CIM031240)**
$IC_{50}$= 2.3 μM

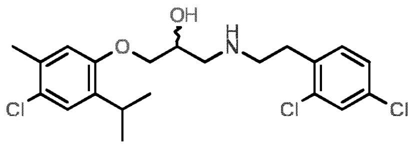

**CD3-E (CIM031237)**
$IC_{50}$= 0.5 μM
(Precipitated)

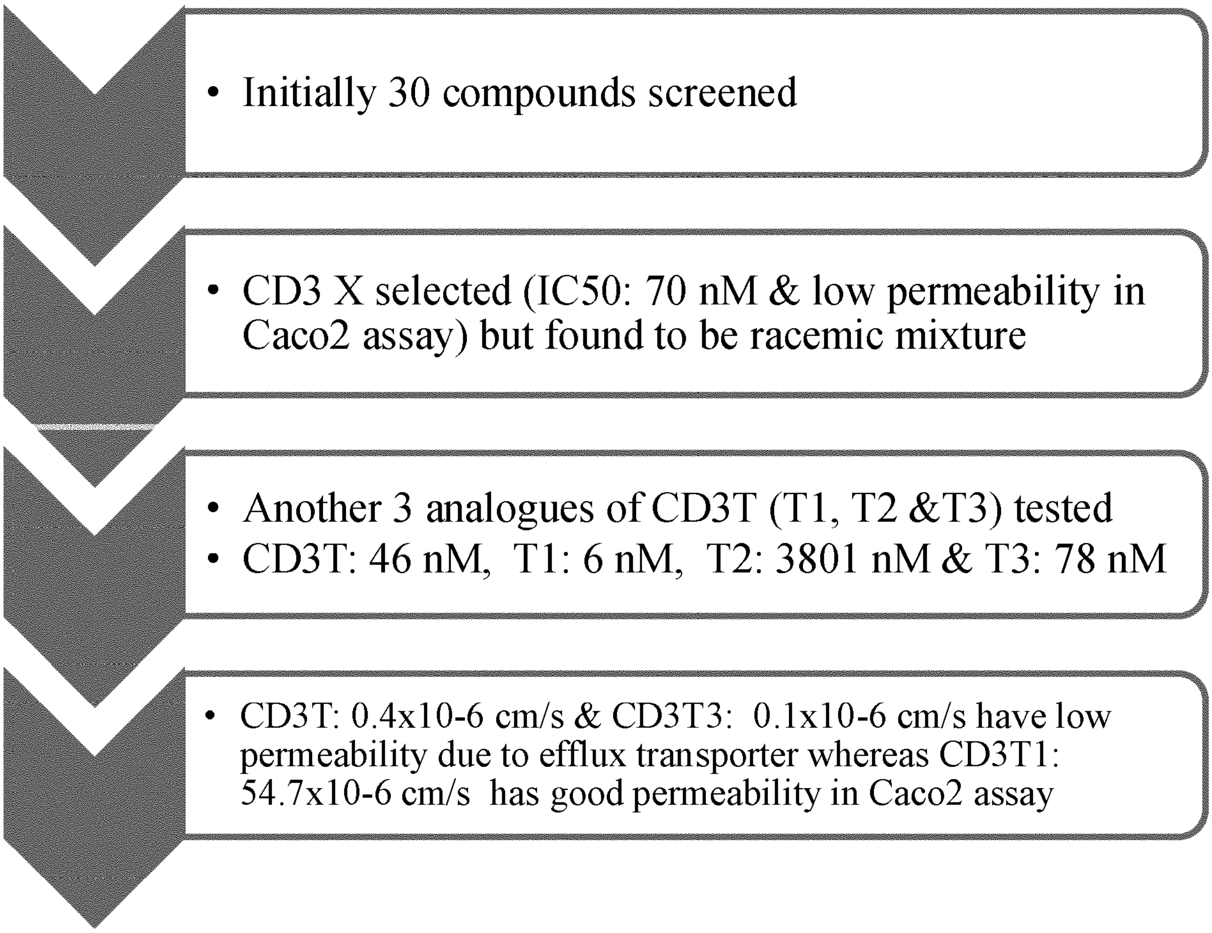


**Claims**

1. A β2-adrenergic receptor (β2-AR) antagonist for use in the treatment of at least one of diabetes and obesity.

2. A β2-AR antagonist for a use according to claim 1, wherein the β2-AR antagonist is an orally non-bioavailable β2-AR antagonist, wherein the non-bioavailable β2-AR antagonist is a β2-AR antagonist having minimal permeability through biological membranes, wherein the non-bioavailable β2-AR antagonist is a β2-AR antagonist has a permeability through biological membranes that is no more than 2, 1.5, 1.2, 1.0, 0.5, 0.4, 0.2 or 0.1 cm/s, as determined in a Caco-2 cell or other intestinal permeability assay.

3. A β2-AR antagonist for a use according to claim 1 or 2, wherein the β2-AR antagonist is CD3-403 (CIM-031403), CD3-T1 (CIM-012783_03_01), or CD3-T3 (CIM-121256_01_01)

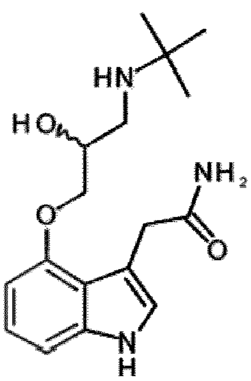

CD3-403 (CIM-031403)

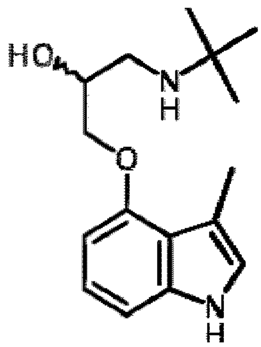

CD3-T1 (CIM-012783_03_01)

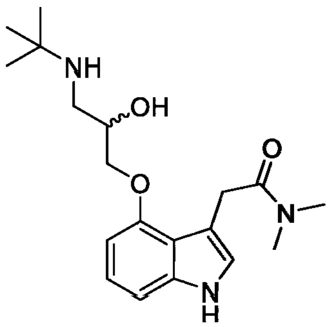

CD3-T3 (CIM-121256_01_01).

4. A β2-AR antagonist for a use according to any one of claims 1 - 3, wherein the diabetes is Type 1 diabetes diabetes.

5. A β2-AR antagonist for a use according to any one of claims 1 - 3, wherein the diabetes is Type 2 diabetes.

## Patentansprüche

1. ß2-adrenerger Rezeptor (ß2-AR)-Antagonist zur Anwendung bei der Behandlung von mindestens einem von Diabetes und Adipositas.

2. ß2-AR-Antagonist für eine Anwendung nach Anspruch 1, wobei der ß2-AR-Antagonist ein oral nicht bioverfügbarer ß2-AR-Antagonist ist, wobei der nicht bioverfügbare ß2-AR-Antagonist ein ß2-AR-Antagonist mit minimaler Permeabilität durch biologische Membranen ist, wobei der nicht bioverfügbare ß2-AR-Antagonist ein ß2-AR-Antagonist ist, der eine Permeabilität durch biologische Membranen aufweist, die nicht mehr als 2, 1,5, 1,2, 1,0, 0,5, 0,4, 0,2 oder 0,1 cm/s beträgt, bestimmt in einem Caco-2-Zell- oder einem anderen Darmpermeabilitätstest.

3. ß2-AR-Antagonist für eine Anwendung nach Anspruch 1 oder 2, wobei der ß2-AR-Antagonist CD3-403 (CIM-031403), CD3-T1 (CIM-012783_03_01) oder CD3-T3 (CIM-121256_01_01) ist

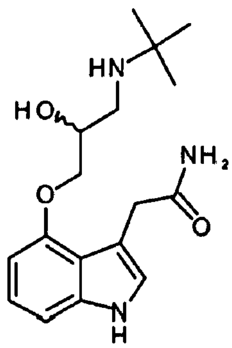

CD3-403 (CIM-031403)

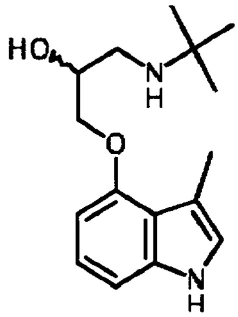

CD3-T1 (CIM-012783_03_01)

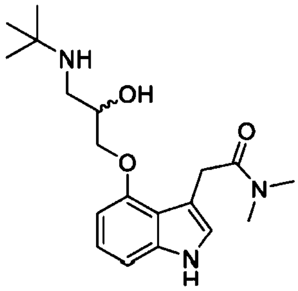

CD3-T3 (CIM-121256_01_01).

4. ß2-AR-Antagonist für eine Anwendung nach einem der Ansprüche 1 - 3, wobei es sich bei dem Diabetes um Typ-1-Diabetes handelt.

5. ß2-AR-Antagonist für eine Anwendung nach einem der Ansprüche 1 - 3, wobei es sich bei dem Diabetes um Typ-2-Diabetes handelt.

## Revendications

1. Antagoniste des récepteurs β2-adrénergiques (β2-AR) destiné à être utilisé dans le traitement d'au moins une des affections suivantes : diabète et obésité.

2. Antagoniste β2-AR pour une utilisation selon la revendication 1, où l'antagoniste β2-AR est un antagoniste β2-AR non biodisponible par voie orale, où l'antagoniste β2-AR non biodisponible est un antagoniste β2-AR ayant une perméabilité minimale à travers des membranes biologiques, où l'antagoniste β2-AR non biodisponible est un antagoniste β2-AR ayant une perméabilité à travers des membranes biologiques qui n'est pas supérieure à 2 ; 1,5 ; 1,2 ; 1,0 ; 0,5 ; 0,4 ; 0,2 ou 0,1 cm/s, telle que déterminée dans un test sur cellules Caco-2 ou un autre test de perméabilité intestinale.

3. Antagoniste β2-AR pour une utilisation selon la revendication 1 ou 2, où l'antagoniste β2-AR est le CD3-403 (CIM-031403), le CD3-T1 (CIM-012783_03_01), ou le CD3-T3 (CIM-121256_01_01)

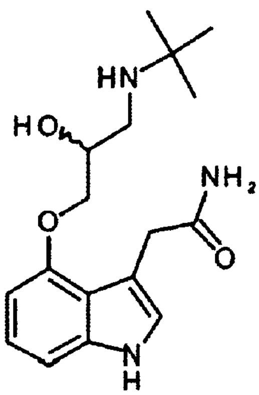

CD3-403 (CIM-031403)

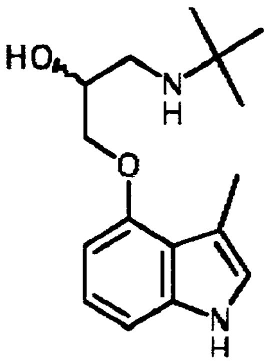

CD3-T1 (CIM-012783_03_01)

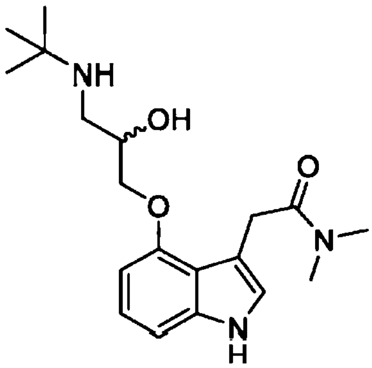

CD3-T3 (CIM-121256_01_01).

4. Antagoniste β2-AR pour une utilisation selon l'une quelconque des revendications 1 à 3, où le diabète est un diabète de type 1.

5. Antagoniste β2-AR pour une utilisation selon l'une quelconque des revendications 1 à 3, où le diabète est un diabète de type 2.

Fig. 1A

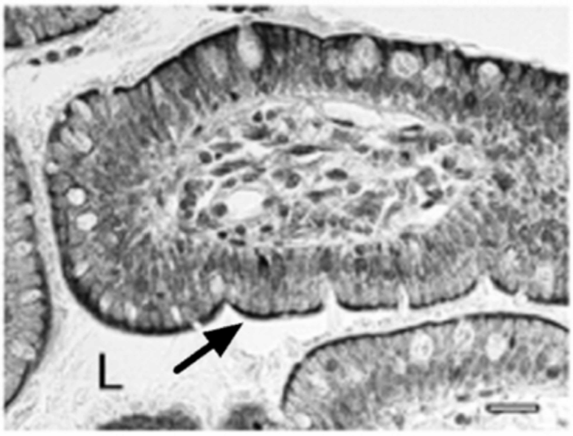


Fig. 1B

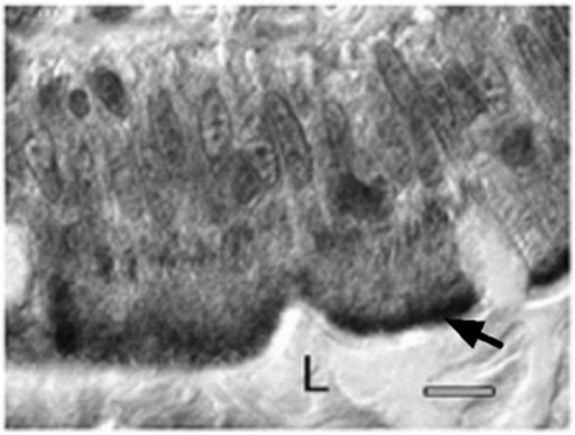


Fig. 1C

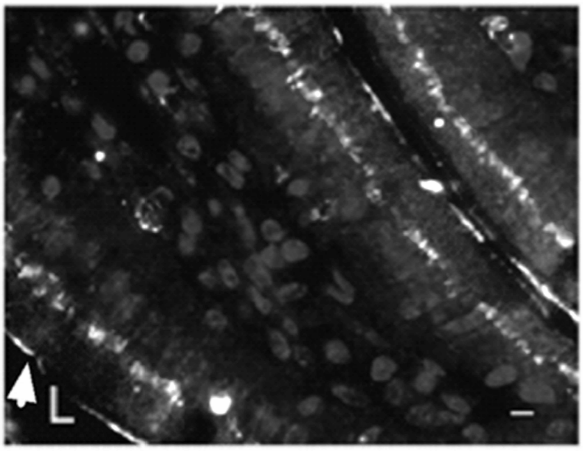


Fig. 1D

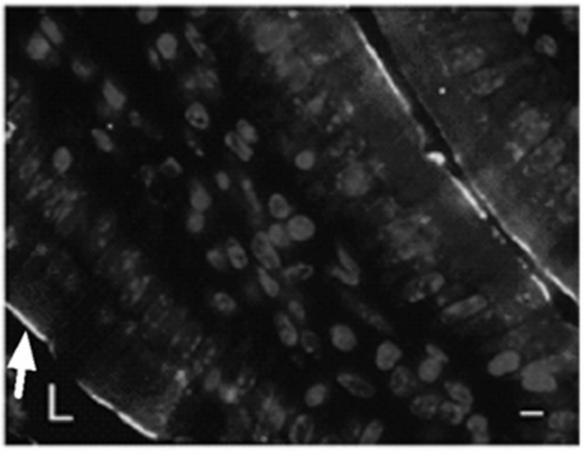


Fig. 1E

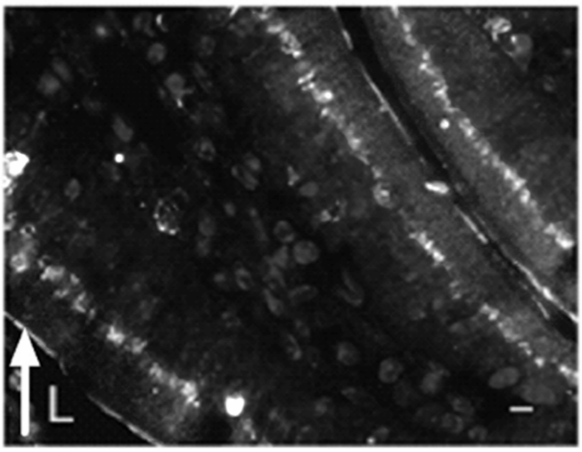

Fig. 2A

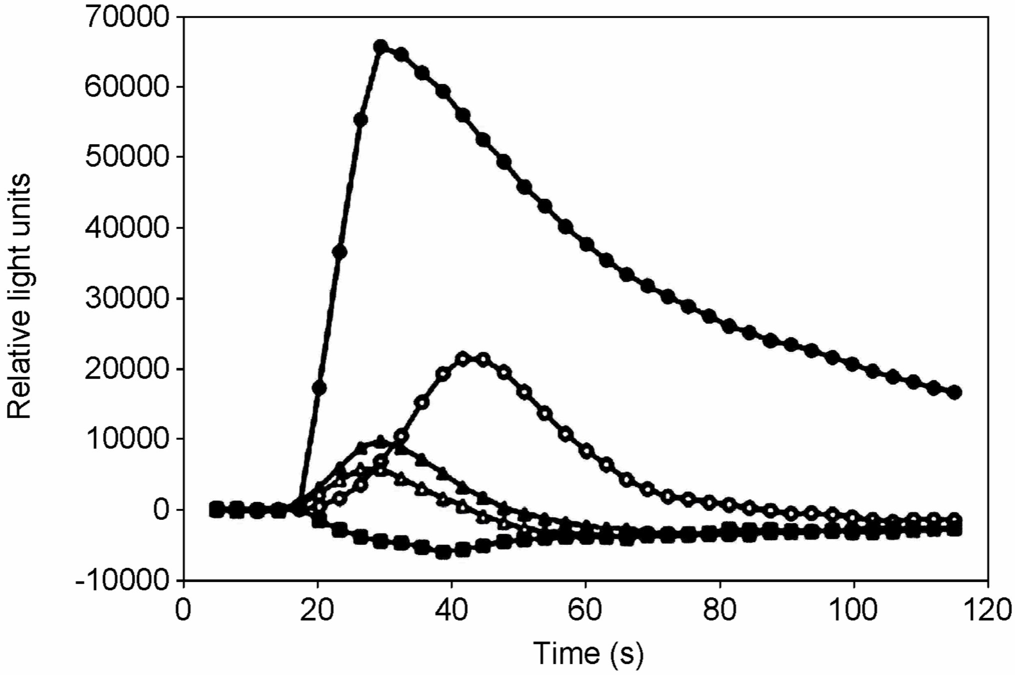
70000
60000
50000
40000
30000
20000
10000
0
-10000
Relative light units
0
20
40
60
80
100
120
Time (s)

Fig. 2B

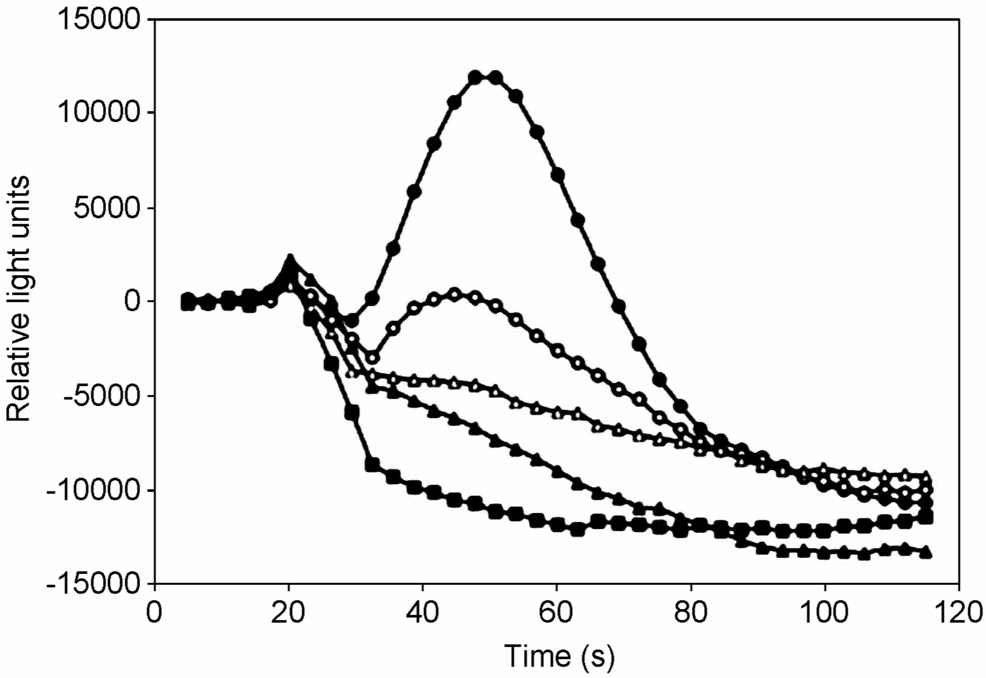
15000
10000
5000
0
-5000
-10000
-15000
Relative light units
0
20
40
60
80
100
120
Time (s)

Fig. 2C

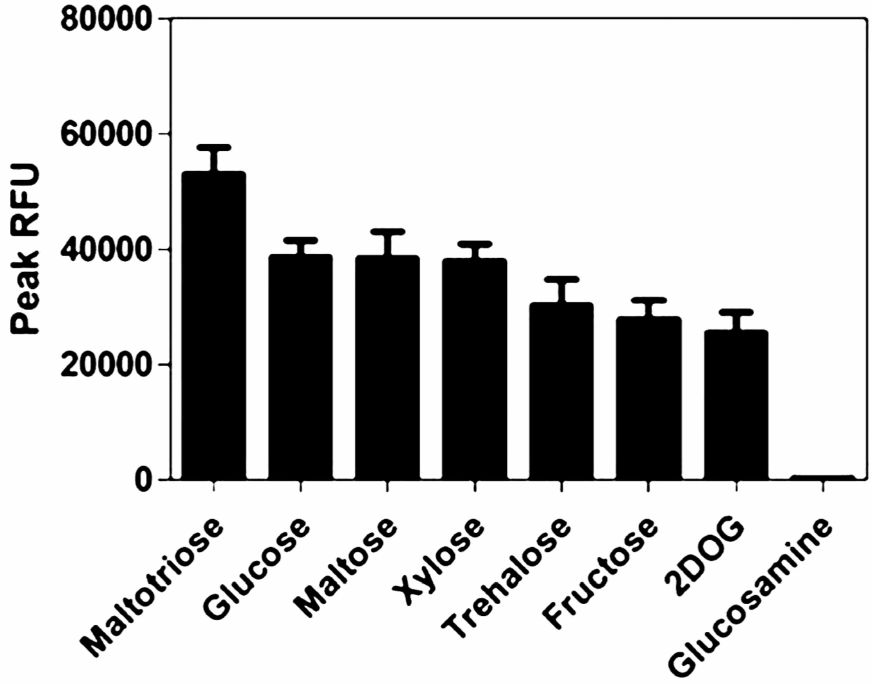
80000
60000
40000
20000
0
Peak RFU
Maltotriose
Glucose
Maltose
Xylose
Trehalose
Fructose
2DOG
Glucosamine

Fig. 2D

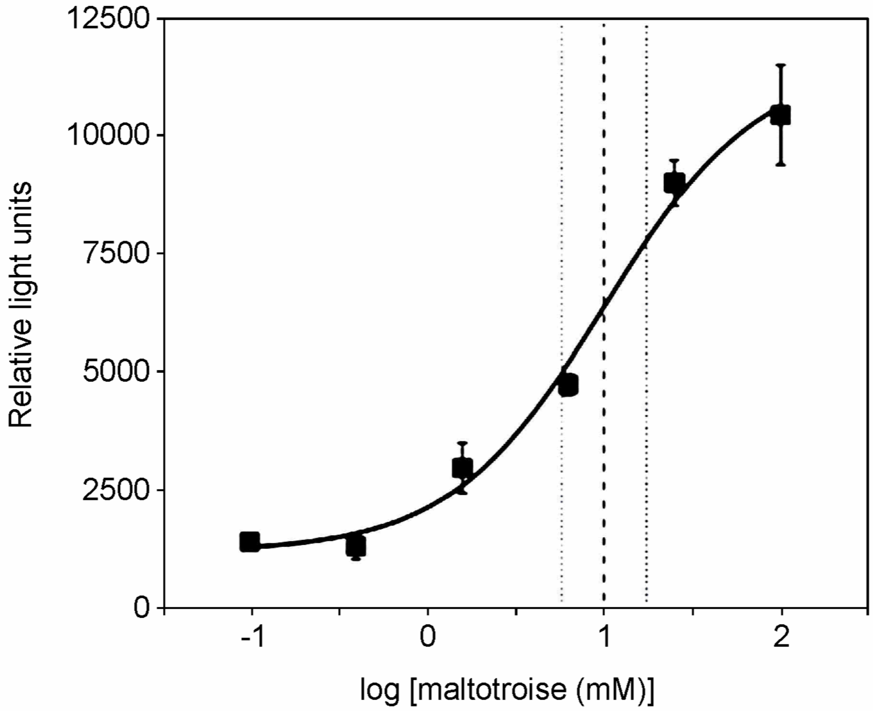
12500
10000
7500
5000
2500
0
Relative light units
-1
0
1
2
log [maltotroise (mM)]

Fig. 2E

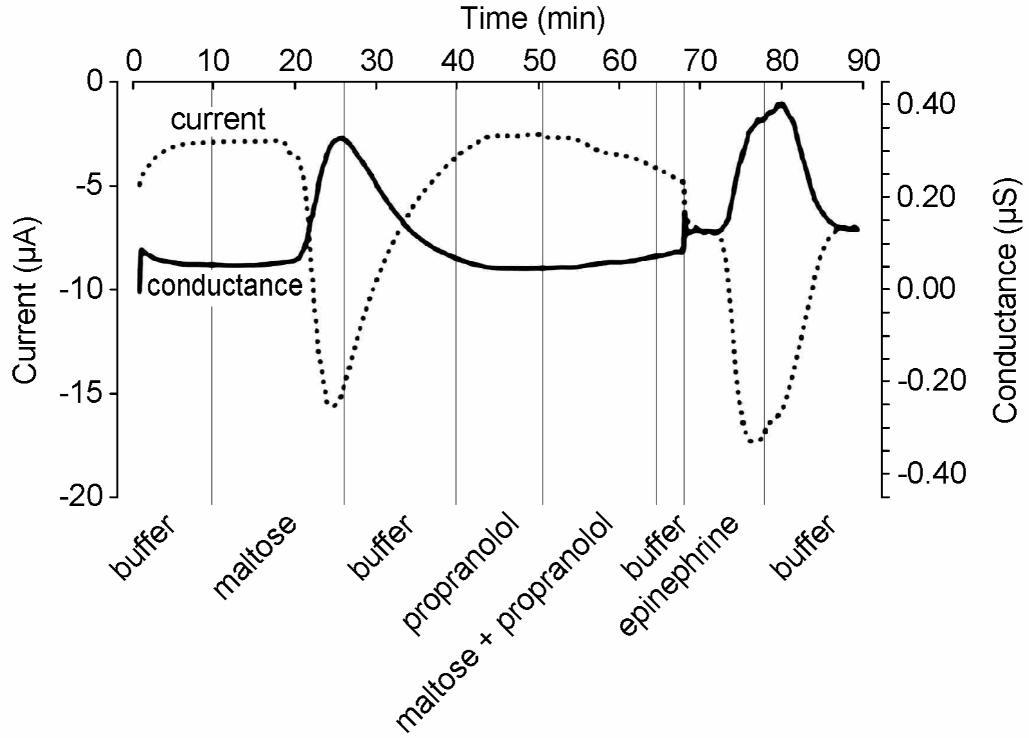
Time (min)
0 10 20 30 40 50 60 70 80 90
0 -5 -10 -15 -20
Current (µA)
0.40 0.20 0.00 -0.20 -0.40
Conductance (µS)
current
conductance
buffer
maltose
buffer
propranolol
maltose + propranolol
buffer
epinephrine
buffer

Fig. 2F

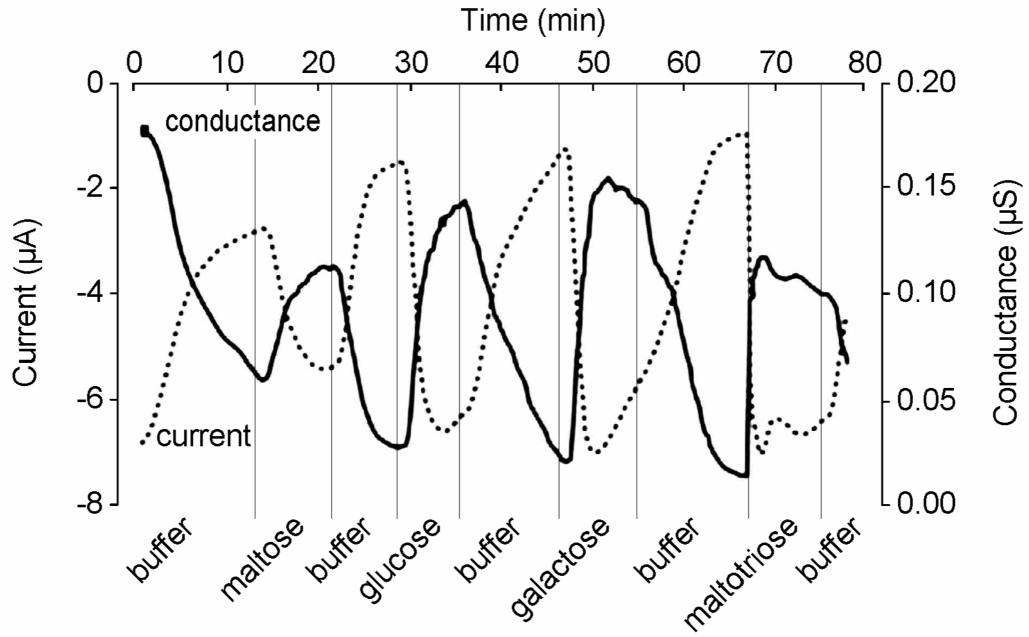
Time (min)
0 10 20 30 40 50 60 70 80
0 -2 -4 -6 -8
Current (µA)
0.20 0.15 0.10 0.05 0.00
Conductance (µS)
conductance
current
buffer
maltose
buffer
glucose
buffer
galactose
buffer
maltotriose
buffer

Fig. 3A

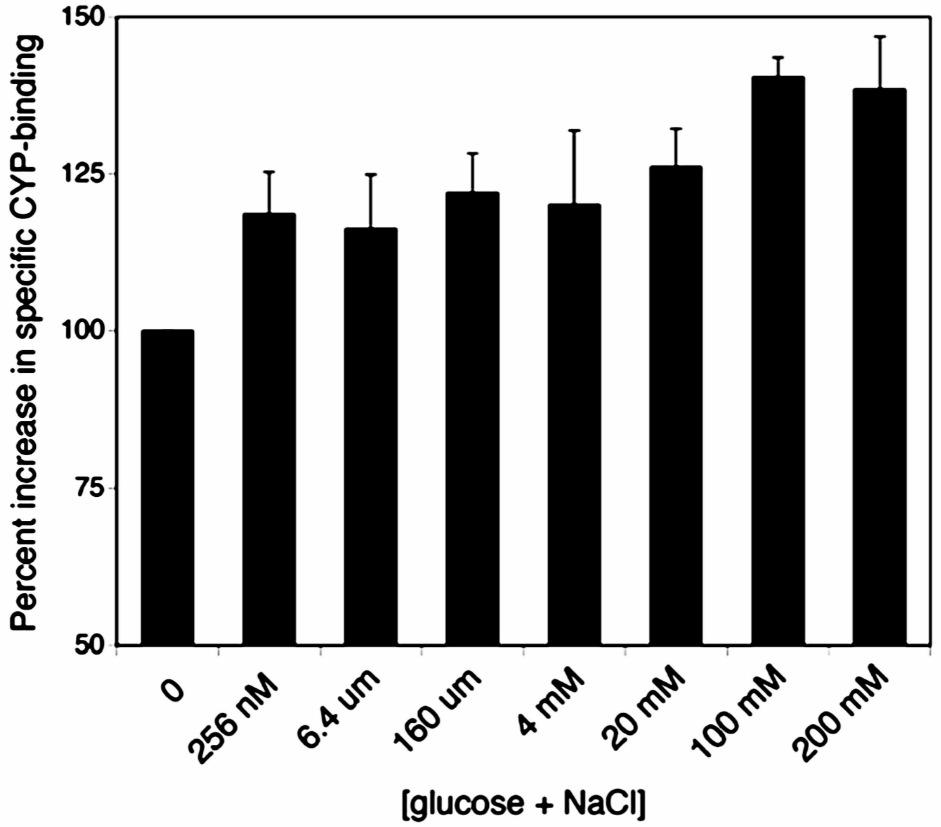
150
125
100
75
50
Percent increase in specific CYP-binding
0
256 nM
6.4 um
160 um
4 mM
20 mM
100 mM
200 mM
[glucose + NaCl]

Fig. 3B

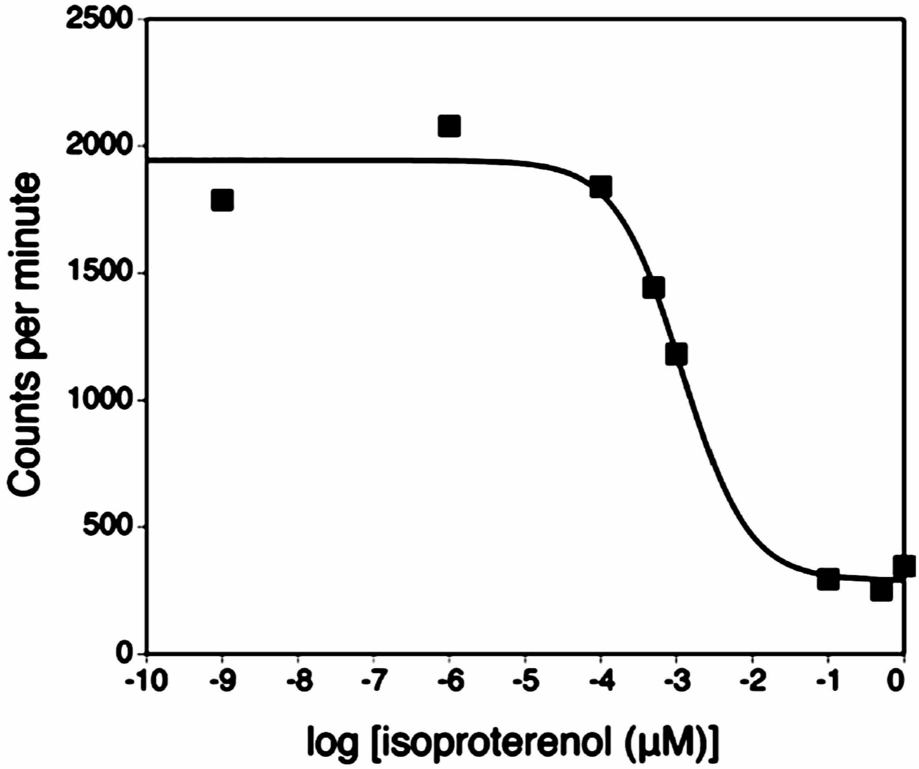
2500
2000
1500
1000
500
0
Counts per minute
-10
-9
-8
-7
-6
-5
-4
-3
-2
-1
0
log [isoproterenol (µM)]

Fig. 4A

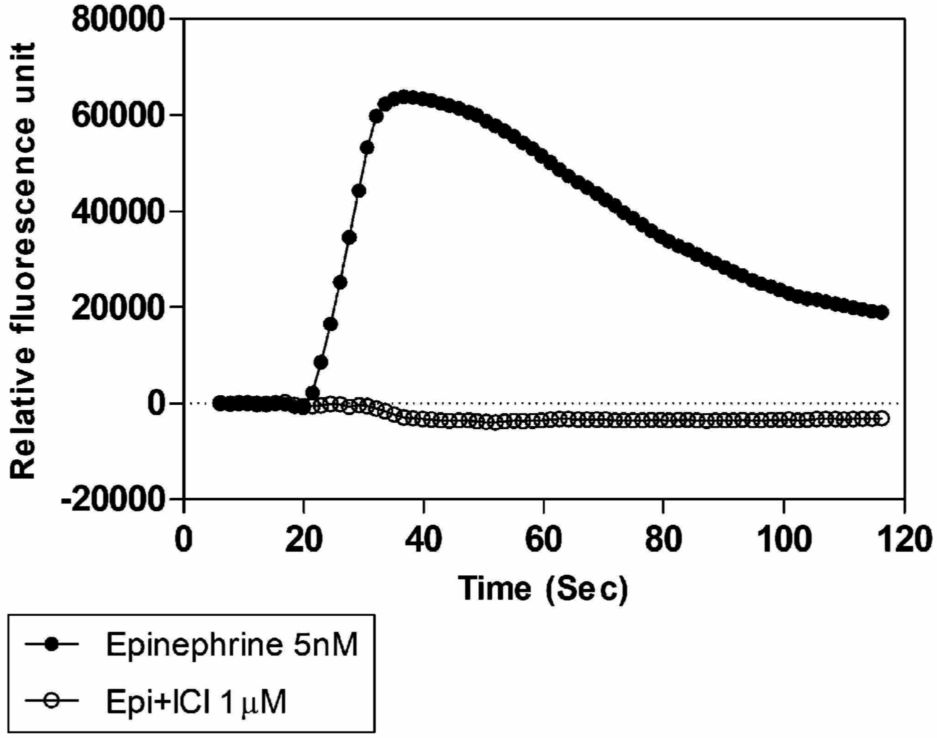
Relative fluorescence unit
80000
60000
40000
20000
0
-20000
0
20
40
60
80
100
120
Time (Sec)
Epinephrine 5nM
Epi+ICI 1 µM

Fig. 4B

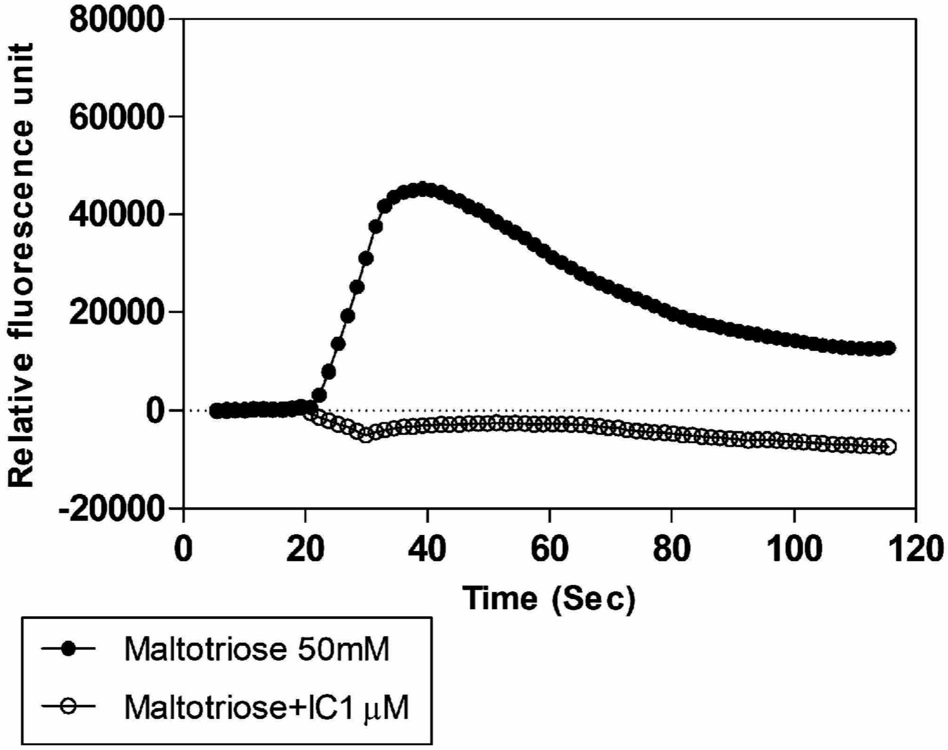
Relative fluorescence unit
80000
60000
40000
20000
0
-20000
0
20
40
60
80
100
120
Time (Sec)
Maltotriose 50mM
Maltotriose+IC1 µM

Fig. 4C

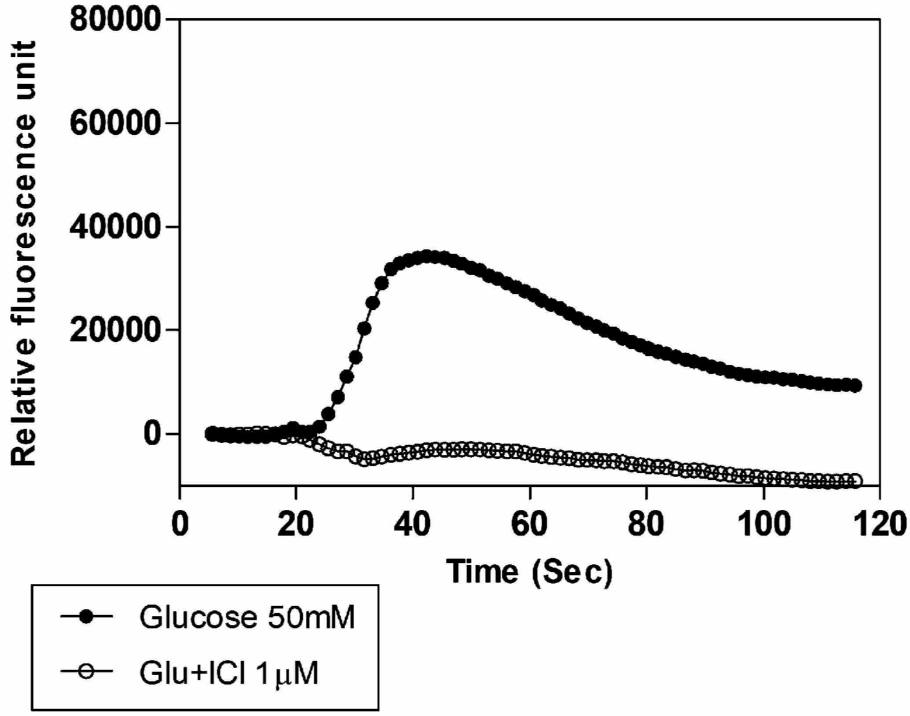
80000
60000
40000
20000
0
Relative fluorescence unit
0
20
40
60
80
100
120
Time (Sec)
Glucose 50mM
Glu+ICI 1μM

Fig. 4D

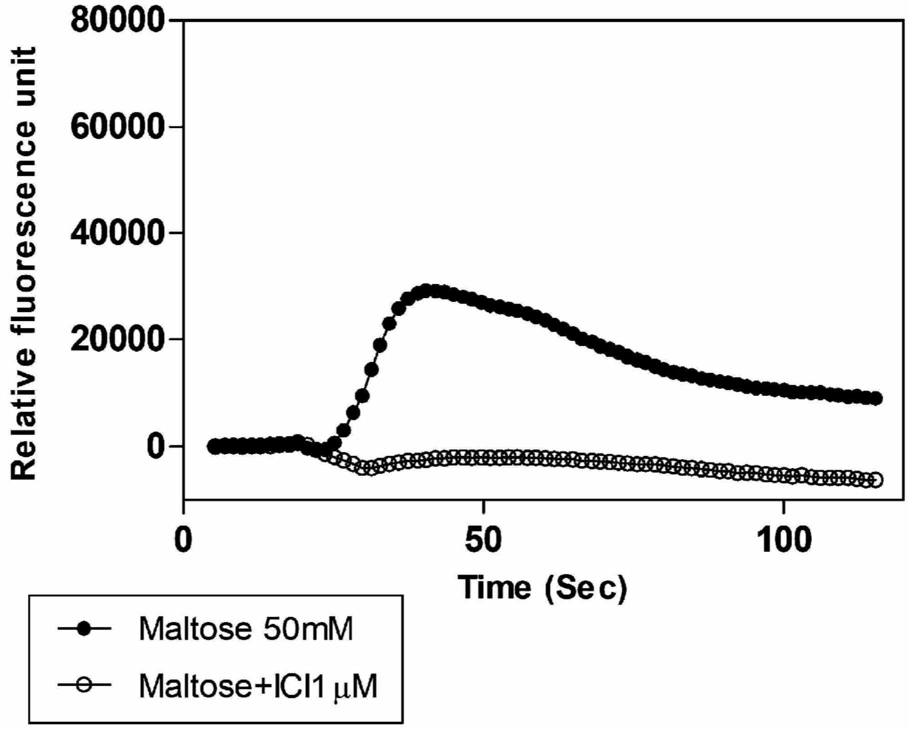
80000
60000
40000
20000
0
Relative fluorescence unit
0
50
100
Time (Sec)
Maltose 50mM
Maltose+ICI1 μM

Fig. 4E

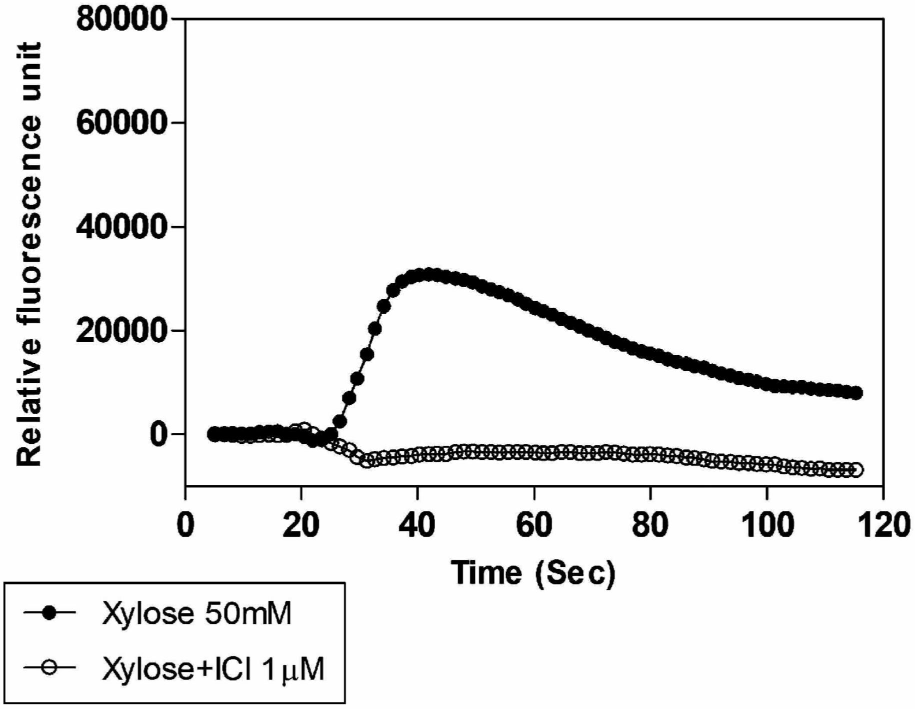
80000
60000
40000
20000
0
Relative fluorescence unit
0
20
40
60
80
100
120
Time (Sec)
Xylose 50mM
Xylose+ICI 1 μM

Fig. 4F

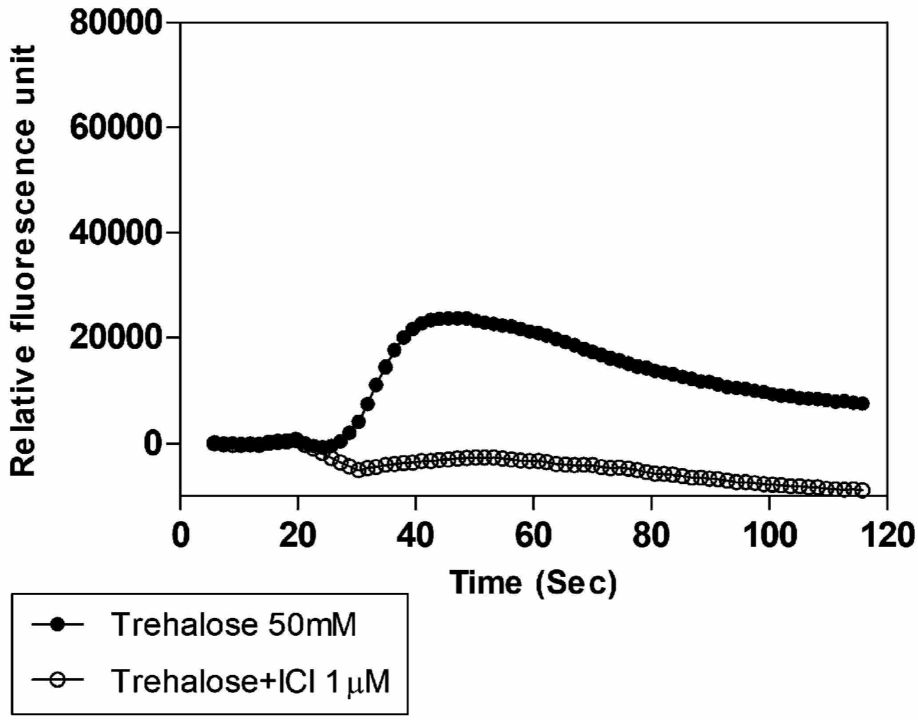
80000
60000
40000
20000
0
Relative fluorescence unit
0
20
40
60
80
100
120
Time (Sec)
Trehalose 50mM
Trehalose+ICI 1 μM

Fig. 4G

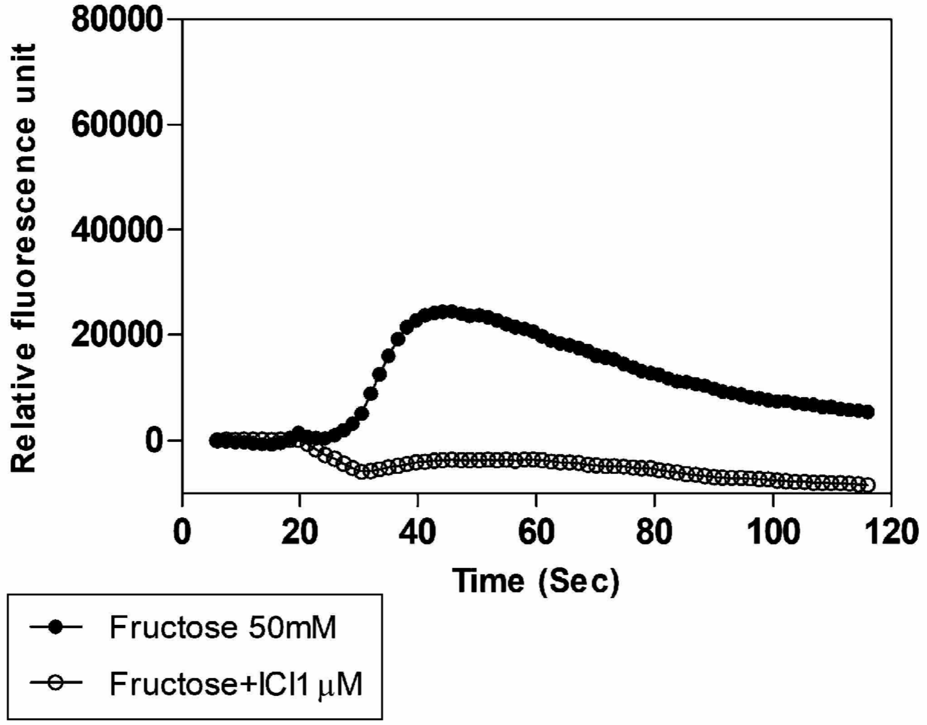
80000
60000
40000
20000
0
Relative fluorescence unit
0
20
40
60
80
100
120
Time (Sec)
Fructose 50mM
Fructose+ICI1 μM

Fig. 4H

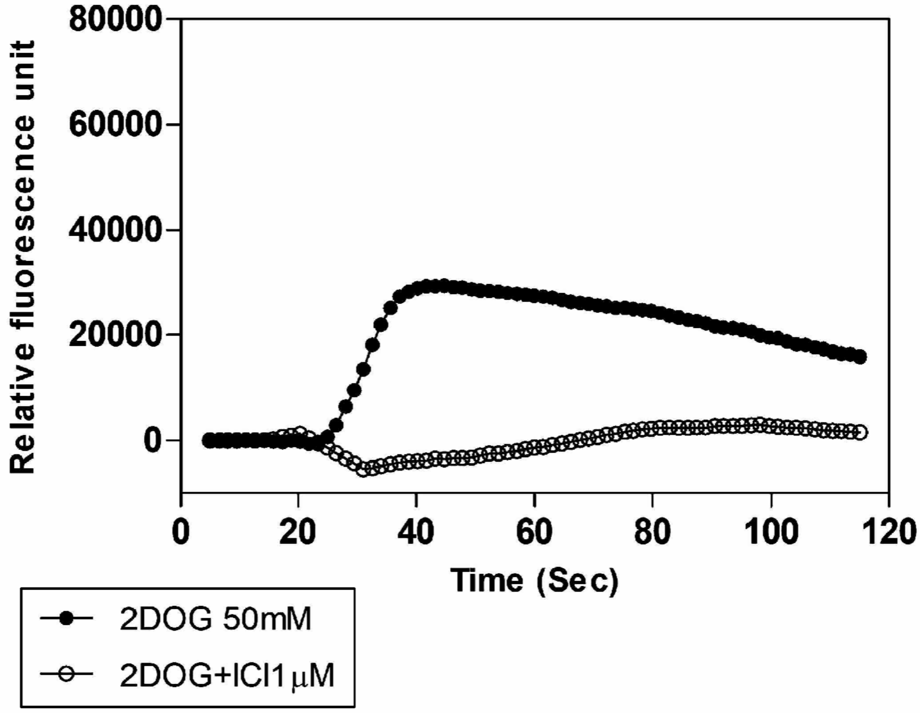
80000
60000
40000
20000
0
Relative fluorescence unit
0
20
40
60
80
100
120
Time (Sec)
2DOG 50mM
2DOG+ICI1 μM

Fig. 5A

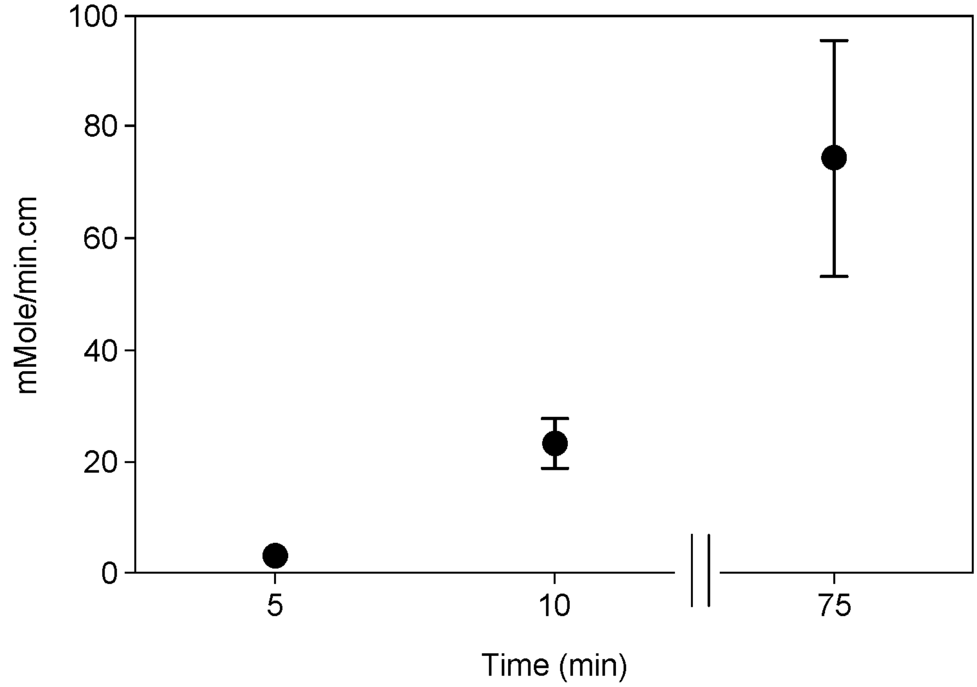
100
80
60
40
20
0
mMole/min.cm
5
10
75
Time (min)

Fig. 5B

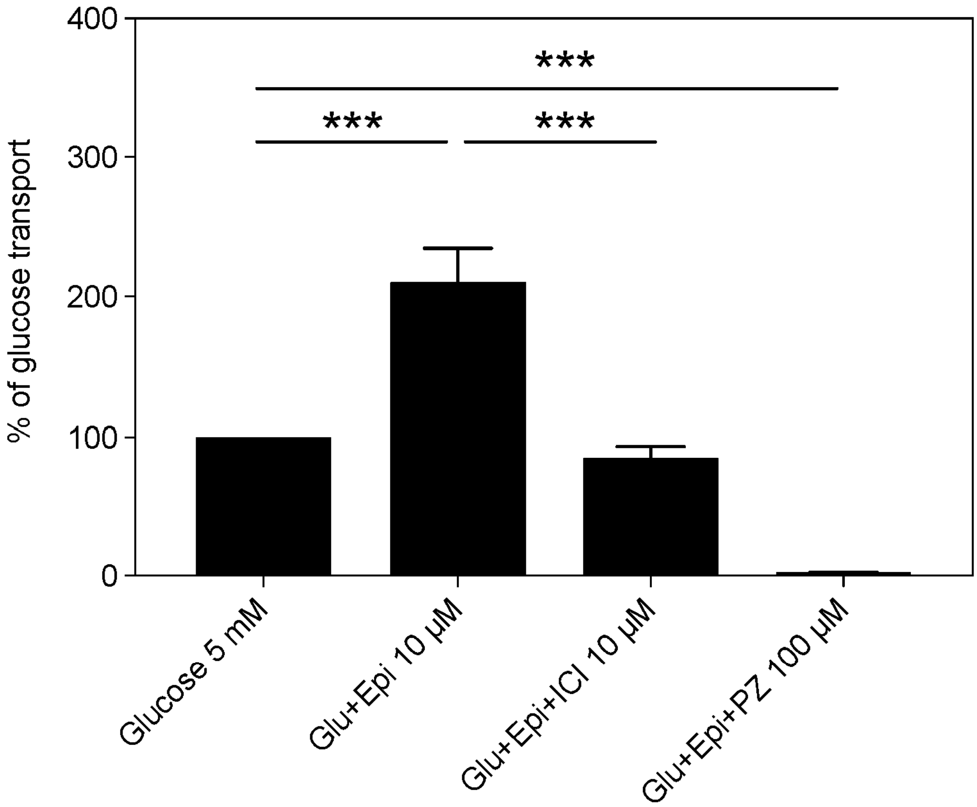
400
300
200
100
0
% of glucose transport
***
***
***
Glucose 5 mM
Glu+Epi 10 µM
Glu+Epi+ICI 10 µM
Glu+Epi+PZ 100 µM

Fig. 5C

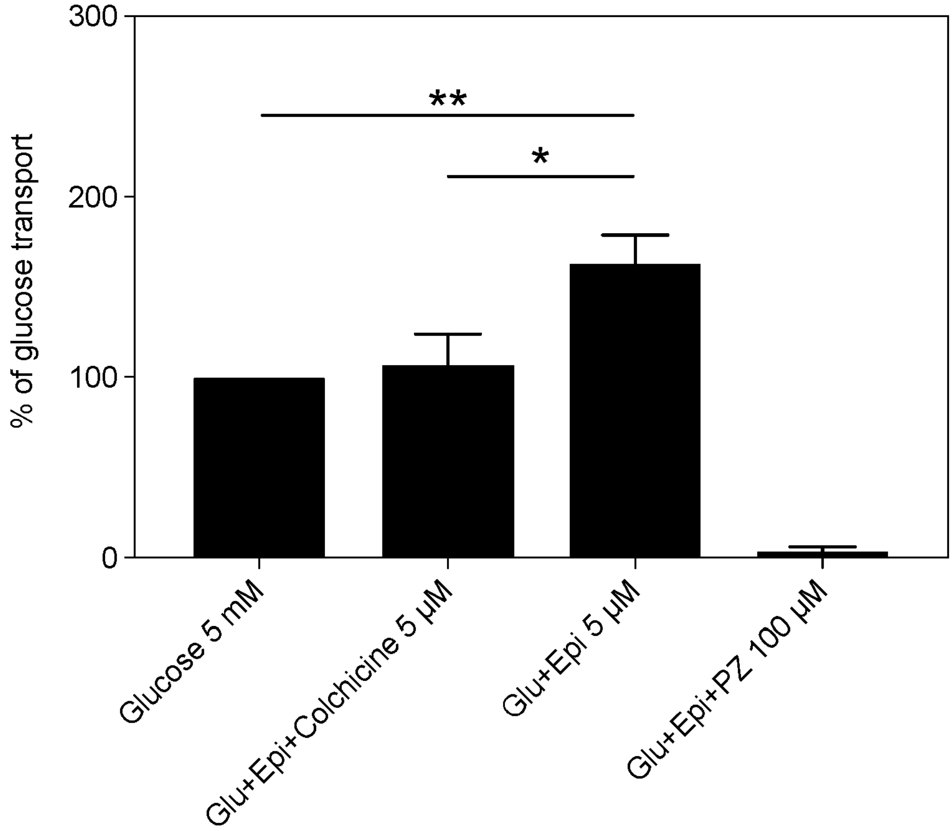
300
200
100
0
% of glucose transport
**
*
Glucose 5 mM
Glu+Epi+Colchicine 5 µM
Glu+Epi 5 µM
Glu+Epi+PZ 100 µM

Fig. 5D

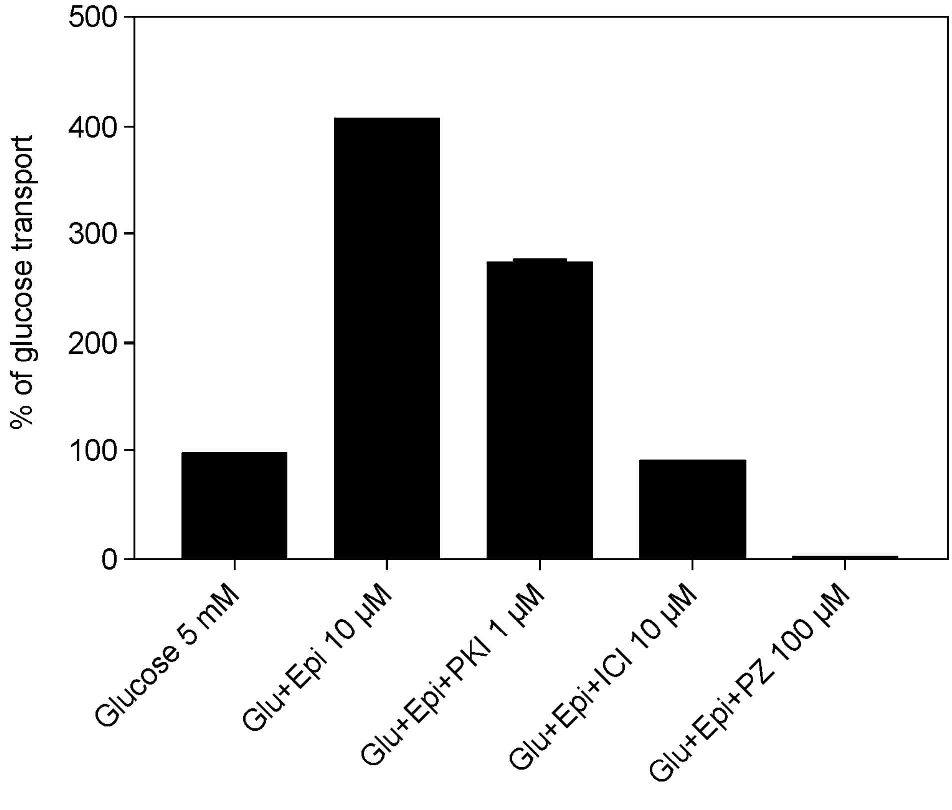
500
400
300
200
100
0
% of glucose transport
Glucose 5 mM
Glu+Epi 10 µM
Glu+Epi+PKI 1 µM
Glu+Epi+ICI 10 µM
Glu+Epi+PZ 100 µM

Fig. 5E

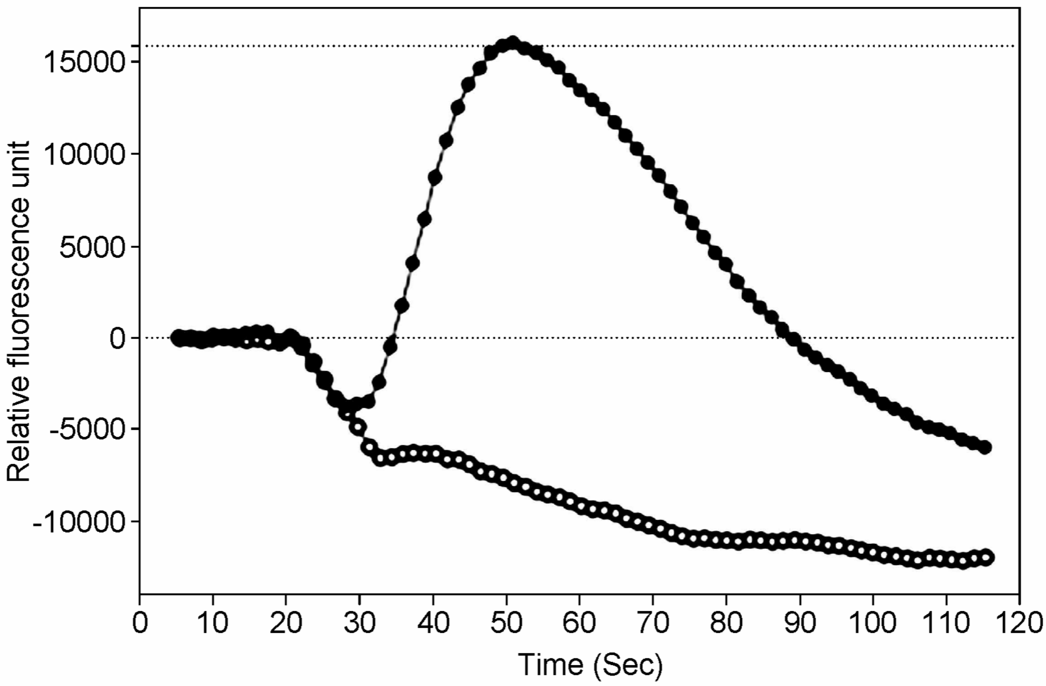
Relative fluorescence unit
15000
10000
5000
0
-5000
-10000
0
10
20
30
40
50
60
70
80
90
100
110
120
Time (Sec)

Fig. 5F

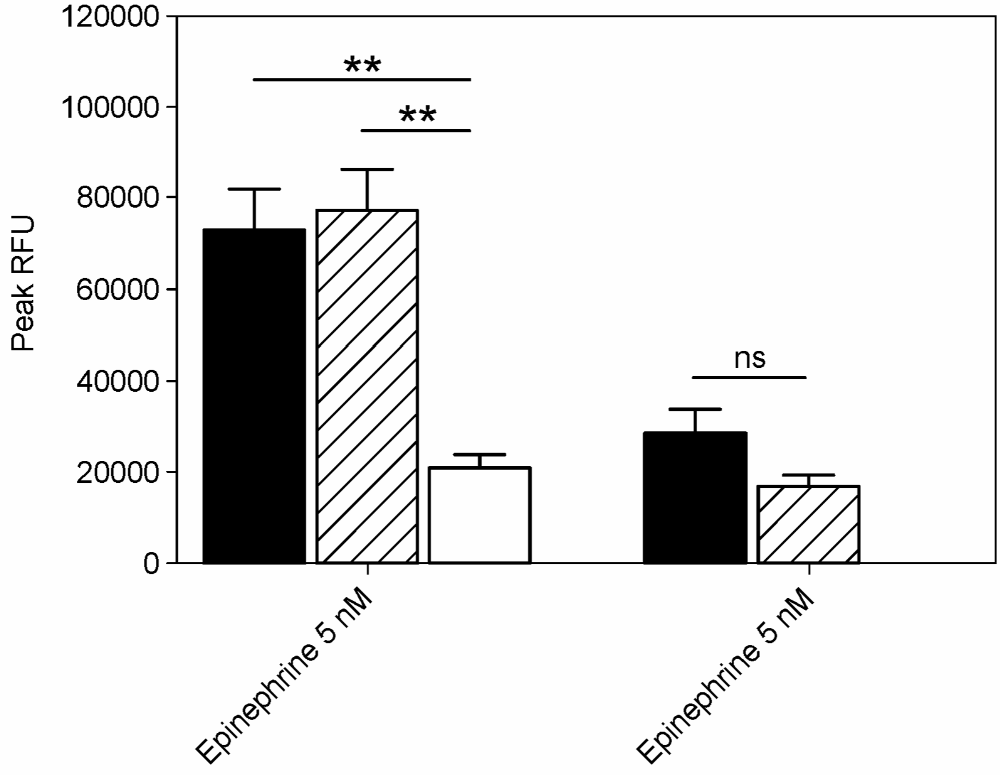
120000
100000
80000
60000
40000
20000
0
Peak RFU
**
**
ns
Epinephrine 5 nM
Epinephrine 5 nM

Fig. 5G

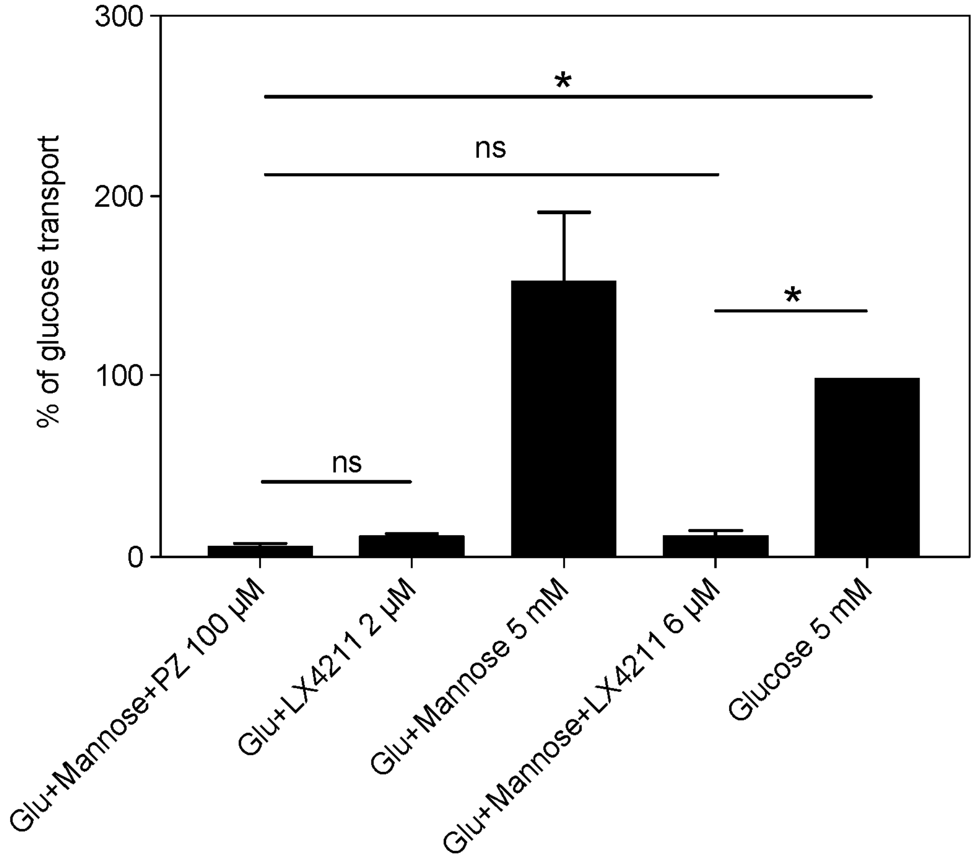
300
200
100
0
% of glucose transport
*
ns
*
ns
Glu+Mannose+PZ 100 µM
Glu+LX4211 2 µM
Glu+Mannose 5 mM
Glu+Mannose+LX4211 6 µM
Glucose 5 mM

Fig. 5H

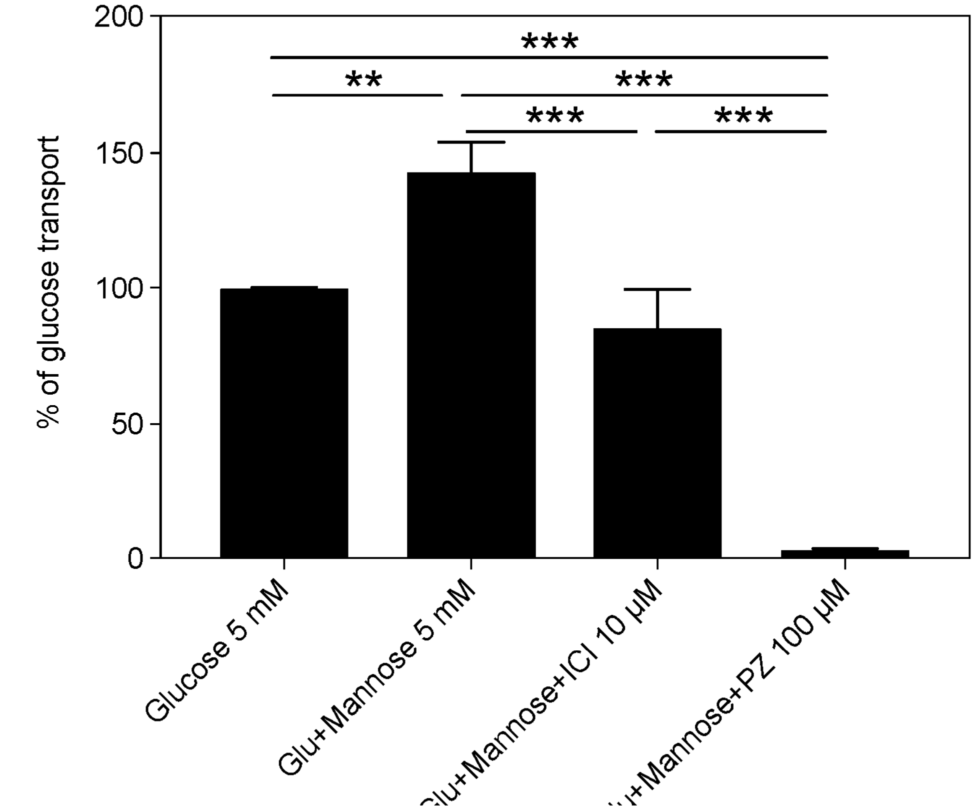
200
150
100
50
0
% of glucose transport
***
**
***
***
***
Glucose 5 mM
Glu+Mannose 5 mM
Glu+Mannose+ICI 10 µM
Glu+Mannose+PZ 100 µM

Fig. 6

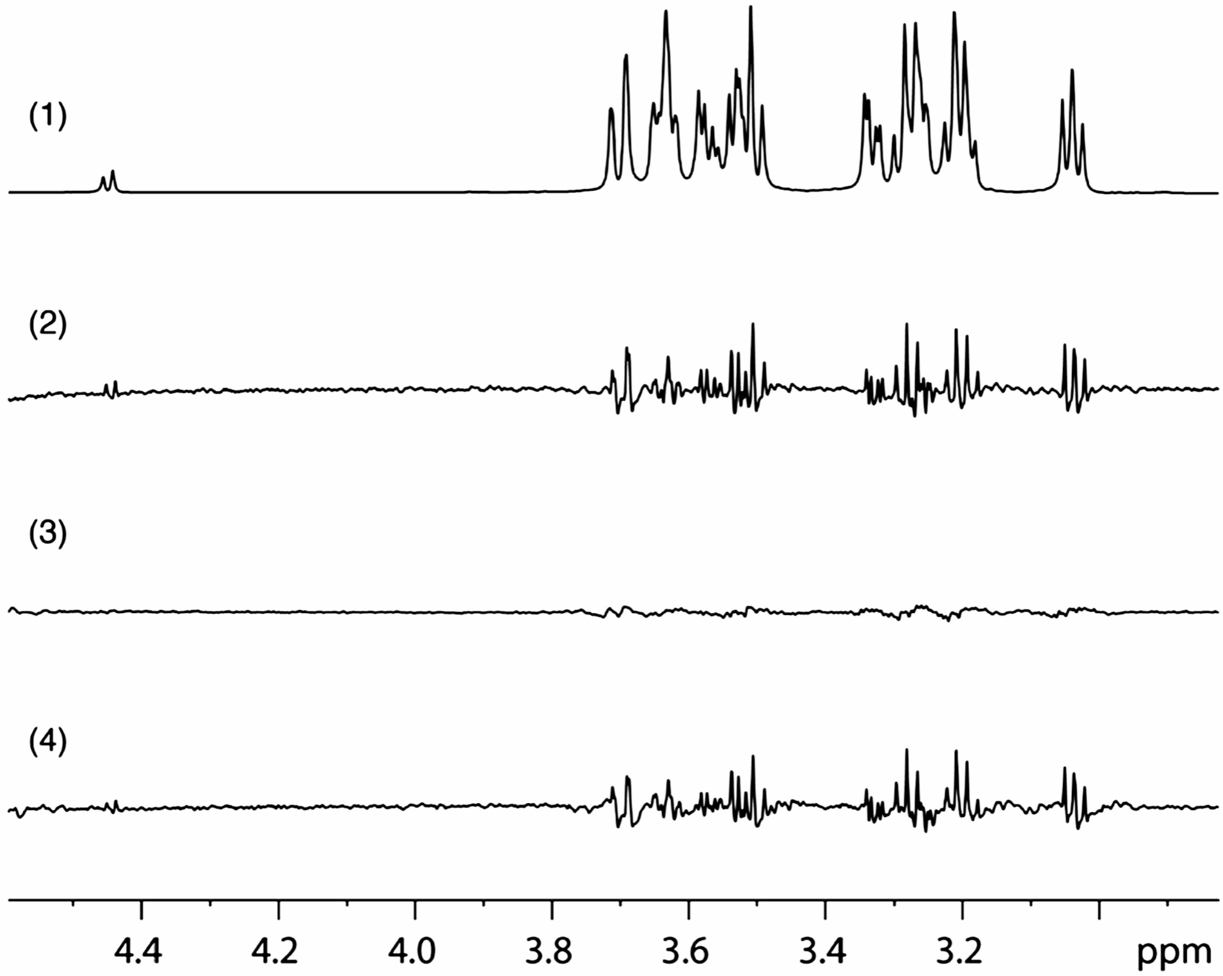

Fig. 7A

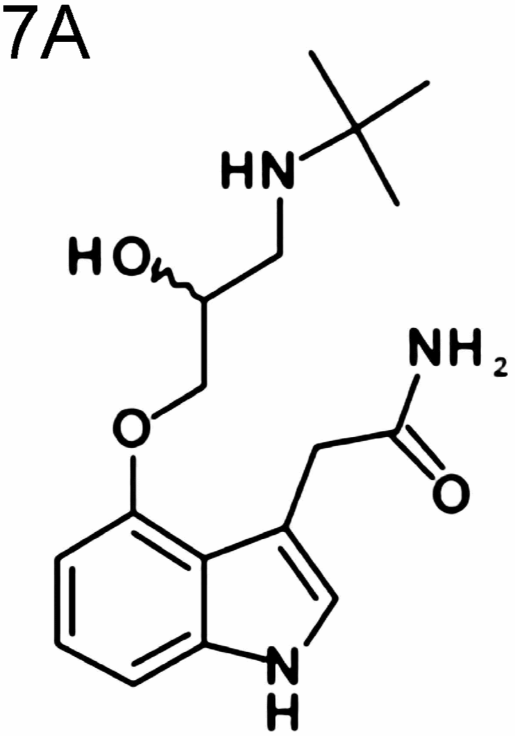

Fig. 7B

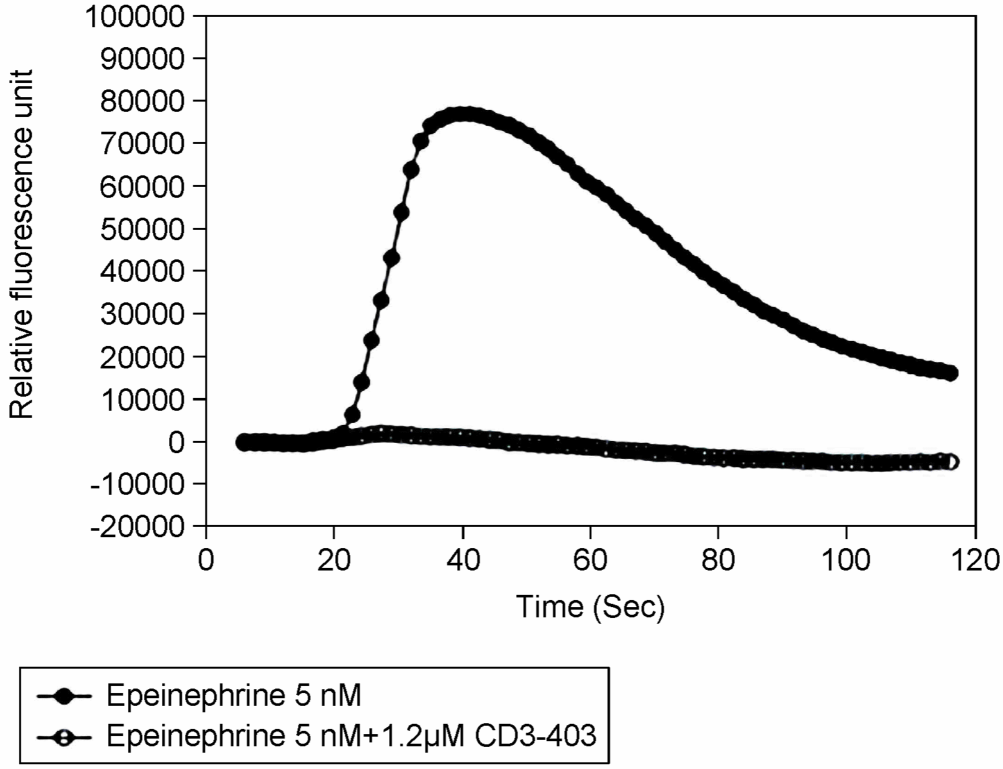
100000
90000
80000
70000
60000
50000
40000
30000
20000
10000
0
-10000
-20000
Relative fluorescence unit
0
20
40
60
80
100
120
Time (Sec)
Epeinephrine 5 nM
Epeinephrine 5 nM+1.2µM CD3-403

Fig. 7C

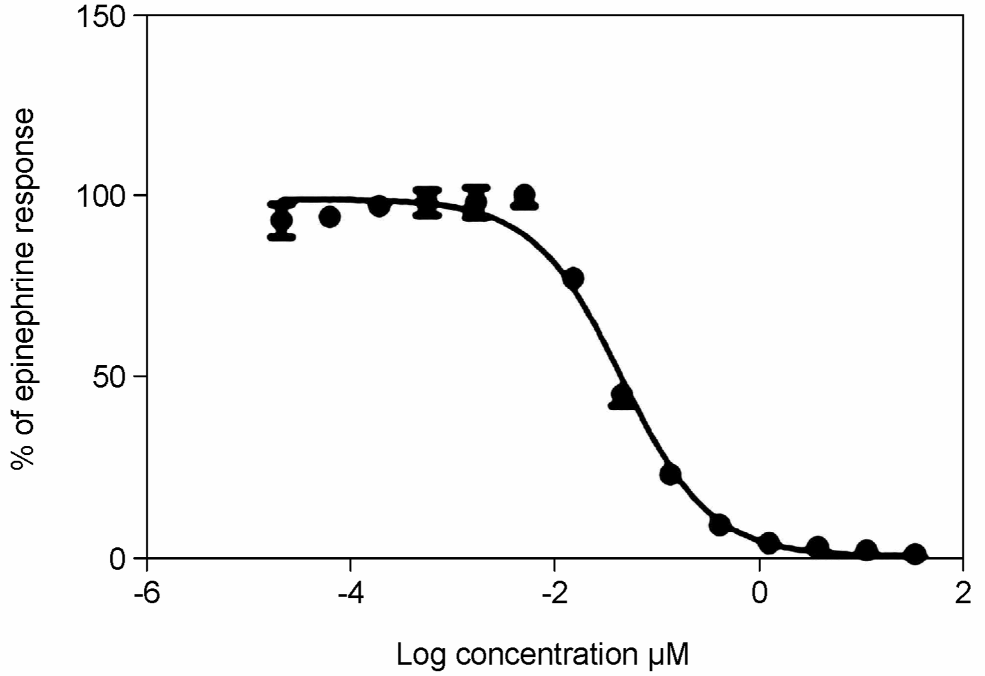
150
100
50
0
% of epinephrine response
-6
-4
-2
0
2
Log concentration μM

Fig. 7D

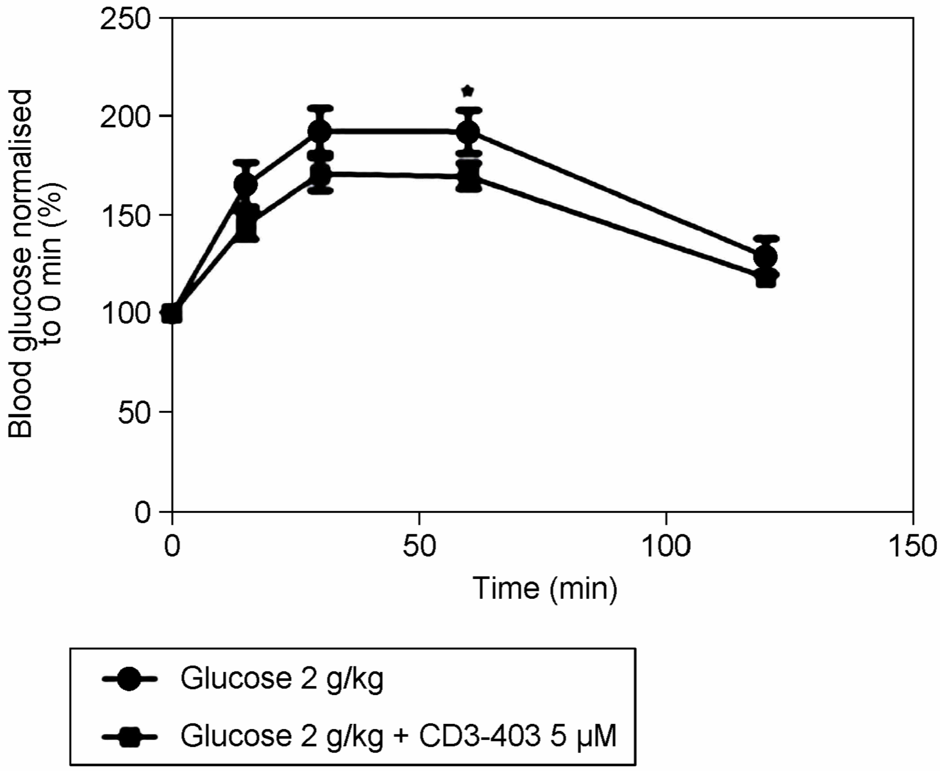
250
200
150
100
50
0
Blood glucose normalised to 0 min (%)
*
0
50
100
150
Time (min)
Glucose 2 g/kg
Glucose 2 g/kg + CD3-403 5 μM

Fig. 7E

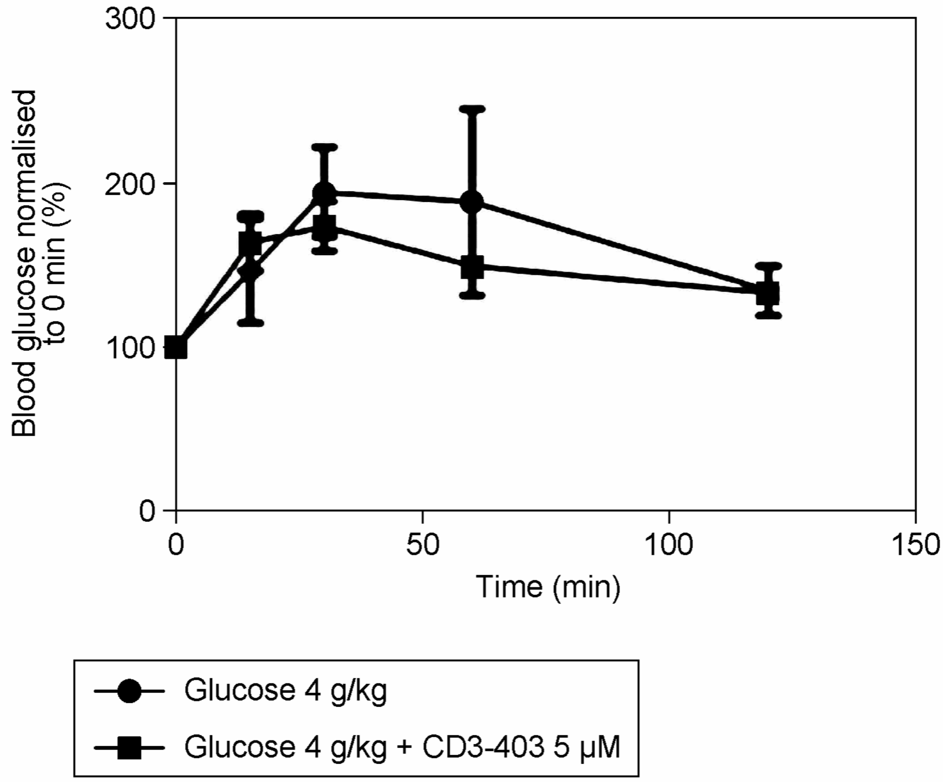
300
200
100
0
Blood glucose normalised to 0 min (%)
0
50
100
150
Time (min)
Glucose 4 g/kg
Glucose 4 g/kg + CD3-403 5 µM

Fig. 7F

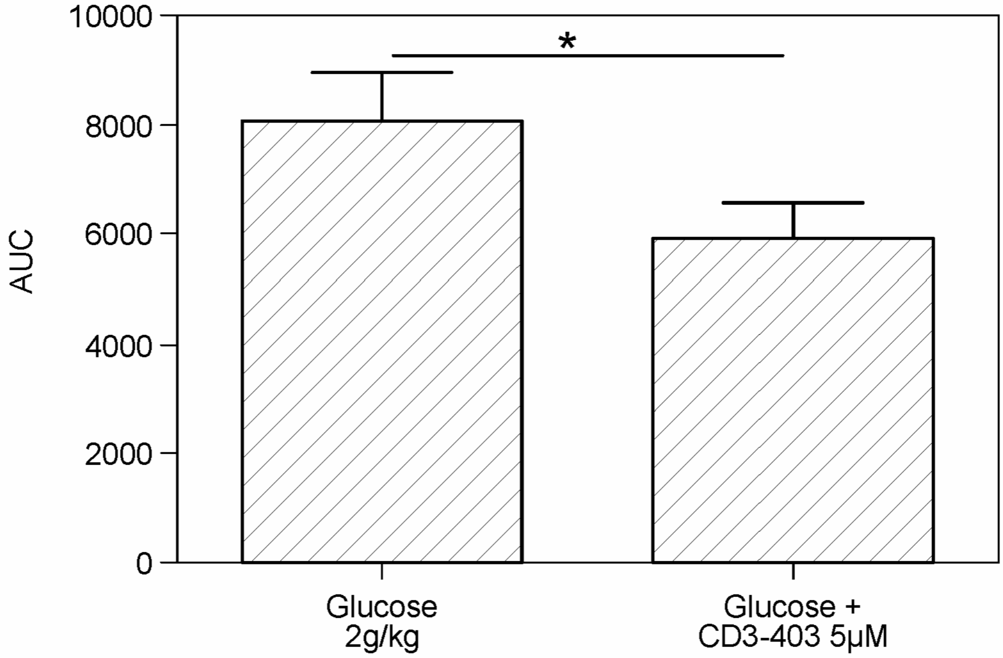
10000
8000
6000
4000
2000
0
AUC
*
Glucose 2g/kg
Glucose + CD3-403 5µM

Fig. 8

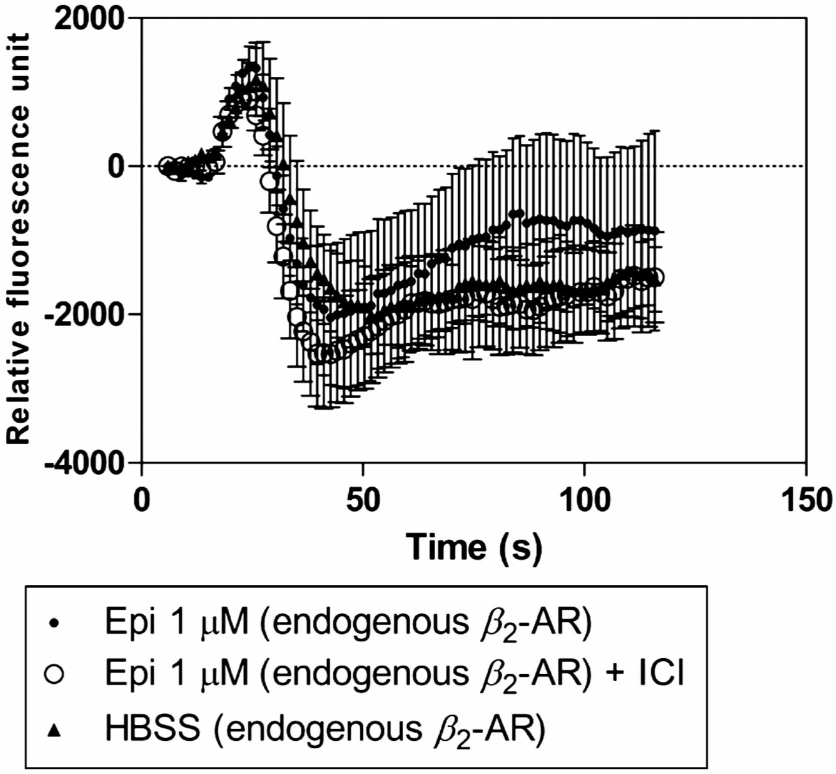
2000
0
-2000
-4000
Relative fluorescence unit
0
50
100
150
Time (s)
Epi 1 μM (endogenous β2-AR)
Epi 1 μM (endogenous β2-AR) + ICI
HBSS (endogenous β2-AR)

Fig. 9

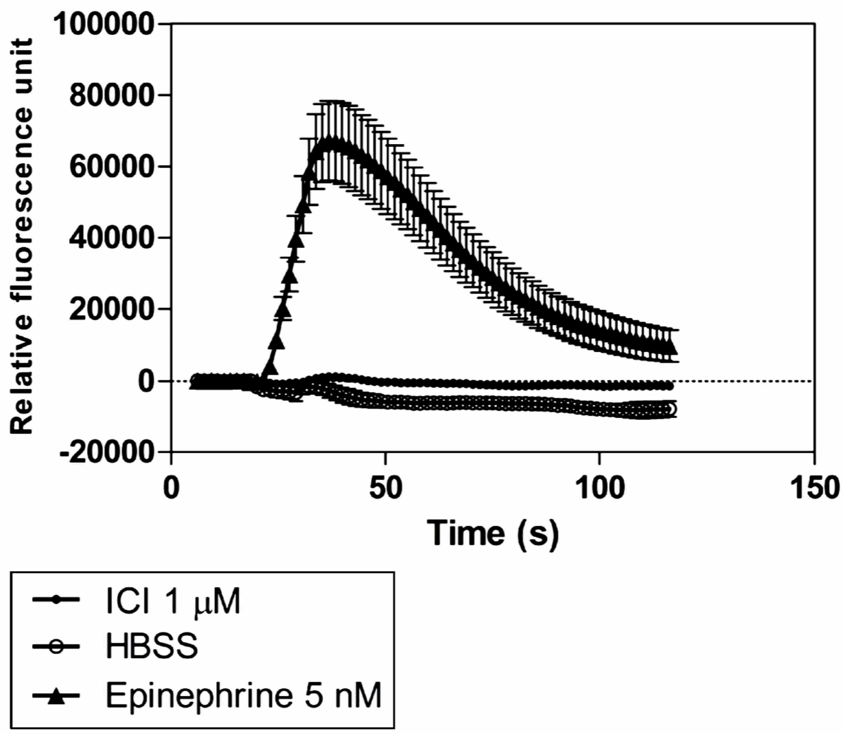
100000
80000
60000
40000
20000
0
-20000
Relative fluorescence unit
0
50
100
150
Time (s)
ICI 1 μM
HBSS
Epinephrine 5 nM

Fig. 10

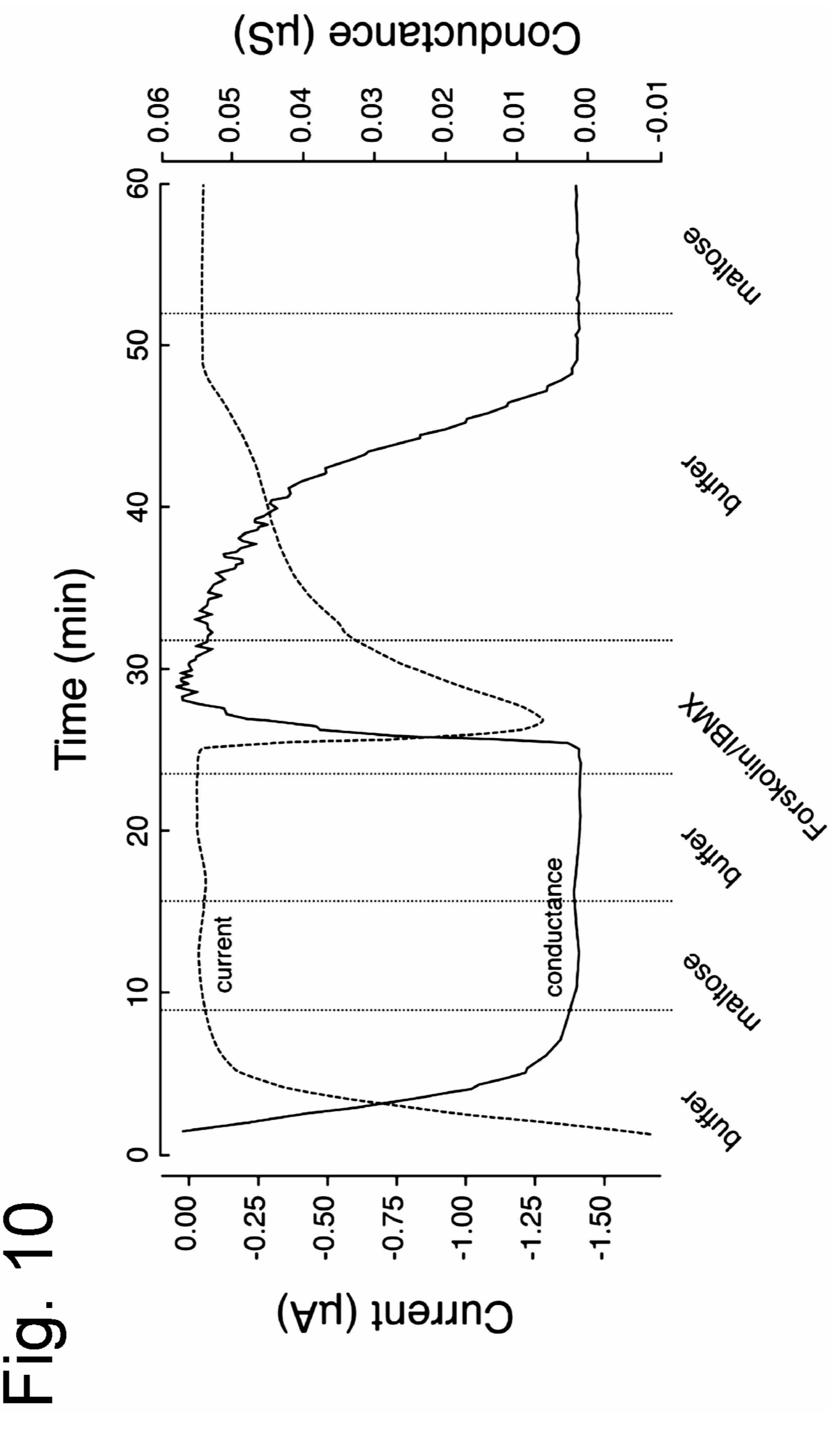
Time (min)
Current (µA)
Conductance (µS)
current
conductance
buffer
maltose
buffer
Forskolin/IBMX
buffer
maltose
0
10
20
30
40
50
60
0.00
-0.25
-0.50
-0.75
-1.00
-1.25
-1.50
0.06
0.05
0.04
0.03
0.02
0.01
0.00
-0.01

Fig. 11A

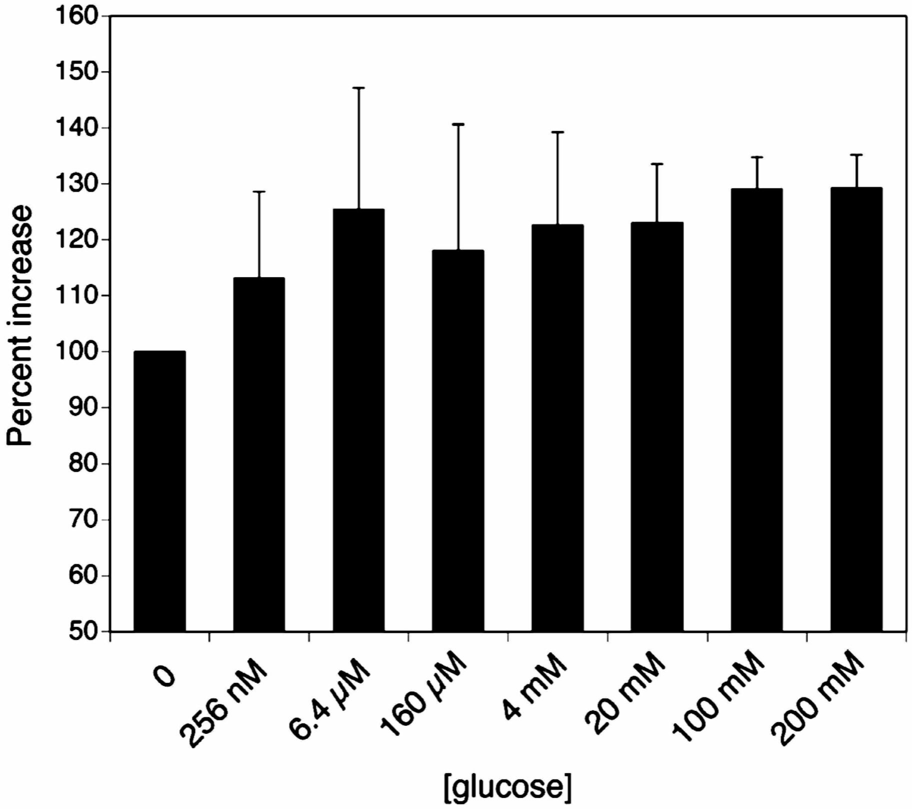


Fig. 11B

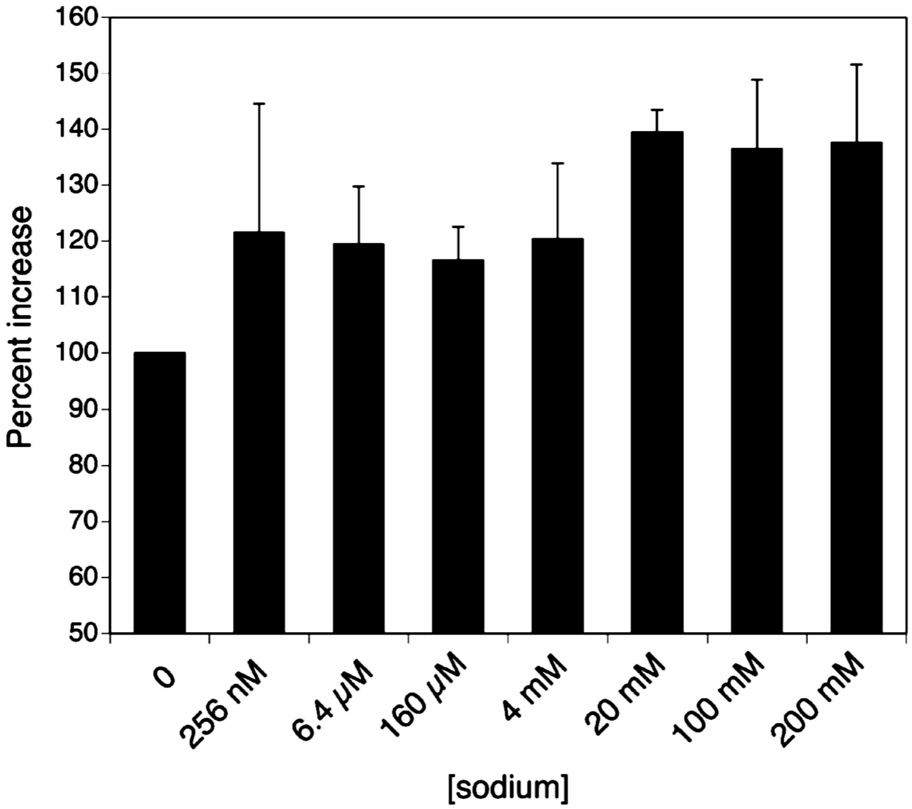

Fig. 12A

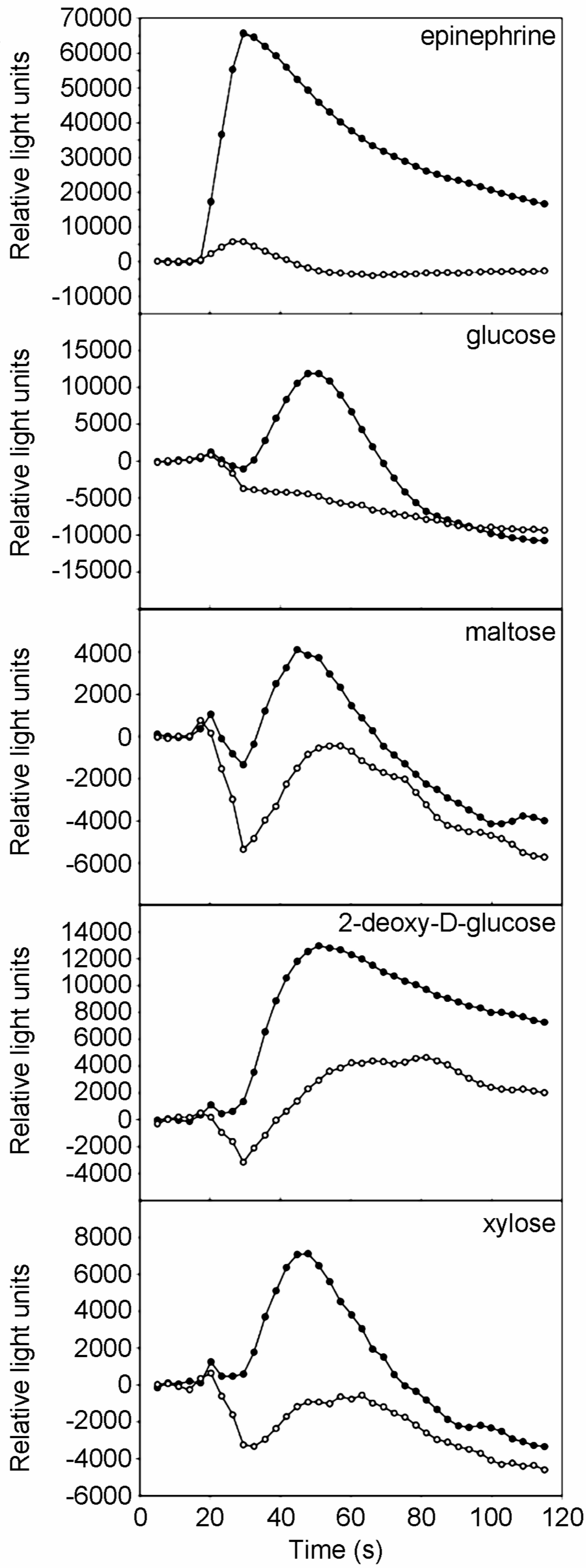
epinephrine
glucose
maltose
2-deoxy-D-glucose
xylose
Relative light units
70000
60000
50000
40000
30000
20000
10000
0
-10000
15000
10000
5000
0
-5000
-10000
-15000
4000
2000
0
-2000
-4000
-6000
14000
12000
10000
8000
6000
4000
2000
0
-2000
-4000
8000
6000
4000
2000
0
-2000
-4000
-6000
0
20
40
60
80
100
120
Time (s)

Fig. 12B

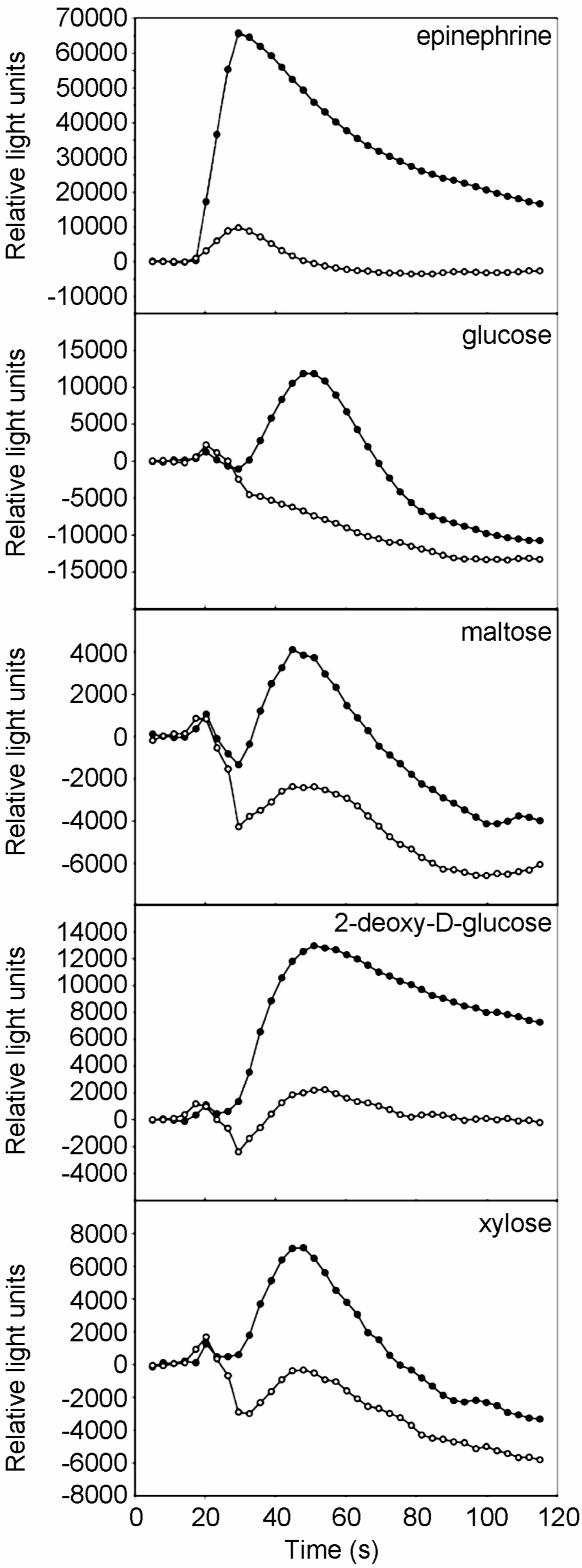
epinephrine
glucose
maltose
2-deoxy-D-glucose
xylose
Relative light units
70000
60000
50000
40000
30000
20000
10000
0
-10000
15000
10000
5000
-5000
-10000
-15000
4000
2000
-2000
-4000
-6000
14000
12000
10000
8000
6000
-8000
0
20
40
60
80
100
120
Time (s)

Fig. 12C

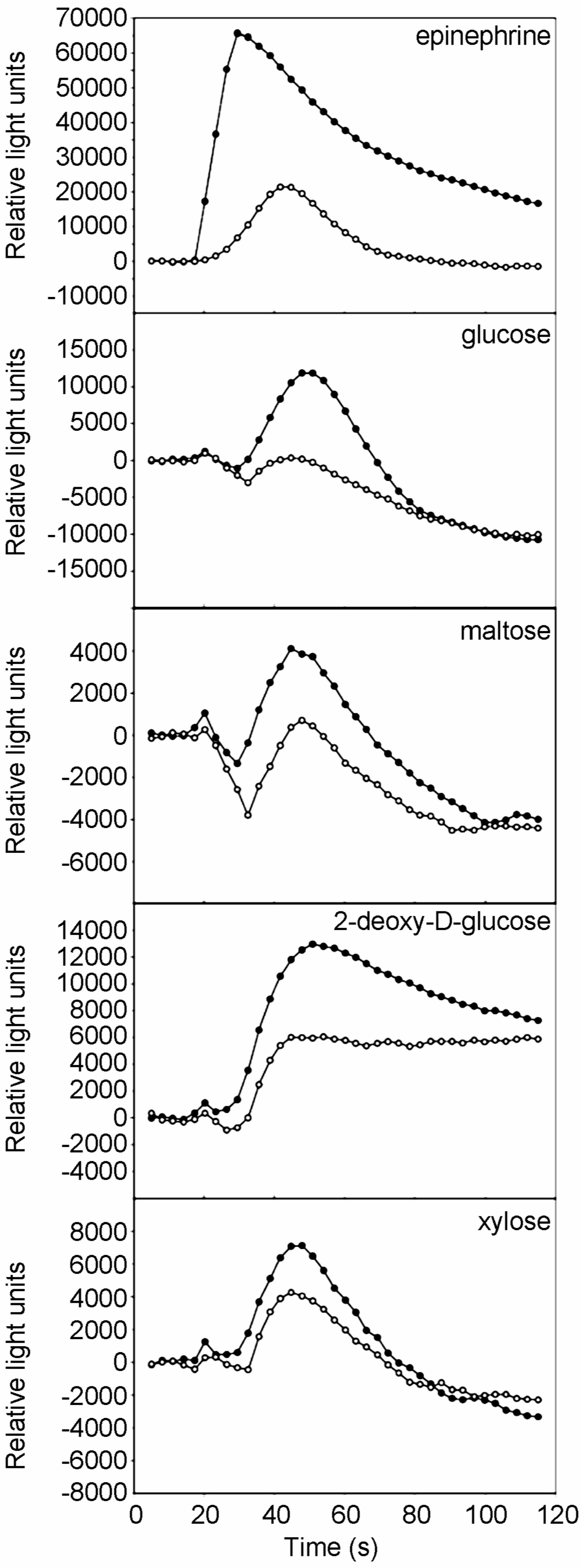
epinephrine
glucose
maltose
2-deoxy-D-glucose
xylose
Relative light units
70000
60000
50000
40000
30000
20000
10000
0
-10000
15000
10000
5000
-5000
-10000
-15000
4000
2000
-2000
-4000
-6000
14000
12000
8000
6000
-8000
0 20 40 60 80 100 120
Time (s)

Fig. 13

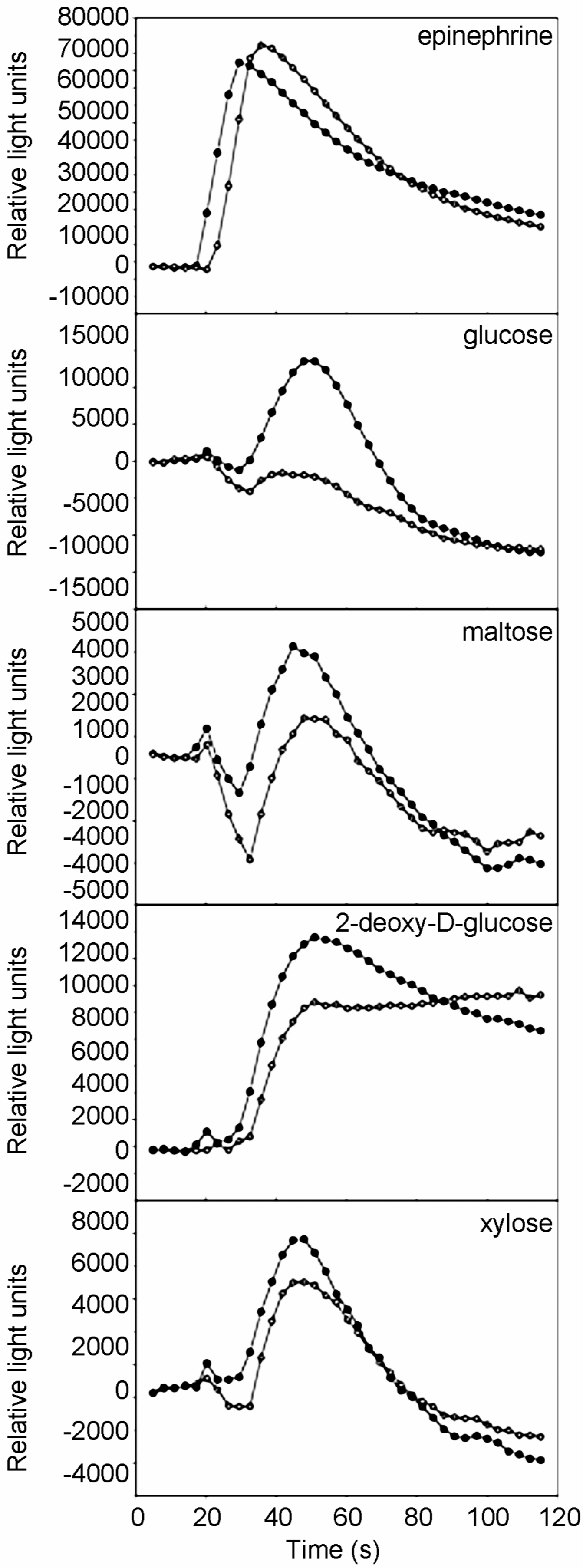
epinephrine
glucose
maltose
2-deoxy-D-glucose
xylose
Relative light units
80000
70000
60000
50000
40000
30000
20000
10000
0
-10000
15000
10000
5000
0
-5000
-10000
-15000
5000
4000
3000
2000
1000
0
-1000
-2000
-4000
-4000
-5000
14000
12000
10000
8000
6000
4000
2000
0
-2000
8000
6000
4000
2000
0
-2000
-4000
0
20
40
60
80
100
120
Time (s)

Fig. 14A

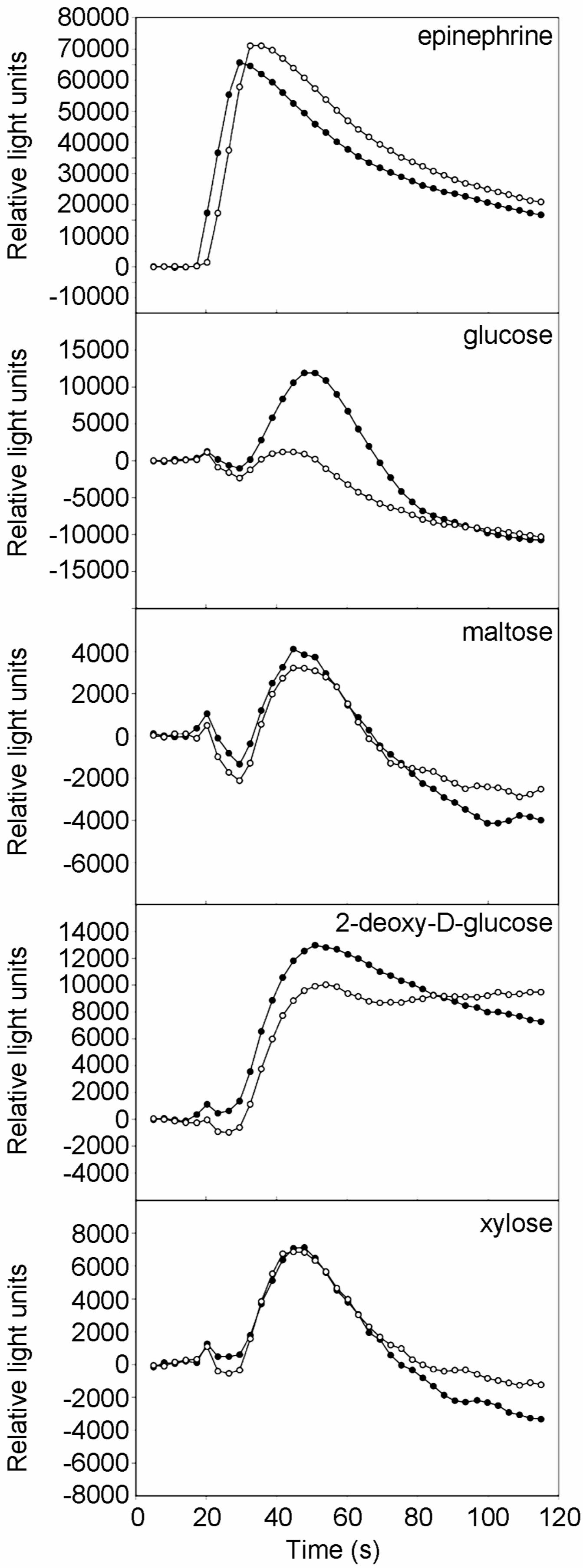
epinephrine
glucose
maltose
2-deoxy-D-glucose
xylose
Relative light units
80000
70000
60000
50000
40000
30000
20000
10000
0
-10000
15000
10000
5000
0
-5000
-10000
-15000
4000
2000
0
-2000
-4000
-6000
14000
12000
10000
8000
6000
4000
2000
0
-2000
-4000
8000
6000
4000
2000
0
-2000
-4000
-6000
-8000
0
20
40
60
80
100
120
Time (s)

Fig. 14B

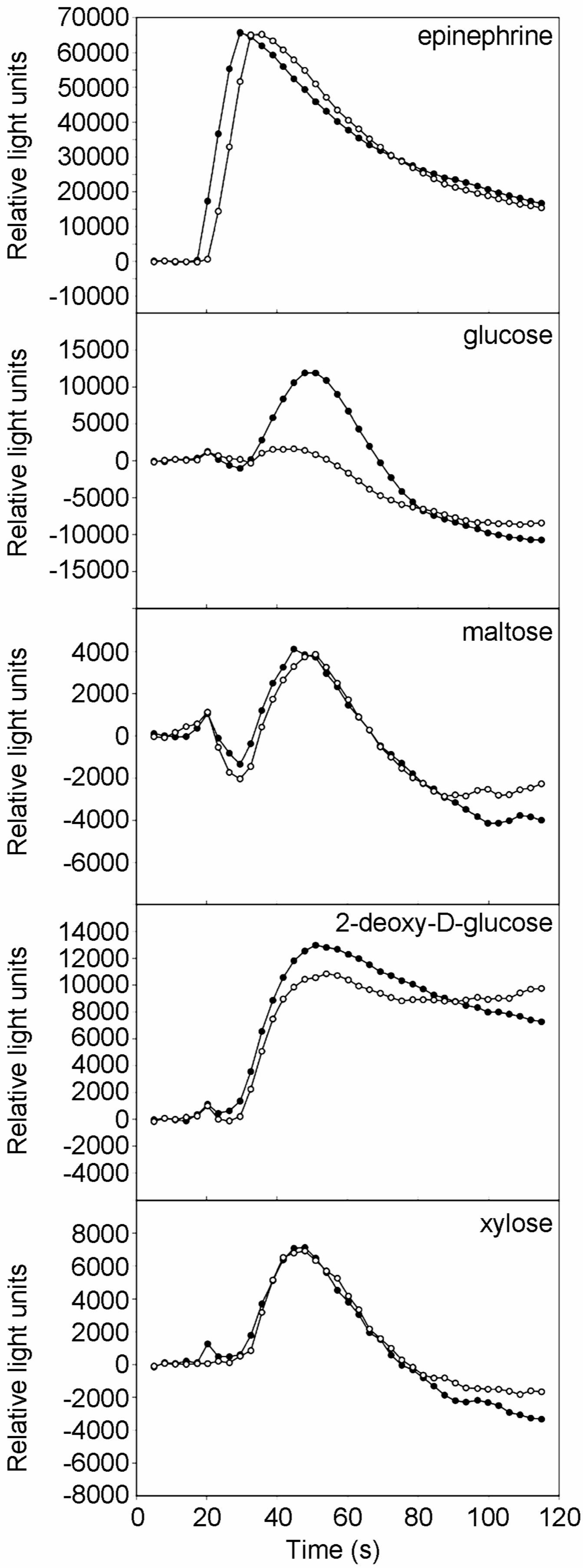
epinephrine
glucose
maltose
2-deoxy-D-glucose
xylose
Relative light units
Time (s)
70000
60000
50000
40000
30000
20000
10000
0
-10000
15000
10000
5000
0
-5000
-10000
-15000
4000
2000
0
-2000
-4000
-6000
14000
12000
10000
8000
6000
4000
2000
0
-2000
-4000
8000
6000
4000
2000
0
-2000
-4000
-6000
-8000
0
20
40
60
80
100
120

Fig. 15

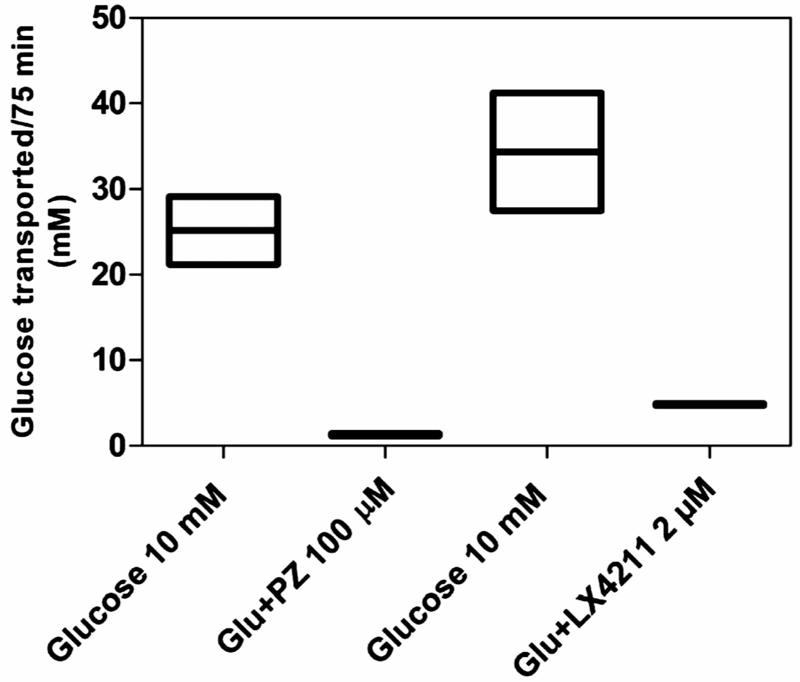
Glucose transported/75 min (mM)
50
40
30
20
10
0
Glucose 10 mM
Glu+PZ 100 µM
Glucose 10 mM
Glu+LX4211 2 µM

Fig. 16

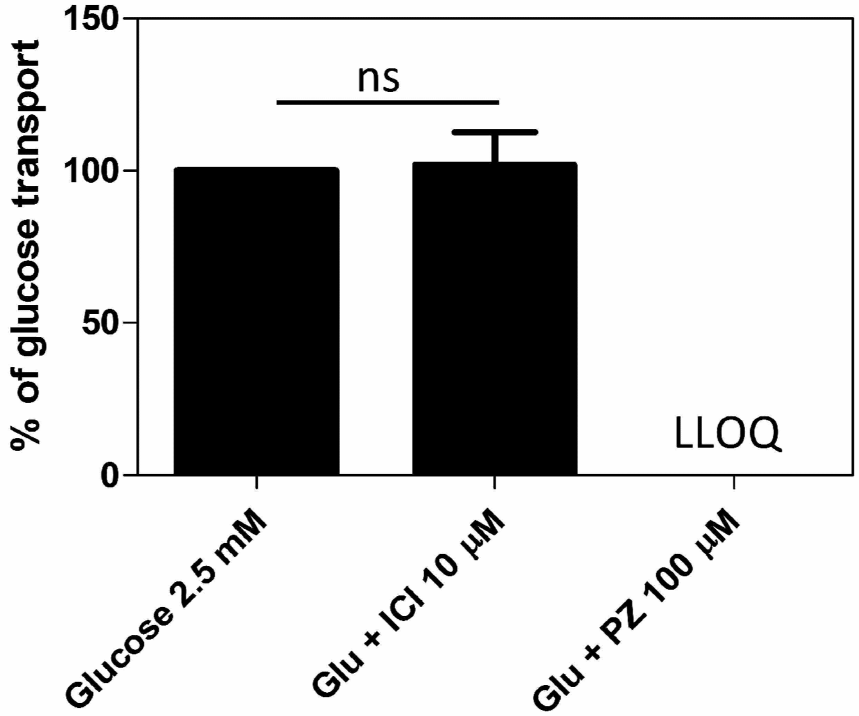
% of glucose transport
150
100
50
0
ns
LLOQ
Glucose 2.5 mM
Glu + ICI 10 µM
Glu + PZ 100 µM

Fig. 17

| | PCR | | | Micro-Array | | qPCR | |
|---|---|---|---|---|---|---|---|
| | mouse | STC-1 | | STC-1 | | STC-1 | |
| | | 5 mM Glu | 25 mM Glu | 5 mM Glu | 25 mM Glu | 5 mM Glu | 25 mM Glu |
| 5-HT1b | | | | + | + | | |
| 5-HT3a | | | | + | + | | |
| ADRB2 | + | + | | / | / | | |
| AGTRL1 | + | | + | - | - | + | + |
| ATIIR | | | | - | + | | |
| celsr3 | | | | + | + | | |
| GPR1 | + | - | | + | + | | + |
| GPR120 | + | - | | + | + | | + |
| GPR125 | + | + | | / | / | | + |
| GPR151 | + | | + | / | / | + | + |
| GPR18 | - | - | | + | + | | + |
| GPR19 | + | + | | + | + | | + |
| GPR22 | - | - | | / | / | | + |
| GPR35 | + | | + | / | / | + | + |
| GPR39 | + | - | | + | + | | + |
| GPR40 | + | + | | / | / | | + |
| GPR43 | + | + | | + | + | | |
| GPR48 | + | + | | + | + | | + |
| GPR49 | - | + | | + | + | | + |
| GPR55 | - | | - | / | / | | |
| GPR56 | + | + | | + | + | | + |
| GPR61 | + | - | | + | + | | + |
| GPR73 | - | | - | - | - | | |
| GPR80 | + | - | | / | / | | + |
| GPR85 | + | + | | + | + | | + |
| GPR88 | - | - | | + | + | | + |
| GPRC5C | + | | + | + | + | + | + |
| PGR22 | + | | + | / | / | | |
| prosta E4 | | | | + | + | | |
| RDC1 | + | | + | - | - | + | + |
| TEM5 | - | | + | - | - | + | + |
| TM7SF1 | + | - | | + | + | | + |
| TM7SF2 | + | + | | + | + | | + |
| TM7SF3 | + | + | | + | + | | + |
| TPRA40 | + | | + | + | + | + | + |

Fig. 18

| Compound | Test Concentration | A-B permeability ($10^{-6}$ cm/s) | Mean Recovery (%) | B-A permeability ($10^{-6}$ cm/s) | Mean Recovery (%) | Efflux |
|---|---|---|---|---|---|---|
| CD3-403 | 1.0 µM | 0.4 | 83 | 1.3 | 94 | 3.25 |

Fig. 19

| |
|---|
| GPR1 fw TGGACCCCTTATCACCTGTTTAG |
| GPR1 re CCTGCAGCACATTCTGGAAA |
| GPR120 fw TCCGAGTGTCCCAACAAGACTA |
| GPR120 re ATGATGGGACTCCACATGATG |
| GPR125 fw CGTGCAGTTTCGAACAAACG |
| GPR125 re CTGGCCCGGTGTCCTTT |
| GPR18 fw GGAGAAGTCCATACGGATCATCA |
| GPR18 re GTGGAAGGGCACGAAGCA |
| GPR19 fw CTACCGCAGCAATGCCTACA |
| GPR19 re GATTTCCGAAATGCCCACAT |
| GPR22 fw TCGTGTTTGGTGTGAGAACTTCA |
| GPR22 re TCCCGGTGGCGTTTCA |
| GPR39 fw CCCATGGAGTTCTACAGCATCAT |
| GPR39 re CGTGTGGAGCTTACAGGACAGA |
| GPR40 fw TGCCTCCAATGTGGCTAGTTT |
| GPR40 re CCCTGTGATGAGTCCCAACTTC |
| GPR48 fw AAGACGACTGGAAGCTCCTGAA |
| GPR48 re CCGCCTTGGCTGCTGAT |
| GPR49 fw TGAGATCAAAACACGCGAGTCT |
| GPR49 re TGCTGGCGTGGGTAAAGG |
| GPR56 fw CCAAGGCTTCCTCATCTTCCT |
| GPR56 re GCGCTGTCTGAGTTGTTCTTCA |
| GPR61 fw AACCTGGATTGGCTACTTTTGC |
| GPR61 re TAAGCTCGCCCCGGATCT |
| GPR80 fw CCTTTGGCAACCTGCTGTTATAT |
| GPR80 re CCGCTGGCTTTGCATCTC |
| GPR85 fw TTGCACGAGGGCCTGTAGTAC |
| GPR85 re ATTCCTGCTTGGGCGAAACT |
| GPR88 fw GCTCTACACGTGGAGGAACGA |
| GPR88 re GTTGCGCCTGGGACATG |
| TM7SF1 fw CCCCCGAAGATATGACAGTGAT |
| TM7SF1 re GGAGCAAAACTTCCCTGAAGTC |
| TM7SF2 fw GCAGCAGTGCCTCCAAAAGT |
| TM7SF2 re GGTAAGGCACACGCTTGCA |
| TM7SF3 fw GAAAGAGGACAGCCGCCTTT |
| TM7SF3 re GATGTTGGTCACTCTGCGTTCTC |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **MORTEN HOSTRUP et al.** *J. Physiol.*, 2021, vol. 600 (5), 1209
- **SIN YEE TAN et al.** *Diabetes Metab. Syndr.*, 2018, vol. 13 (1), 364
- **DAVID M. WILLIAMS et al.** *Diabetes Ther.*, 2020, vol. 11 (6), 1199
- **DOCKRAY GJ**. Luminal sensing in the gut: an overview. *J Physiol Pharmacol*, 2003, vol. 54 (4), 9-17
- **FERRARIS RP**. Dietary and developmental regulation of intestinal sugar transport. *Biochem J.*, 2001, vol. 360 (2), 265-76
- **DIAMOND JM** ; **KARASOV WH**. Adaptive regulation of intestinal nutrient transporters. *Proc Natl Acad Sci U S A.*, 1987, vol. 84 (8), 2242-5
- **DYER J** ; **VAYRO S** ; **KING TP** ; **SHIRAZI-BEECHEY SP**. Glucose sensing in the intestinal epithelium. *Eur J Biochem.*, 2003, vol. 270 (16), 3377-88
- **SHIRAZI-BEECHEY SP** ; **MORAN AW** ; **BATCHELOR DJ** ; **DALY K** ; **AL-RAMMAHI M**. Glucose sensing and signalling; regulation of intestinal glucose transport. *Proc Nutr Soc.*, 2011, vol. 70 (2), 185-93
- **SHARP PA** ; **DEBNAM ES** ; **SRAI SK**. Rapid enhancement of brush border glucose uptake after exposure of rat jejunal mucosa to. *Gut*, 1996, vol. 39 (4), 545-50
- **MIYAMOTO K** ; **HASE K** ; **TAKAGI T** ; **FUJII T** ; **TAKETANI Y** ; **MINAMI H et al.** Differential responses of intestinal glucose transporter mRNA transcripts to levels of dietary sugars. *Biochem J.*, 1993, vol. 295 (1), 211-5
- **SHARP PA** ; **DEBNAM ES**. The role of cyclic AMP in the control of sugar transport across the brush-border and basolateral membranes of rat jejunal enterocytes. *Exp Physiol.*, 1994, vol. 79 (2), 203-14
- **DYER J** ; **SALMON KS** ; **ZIBRIK L** ; **SHIRAZI-BEECHEY SP**. Expression of sweet taste receptors of the T1R family in the intestinal tract and enteroendocrine cells. *Biochem Soc Trans*, 2005, vol. 33 (1), 302-5
- **MARGOLSKEE RF** ; **DYER J** ; **KOKRASHVILI Z** ; **SALMON KS** ; **ILEGEMS E** ; **DALY K et al.** T1R3 and gustducin in gut sense sugars to regulate expression of Na+-glucose cotransporter 1. *Proc Natl Acad Sci U S A.*, 2007, vol. 104 (38), 15075-80
- **BUDDINGTON RK** ; **CHEN JW** ; **DIAMOND JM**. Dietary regulation of intestinal brush-border sugar and amino acid transport in carnivores. *Am J Physiol.*, 1991, vol. 261 (4), R793-801
- **BATCHELOR DJ** ; **AL-RAMMAHI M** ; **MORAN AW** ; **BRAND JG** ; **LI X** ; **HASKINS M et al.** Sodium/glucose cotransporter-1, sweet receptor, and disaccharidase expression in the intestine of the domestic dog and cat: two species of different dietary habit. *Am J Physiol Regul Integr Comp Physiol.*, 2011, vol. 300 (1), R67-75
- **STERNINI C** ; **ANSELMI L** ; **ROZENGURT E**. Enteroendocrine cells: a site of 'taste' in gastrointestinal chemosensing. *Curr Opin Endocrinol Diabetes Obes*, 2008, vol. 15 (1), 73-8
- **STEINERT RE** ; **BEGLINGER C**. Nutrient sensing in the gut: interactions between chemosensory cells, visceral afferents and the secretion of satiation peptides. *Physiol Behav.*, 2011, vol. 105 (1), 62-70
- **GLENDINNING JI**. Oral Post-Oral Actions of Low-Calorie Sweeteners: A Tale of Contradictions and Controversies. *Obesity (Silver Spring)*, 2018, vol. 26 (3), S9-S17
- **EZCURRA M** ; **REIMANN F** ; **GRIBBLE FM** ; **EMERY E**. Molecular mechanisms of incretin hormone secretion. *Curr Opin Pharmacol.*, 2013, vol. 13 (6), 922-7
- **KRAAKMAN L** ; **LEMAIRE K** ; **MA P** ; **TEUNISSEN AW** ; **DONATON MC** ; **VAN DIJCK P et al.** A Saccharomyces cerevisiae G-protein coupled receptor, Gpr1, is specifically required for glucose activation of the cAMP pathway during the transition to growth on glucose. *Mol Microbiol.*, 1999, vol. 32 (5), 1002-12
- **LEMAIRE K** ; **VAN DE VELDE S** ; **VAN DIJCK P** ; **THEVELEIN JM**. Glucose and sucrose act as agonist and mannose as antagonist ligands of the G protein-coupled receptor Gpr1 in the yeast Saccharomyces cerevisiae. *Mol Cell*, 2004, vol. 16 (2), 293-9
- **WELTON RM** ; **HOFFMAN CS**. Glucose monitoring in fission yeast via the Gpa2 galpha, the git5 Gbeta and the git3 putative glucose receptor. *Genetics*, 2000, vol. 156 (2), 513-21

- **MAIDAN MM** ; **DE ROP L** ; **SERNEELS J** ; **EXLER S** ; **RUPP S** ; **TOURNU H et al.** The G protein-coupled receptor Gpr1 and the Galpha protein Gpa2 act through the cAMP-protein kinase A pathway to induce morphogenesis in Candida albicans. *Mol Biol Cell*, 2005, vol. 16 (4), 1971-86
- **MIWA T** ; **TAKAGI Y** ; **SHINOZAKI M** ; **YUN CW** ; **SCHELL WA** ; **PERFECT JR et al.** Gpr1, a putative G-protein-coupled receptor, regulates morphogenesis and hypha formation in the pathogenic fungus Candida albicans. *Eukaryot Cell*, 2004, vol. 3 (4), 919-31
- **LI L** ; **BORKOVICH KA**. GPR-4 is a predicted G-protein-coupled receptor required for carbon source-dependent asexual growth and development in Neurospora crassa. *Eukaryot Cell*, 2006, vol. 5 (8), 1287-300
- **XUE C** ; **HSUEH YP** ; **HEITMAN J**. Magnificent seven: roles of G protein-coupled receptors in extracellular sensing in fungi. *FEMS Microbiol Rev*, 2008, vol. 32 (6), 1010-32
- **RUI L**. Energy metabolism in the liver. *Compr Physiol.*, 2014, vol. 4 (1), 177-97
- **ARNER P**. Human fat cell lipolysis: biochemistry, regulation and clinical role. *Best Pract Res Clin Endocrinol Metab.*, 2005, vol. 19 (4), 471-82
- **SHUKLA AK** ; **SUN JP** ; **LEFKOWITZ RJ**. Crystallizing thinking about the beta2-adrenergic receptor. *Mol Pharmacol.*, 2008, vol. 73 (5), 1333-8
- **FRISHMAN WH**. beta-Adrenergic blockers: a 50-year historical perspective. *Am J Ther.*, 2008, vol. 15 (6), 565-76
- **CHEREZOV V** ; **ROSENBAUM DM** ; **HANSON MA** ; **RASMUSSEN SG** ; **THIAN FS** ; **KOBILKA TS et al.** High-resolution crystal structure of an engineered human beta2-adrenergic G protein-coupled receptor. *Science*, 2007, vol. 318 (5854), 1258-65
- **RASMUSSEN SG** ; **CHOI HJ** ; **ROSENBAUM DM** ; **KOBILKA TS** ; **THIAN FS** ; **EDWARDS PC et al.** Crystal structure of the human beta2 adrenergic G-protein-coupled receptor. *Nature*, 2007, vol. 450 (7168), 383-7
- **ROSENBAUM DM** ; **CHEREZOV V** ; **HANSON MA** ; **RASMUSSEN SG** ; **THIAN FS** ; **KOBILKA TS et al.** GPCR engineering yields high-resolution structural insights into beta2-adrenergic receptor function. *Science*, 2007, vol. 318 (5854), 1266-73
- **RASMUSSEN SG** ; **CHOI HJ** ; **FUNG JJ** ; **PARDON E** ; **CASAROSA P** ; **CHAE PS et al.** Structure of a nanobody-stabilized active state of the beta(2) adrenoceptor. *Nature*, 2011, vol. 469 (7329), 175-80
- **RASMUSSEN SG** ; **DEVREE BT** ; **ZOU Y** ; **KRUSE AC** ; **CHUNG KY** ; **KOBILKA TS et al.** Crystal structure of the beta2 adrenergic receptor-Gs protein complex. *Nature*, 2011, vol. 477 (7366), 549-55
- **STROSBERG AD**. Structure, function, and regulation of adrenergic receptors. *Protein Sci.*, 1993, vol. 2 (8), 1198-209
- **BAKER JG**. The selectivity of beta-adrenoceptor antagonists at the human beta1, beta2 and beta3 adrenoceptors. *Br J Pharmacol.*, 2005, vol. 144 (3), 317-22
- **SMITH C** ; **TEITLER M**. Beta-blocker selectivity at cloned human beta 1- and beta 2-adrenergic receptors. *Cardiovasc Drugs Ther.*, 1999, vol. 13 (2), 123-6
- **TYNDALL JD** ; **SANDILYA R**. GPCR agonists and antagonists in the clinic. *Med Chem.*, 2005, vol. 1 (4), 405-21
- **VASSILATIS DK** ; **HOHMANN JG** ; **ZENG H** ; **LI F** ; **RANCHALIS JE** ; **MORTRUD MT et al.** The G protein-coupled receptor repertoires of human and mouse. *Proc Natl Acad Sci U S A.*, 2003, vol. 100 (8), 4903-8
- **SINGH AK** ; **RIEDERER B** ; **KRABBENHOFT A** ; **RAUSCH B** ; **BONHAGEN J** ; **LEHMANN U et al.** Differential roles of NHERF1, NHERF2, and PDZK1 in regulating CFTR-mediated intestinal anion secretion in mice. *J Clin Invest.*, 2009, vol. 119 (3), 540-50
- **ZHANG J** ; **HALM ST** ; **HALM DR**. Adrenergic activation of electrogenic K+ secretion in guinea pig distal colonic epithelium: involvement of beta1- and beta2-adrenergic receptors. *Am J Physiol Gastrointest Liver Physiol*, 2009, vol. 297 (2), G269-77
- **OLSEN WA** ; **INGELFINGER FJ**. The role of sodium in intestinal glucose absorption in man. *J Clin Invest.*, 1968, vol. 47 (5), 1133-42
- **FERRARIS RP** ; **YASHARPOUR S** ; **LLOYD KC** ; **MIRZAYAN R** ; **DIAMOND JM**. Luminal glucose concentrations in the gut under normal conditions. *Am J Physiol.*, 1990, vol. 259 (5), G822-37
- **MINUTH M** ; **JAKOBS KH**. Sodium regulation of agonist and antagonist binding to beta-adrenoceptors in intact and Ns-deficient membranes. *Naunyn Schmiedebergs Arch Pharmacol.*, 1986, vol. 333 (2), 124-9
- **BOKOCH MP** ; **ZOU Y** ; **RASMUSSEN SG** ; **LIU CW** ; **NYGAARD R** ; **ROSENBAUM DM et al.** Ligand-specific regulation of the extracellular surface of a G-protein-coupled receptor. *Nature*, 2010, vol. 463 (7277), 108-12
- **CANALS M** ; **SEXTON PM** ; **CHRISTOPOULOS A**. Allostery in GPCRs: 'MWC' revisited. *Trends Biochem Sci.*, 2011, vol. 36 (12), 663-72
- **ALAM MA** ; **AL-JENOOBI FI** ; **AL-MOHIZEA AM**. Everted gut sac model as a tool in pharmaceutical research: limitations and applications. *J Pharm Pharmacol.*, 2012, vol. 64 (3), 326-36
- **HAMILTON KL** ; **BUTT AG**. Glucose transport into everted sacs of the small intestine of mice. *Adv Physiol Educ.*, 2013, vol. 37 (4), 415-26

- **YU LC** ; **HUANG CY** ; **KUO WT** ; **SAYER H** ; **TURNER JR** ; **BURET AG**. SGLT-1-mediated glucose uptake protects human intestinal epithelial cells against Giardia duodenalis-induced apoptosis. *Int J Parasitol*, 2008, vol. 38 (8-9), 923-34
- **NALLI AD** ; **KUMAR DP** ; **MAHAVADI S** ; **AL-SHBOUL O** ; **ALKAHTANI R** ; **KUEMMERLE JF et al.** Hypercontractility of intestinal longitudinal smooth muscle induced by cytokines is mediated by the nuclear factor-kappaB/AMP-activated kinase/myosin light chain kinase pathway. *J Pharmacol Exp Ther.*, 2014, vol. 350 (1), 89-98
- **TAZAWA S** ; **YAMATO T** ; **FUJIKURA H** ; **HIRATOCHI M** ; **ITOH F** ; **TOMAE M et al.** SLC5A9/SGLT4, a new Na+-dependent glucose transporter, is an essential transporter for mannose, 1,5-anhydro-D-glucitol, and fructose. *Life Sci.*, 2005, vol. 76 (9), 1039-50
- **WRIGHT EM** ; **LOO DD** ; **HIRAYAMA BA**. Biology of human sodium glucose transporters. *Physiol Rev.*, 2011, vol. 91 (2), 733-94
- **ASSADI-PORTER FM** ; **TONELLI M** ; **MAILLET E** ; **HALLENGA K** ; **BENARD O** ; **MAX M et al.** Direct NMR detection of the binding of functional ligands to the human sweet receptor, a heterodimeric family 3 GPCR. *J Am Chem Soc.*, 2008, vol. 130 (23), 7212-3
- **ASSADI-PORTER FM** ; **TONELLI M** ; **MAILLET EL** ; **MARKLEY JL** ; **MAX M**. Interactions between the human sweet-sensing T1R2-T1R3 receptor and sweeteners detected by saturation transfer difference NMR spectroscopy. *Biochim Biophys Acta*, 2010, vol. 1798 (2), 82-6
- **VENKITAKRISHNAN RP** ; **BENARD O** ; **MAX M** ; **MARKLEY JL** ; **ASSADI-PORTER FM**. Use of NMR saturation transfer difference spectroscopy to study ligand binding to membrane proteins. *Methods Mol Biol.*, 2012, vol. 914, 47-63
- **MAYER M** ; **MEYER B**. Characterization of ligand binding by Saturation Transfer Difference NMR Spectroscopy. *Angew Chem Int Ed Engl*, 1999, vol. 38 (12), 1784-8
- **BOIVIN V** ; **JAHNS R** ; **GAMBARYAN S** ; **NESS W** ; **BOEGE F** ; **LOHSE MJ**. Immunofluorescent imaging of beta 1- and beta 2-adrenergic receptors in rat kidney. *Kidney Int.*, 2001, vol. 59 (2), 515-31
- **BLAKE WD**. Effect of epinephrine on tubular reabsorption of glucose by dog kidney. *Am J Physiol.*, 1962, vol. 202, 897-900
- **GOFF JP**. Invited review: Mineral absorption mechanisms, mineral interactions that affect acid-base and antioxidant status, and diet considerations to improve mineral status. *J Dairy Sci.*, 2018, vol. 101 (4), 2763-813
- **YU O** ; **OUYANG A**. Distribution of beta-adrenoceptor subtypes in gastrointestinal tract of nondiabetic and diabetic BB rats. A longitudinal study. *Dig Dis Sci.*, 1997, vol. 42 (6), 1146-53
- **VIJFTIGSCHILD LA** ; **BERKERS G** ; **DEKKERS JF** ; **ZOMER-VAN OMMEN DD** ; **MATTHES E** ; **KRUISSELBRINK E et al.** beta2-Adrenergic receptor agonists activate CFTR in intestinal organoids and subjects with cystic fibrosis. *Eur Respir J.*, 2016, vol. 48 (3), 768-79
- **MAILLEAU C** ; **CAPEAU J** ; **BRAHIMI-HORN MC**. Interrelationship between the Na+/glucose cotransporter and CFTR in Caco-2 cells: relevance to cystic fibrosis. *J Cell Physiol.*, 1998, vol. 176 (3), 472-81
- **RE G** ; **BADINO P** ; **DE ANGELIS I** ; **ODORE R** ; **BELLOLI C** ; **STAMMATI A et al.** Identification and coupling to adenylate cyclase of three different [(3)H] CGP 12177 binding sites in Caco-2 cell membranes. *Pharmacol Res.*, 2001, vol. 43 (4), 393-8
- **WRIGHT EM** ; **HIRSCH JR** ; **LOO DD** ; **ZAMPIGHI GA**. Regulation of Na+/glucose cotransporters. *J Exp Biol*, 1997, vol. 200 (2), 287-93
- **VESTRI S** ; **OKAMOTO MM** ; **DE FREITAS HS** ; **APARECIDA DOS SANTOS R** ; **NUNES MT** ; **MORIMATSU M et al.** Changes in sodium or glucose filtration rate modulate expression of glucose transporters in renal proximal tubular cells of rat. *J Membr Biol.*, 2001, vol. 182 (2), 105-12
- **SHEPARD BD** ; **PLUZNICK JL**. Saving the sweetness: renal glucose handling in health and disease. *Am J Physiol Renal Physiol.*, 2017, vol. 313 (1), F55-F61
- **SUBRAMANIAN S** ; **GLITZ P** ; **KIPP H** ; **KINNE RK** ; **CASTANEDA F**. Protein kinase-A affects sorting and conformation of the sodium-dependent glucose cotransporter SGLT1. *J Cell Biochem.*, 2009, vol. 106 (3), 444-52
- **SCHAFER N** ; **RIKKALA PR** ; **VEYHL-WICHMANN M** ; **KELLER T** ; **JUROWICH CF** ; **GEIGER D et al.** A Modified Tripeptide Motif of RS1 (RSC1A1) Down-Regulates Exocytotic Pathways of Human Na(+)-d-glucose Cotransporters SGLT1, SGLT2, and Glucose Sensor SGLT3 in the Presence of Glucose. *Mol Pharmacol.*, 2019, vol. 95 (1), 82-96
- **DOENST T** ; **TAEGTMEYER H**. alpha-adrenergic stimulation mediates glucose uptake through phosphatidylinositol 3-kinase in rat heart. *Circ Res.*, 1999, vol. 84 (4), 467-74
- **FAINTRENIE G** ; **GELOEN A**. Alpha-1 adrenergic stimulation of glucose uptake in rat white adipocytes. *J Pharmacol Exp Ther*, 1998, vol. 286 (2), 607-10
- **ABE H** ; **MINOKOSHI Y** ; **SHIMAZU T**. Effect of a beta 3-adrenergic agonist, BRL35135A, on glucose uptake in rat skeletal muscle in vivo and in vitro. *J Endocrinol.*, 1993, vol. 139 (3), 479-86

- **CHERNOGUBOVA E** ; **CANNON B** ; **BENGTSSON T**. Norepinephrine increases glucose transport in brown adipocytes via beta3-adrenoceptors through a cAMP, PKA, and PI3-kinase-dependent pathway stimulating conventional and novel PKCs. *Endocrinology*, 2004, vol. 145 (1), 269-80
- **CATUS SL** ; **GIBBS ME** ; **SATO M** ; **SUMMERS RJ** ; **HUTCHINSON DS**. Role of beta-adrenoceptors in glucose uptake in astrocytes using beta-adrenoceptor knockout mice. *Br J Pharmacol.*, 2011, vol. 162 (8), 1700-15
- **HUTCHINSON DS** ; **CHERNOGUBOVA E** ; **DALLNER OS** ; **CANNON B** ; **BENGTSSON T**. Beta-adrenoceptors, but not alpha-adrenoceptors, stimulate AMP-activated protein kinase in brown adipocytes independently of uncoupling protein-1. *Diabetologia*, 2005, vol. 48 (11), 2386-95
- **NEVZOROVA J** ; **BENGTSSON T** ; **EVANS BA** ; **SUMMERS RJ**. Characterization of the beta-adrenoceptor subtype involved in mediation of glucose transport in L6 cells. *Br J Pharmacol.*, 2002, vol. 137 (1), 9-18
- **KANDA Y** ; **WATANABE Y**. Adrenaline increases glucose transport via a Rap1-p38MAPK pathway in rat vascular smooth muscle cells. *Br J Pharmacol.*, 2007, vol. 151 (4), 476-82
- **NGALA RA** ; **O'DOWD J** ; **WANG SJ** ; **AGARWAL A** ; **STOCKER C** ; **CAWTHORNE MA et al.** Metabolic responses to BRL37344 and clenbuterol in soleus muscle and C2C12 cells via different atypical pharmacologies and beta2-adrenoceptor mechanisms. *Br J Pharmacol.*, 2008, vol. 155 (3), 395-406
- **NEVZOROVA J** ; **EVANS BA** ; **BENGTSSON T** ; **SUMMERS RJ**. Multiple signalling pathways involved in beta2-adrenoceptor-mediated glucose uptake in rat skeletal muscle cells. *Br J Pharmacol.*, 2006, vol. 147 (4), 446-54
- **LIU YL** ; **STOCK MJ**. Acute effects of the beta 3-adrenoceptor agonist, BRL 35135, on tissue glucose utilisation. *Br J Pharmacol.*, 1995, vol. 114 (4), 888-94
- **DONG JH** ; **CHEN X** ; **CUI M** ; **YU X** ; **PANG Q** ; **SUN JP**. beta2-adrenergic receptor and astrocyte glucose metabolism. *J Mol Neurosci.*, 2012, vol. 48 (2), 456-63
- **DEHVARI N** ; **HUTCHINSON DS** ; **NEVZOROVA J** ; **DALLNER OS** ; **SATO M** ; **KOCAN M et al.** beta(2)-Adrenoceptors increase translocation of GLUT4 via GPCR kinase sites in the receptor C-terminal tail. *Br J Pharmacol.*, 2012, vol. 165 (5), 1442-56
- **MUKAIDA S** ; **SATO M** ; **OBERG AI** ; **DEHVARI N** ; **OLSEN JM** ; **KOCAN M et al.** BRL37344 stimulates GLUT4 translocation and glucose uptake in skeletal muscle via beta2-adrenoceptors without causing classical receptor desensitization. *Am J Physiol Regul Integr Comp Physiol*, 2019, vol. 316 (5), R666-R77
- **SHIRAZI-BEECHEY SP** ; **MORAN AW** ; **BRAVO D** ; **AL-RAMMAHI M**. Nonruminant Nutrition Symposium: intestinal glucose sensing and regulation of glucose absorption: implications for swine nutrition. *J Anim Sci.*, 2011, vol. 89 (6), 1854-62
- **STEARNS AT** ; **BALAKRISHNAN A** ; **RHOADS DB** ; **TAVAKKOLIZADEH A**. Rapid upregulation of sodium-glucose transporter SGLT1 in response to intestinal sweet taste stimulation. *Ann Surg.*, 2010, vol. 251 (5), 865-71
- **GROMOVA LV** ; **FETISSOV SO** ; **GRUZDKOV AA**. Mechanisms of Glucose Absorption in the Small Intestine in Health and Metabolic Diseases and Their Role in Appetite Regulation. *Nutrients*, 2021, vol. 13 (7)
- **THEVELEIN JM** ; **VOORDECKERS K**. Functioning and evolutionary significance of nutrient transceptors. *Mol Biol Evol.*, 2009, vol. 26 (11), 2407-14
- **NORDSTROM KJ** ; **SALLMAN ALMEN M** ; **EDSTAM MM** ; **FREDRIKSSON R** ; **SCHIOTH HB**. Independent HHsearch, Needleman--Wunsch-based, and motif analyses reveal the overall hierarchy for most of the G protein-coupled receptor families. *Mol Biol Evol.*, 2011, vol. 28 (9), 2471-80
- **DE MENDOZA A** ; **SEBE-PEDROS A** ; **RUIZ-TRILLO I**. The evolution of the GPCR signaling system in eukaryotes: modularity, conservation, and the transition to metazoan multicellularity. *Genome Biol Evol.*, 2014, vol. 6 (3), 606-19
- **KRISHNAN A** ; **ALMEN MS** ; **FREDRIKSSON R** ; **SCHIOTH HB**. The origin of GPCRs: identification of mammalian like Rhodopsin, Adhesion, Glutamate and Frizzled GPCRs in fungi. *PLoS One*, 2012, vol. 7 (1), e29817
- **GRAUL RC** ; **SADEE W**. Evolutionary relationships among G protein-coupled receptors using a clustered database approach. *AAPS PharmSci.*, 2001, vol. 3 (2), E12
- **WIEJAK J** ; **SURMACZ L** ; **WYROBA E**. Immunoanalogue of vertebrate beta-adrenergic receptor in the unicellular eukaryote Paramecium. *Histochem J*, 2002, vol. 34 (1-2), 51-6
- **WIEJAK J** ; **SURMACZ L** ; **WYROBA E**. Dynamin-association with agonist-mediated sequestration of beta-adrenergic receptor in single-cell eukaryote Paramecium. *J Exp Biol*, 2004, vol. 207 (10), 1625-32
- **PLATEK A** ; **WIEJAK J** ; **WYROBA E**. RT-PCR and Northern blot analysis in search for a putative Paramecium beta-adrenergic receptor. *Acta Biochim Pol.*, 1999, vol. 46 (3), 813-21

- **WYROBA E**. Stimulation of Paramecium phagocytosis by phorbol ester and forskolin. *Cell Biol Int Rep.*, 1987, vol. 11 (9), 657-64
- **WYROBA E**. Beta-adrenergic stimulation of phagocytosis in the unicellular eukaryote Paramecium aurelia. *Cell Biol Int Rep.*, 1989, vol. 13 (8), 667-78
- **DE CASTRO SL** ; **OLIVEIRA MM**. Radioligand binding characterization of beta-adrenergic receptors in the protozoa Trypanosoma cruzi. *Comp Biochem Physiol C.*, 1987, vol. 87 (1), 5-8
- **GHANOUNI P** ; **STEENHUIS JJ** ; **FARRENS DL** ; **KOBILKA BK**. Agonist-induced conformational changes in the G-protein-coupling domain of the beta 2 adrenergic receptor. *Proc Natl Acad Sci U S A.*, 2001, vol. 98 (11), 5997-6002
- **THEVELEIN JM** ; **DE WINDE JH**. Novel sensing mechanisms and targets for the cAMP-protein kinase A pathway in the yeast Saccharomyces cerevisiae. *Mol Microbiol*, 1999, vol. 33 (5), 904-18
- **AULSEBROOK KA**. Intestinal absorption of glucose and sodium: Effects of epinephrine and norepinephrine. *Biochem Biophys Res Commun.*, 1965, vol. 18, 165-9
- **ISHIKAWA Y** ; **EGUCHI T** ; **ISHIDA H**. Mechanism of beta-adrenergic agonist-induced transmural transport of glucose in rat small intestine. Regulation of phosphorylation of SGLT1 controls the function. *Biochim Biophys Acta*, 1997, vol. 1357 (3), 306-18
- **BOCHAIN A** ; **ESTEY L** ; **HARONIAN G** ; **REALE M** ; **ROJAS C** ; **CRAMER J**. Determination of catecholamine permeability coefficients for passive diffusion across phospholipid vesicle membranes. *J Membr Biol.*, 1981, vol. 60 (1), 73-6
- **ASCHENBACH JR** ; **BORAU T** ; **GABEL G**. Glucose uptake via SGLT-1 is stimulated by beta(2)-adrenoceptors in the ruminal epithelium of sheep. *J Nutr.*, 2002, vol. 132 (6), 1254-7
- **TAATJES DJ** ; **ROTH J**. Glycosylation in intestinal epithelium. *Int Rev Cytol.*, 1991, vol. 126, 135-93
- **BIOL MC** ; **MARTIN A** ; **LOUISOT P**. Nutritional and developmental regulation of glycosylation processes in digestive organs. *Biochimie*, 1992, vol. 74 (1), 13-24
- **MOREMEN KW** ; **TIEMEYER M** ; **NAIRN AV**. Vertebrate protein glycosylation: diversity, synthesis and function. *Nat Rev Mol Cell Biol.*, 2012, vol. 13 (7), 448-62
- **ASANO T** ; **OGIHARA T** ; **KATAGIRI H** ; **SAKODA H** ; **ONO H** ; **FUJISHIRO M et al.** Glucose transporter and Na+/glucose cotransporter as molecular targets of anti-diabetic drugs. *Curr Med Chem.*, 2004, vol. 11 (20), 2717-24
- **WAGMAN AS** ; **NUSS JM**. Current therapies and emerging targets for the treatment of diabetes. *Curr Pharm Des.*, 2001, vol. 7 (6), 417-50
- **OSSWALD C** ; **BAUMGARTEN K** ; **STUMPEL F** ; **GORBOULEV V** ; **AKIMJANOVA M** ; **KNOBELOCH KP et al.** Mice without the regulator gene Rsc1A1 exhibit increased Na+-D-glucose cotransport in small intestine and develop obesity. *Mol Cell Biol.*, 2005, vol. 25 (1), 78-87
- **DYER J** ; **WOOD IS** ; **PALEJWALA A** ; **ELLIS A** ; **SHIRAZI-BEECHEY SP**. Expression of monosaccharide transporters in intestine of diabetic humans. *Am J Physiol Gastrointest Liver Physiol*, 2002, vol. 282 (2), G241-8
- **KOEPSELL H**. Glucose transporters in the small intestine in health and disease. *Pflugers Arch.*, 2020, vol. 472 (9), 1207-48
- **NGUYEN NQ** ; **DEBRECENI TL** ; **BAMBRICK JE** ; **CHIA B** ; **WISHART J** ; **DEANE AM et al.** Accelerated intestinal glucose absorption in morbidly obese humans: relationship to glucose transporters, incretin hormones, and glycemia. *J Clin Endocrinol Metab.*, 2015, vol. 100 (3), 968-76
- **SOERGEL KH**. Colonic fermentation: metabolic and clinical implications. *Clin Investig.*, 1994, vol. 72 (10), 742-8
- **WRIGHT EM** ; **HIRAYAMA BA** ; **LOO DF**. Active sugar transport in health and disease. *J Intern Med.*, 2007, vol. 261 (1), 32-43
- **SANTULLI G** ; **LOMBARDI A** ; **SORRIENTO D** ; **ANASTASIO A** ; **DEL GIUDICE C** ; **FORMISANO P et al.** Age-related impairment in insulin release: the essential role of beta(2)-adrenergic receptor. *Diabetes*, 2012, vol. 61 (3), 692-701
- **SKIKAMA H** ; **UI M**. Adrenergic receptor and epinephrine-induced hyperglycemia and glucose tolerance. *Am J Physiol.*, 1975, vol. 229 (4), 962-6
- **HOLM G** ; **JOHANSSON S** ; **VEDIN A** ; **WILHELMSSON C** ; **SMITH U**. The effect of beta-blockade on glucose tolerance and insulin release in adult diabetes. *Acta Med Scand.*, 1980, vol. 208 (3), 187-91
- **MOORE CA** ; **MILANO SK** ; **BENOVIC JL**. Regulation of receptor trafficking by GRKs and arrestins. *Annu Rev Physiol.*, 2007, vol. 69, 451-82
- **YUDOWSKI GA** ; **PUTHENVEEDU MA** ; **HENRY AG** ; **VON ZASTROW M**. Cargo-mediated regulation of a rapid Rab4-dependent recycling pathway. *Mol Biol Cell*, 2009, vol. 20 (11), 2774-84
- **BARRON AJ** ; **ZAMAN N** ; **COLE GD** ; **WENSEL R** ; **OKONKO DO** ; **FRANCIS DP**. Systematic review of genuine versus spurious side-effects of beta-blockers in heart failure using placebo control: recommendations for patient information. *Int J Cardiol.*, 2013, vol. 168 (4), 3572-9
- **SEARS MR**. Adverse effects of beta-agonists. *J Allergy Clin Immunol.*, 2002, vol. 110 (6), 322-8

- **LEONETTI G** ; **EGAN CG**. Use of carvedilol in hypertension: an update. *Vasc Health Risk Manag.*, 2012, vol. 8, 307-22
- **MESSERLI FH** ; **GROSSMAN E**. beta-Blockers in hypertension: is carvedilol different?. *Am J Cardiol.*, 2004, vol. 93 (9A), 7B-12B
- **NILSSON PM** ; **CEDERHOLM J** ; **ZETHELIUS BR** ; **ELIASSON BR** ; **EEG-OLOFSSON K** ; **GUDBJ RNSDOTTIR S**. Trends in blood pressure control in patients with type 2 diabetes: data from the Swedish National Diabetes Register (NDR). *Blood Press.*, 2011, vol. 20 (6), 348-54
- **ELAYAN H** ; **MILIC M** ; **SUN P** ; **GHARAIBEH M** ; **ZIEGLER MG**. Chronic beta2 adrenergic agonist, but not exercise, improves glucose handling in older type 2 diabetic mice. *Cell Mol Neurobiol.*, 2012, vol. 32 (5), 871-7
- **ZIEGLER MG** ; **ELAYAN H** ; **MILIC M** ; **SUN P** ; **GHARAIBEH M**. Epinephrine and the metabolic syndrome. *Curr Hypertens Rep.*, 2012, vol. 14 (1), 1-7
- **JENSEN J** ; **RUZZIN J** ; **JEBENS E** ; **BRENNESVIK EO** ; **KNARDAHL S**. Improved insulin-stimulated glucose uptake and glycogen synthase activation in rat skeletal muscles after adrenaline infusion: role of glycogen content and PKB phosphorylation. *Acta Physiol Scand.*, 2005, vol. 184 (2), 121-30
- **ERNANDE L** ; **STANFORD KI** ; **THOONEN R** ; **ZHANG H** ; **CLERTE M** ; **HIRSHMAN MF et al.** Relationship of brown adipose tissue perfusion and function: a study through beta2-adrenoreceptor stimulation. *J Appl Physiol (1985)*, 2016, vol. 120 (8), 825-32
- **COLE SW** ; **SOOD AK**. Molecular pathways: beta-adrenergic signaling in cancer. *Clin Cancer Res.*, 2012, vol. 18 (5), 1201-6
- **PEREZ-SAYANS M** ; **SOMOZA-MARTIN JM** ; **BARROS-ANGUEIRA F** ; **GAYOSO-DIZ P** ; **OTERO-REY EM** ; **GANDRA-REY JM et al.** Activity of beta2-adrenergic receptor in oral squamous cell carcinoma is mediated by overexpression of the ADRBK2 gene: a pilot study. *Biotech Histochem.*, 2012, vol. 87 (3), 179-86
- **JI Y** ; **CHEN S** ; **XIAO X** ; **ZHENG S** ; **LI K**. beta-blockers: a novel class of antitumor agents. *Onco Targets Ther.*, 2012, vol. 5, 391-401
- **CHAKIR K** ; **XIANG Y** ; **YANG D** ; **ZHANG SJ** ; **CHENG H** ; **KOBILKA BK et al.** The third intracellular loop and the carboxyl terminus of beta2-adrenergic receptor confer spontaneous activity of the receptor. *Mol Pharmacol.*, 2003, vol. 64 (5), 1048-58
- **MARGULIS L** ; **BANERJEE S** ; **WHITE T**. Colchicine-inhibited cilia regeneration: explanation for lack of effect in tris buffer medium. *Science*, 1969, vol. 164 (3884), 1177-8
- **RABA J** ; **MOTTOLA HA**. Glucose oxidase as an analytical reagent. *Crit Rev Anal Chem.*, 1995, vol. 25, 1-42
- **HOARE SR** ; **USDIN TB**. Quantitative cell membrane-based radioligand binding assays for parathyroid hormone receptors. *J Pharmacol Toxicol Methods*, 1999, vol. 41 (2-3), 83-90